(19) Europäisches Patentamt
European Patent Office
Office européen des brevets

(11) Veröffentlichungsnummer: **0 310 126 B1**

(12) **EUROPÄISCHE PATENTSCHRIFT**

(45) Veröffentlichungstag der Patentschrift: **18.08.93**

(51) Int. Cl.$^5$: **C07C 65/24**, C07C 65/40, C07C 49/255, C07C 43/295, C07C 69/76

(21) Anmeldenummer: **88116255.6**

(22) Anmeldetag: **30.09.88**

# (54) Brenzcatechincarbonsäurederivate, ihre Herstellung und Verwendung als Wirkstoffe für Arzneimittel.

(30) Priorität: **01.10.87 US 103789**
**22.07.88 US 223470**

(43) Veröffentlichungstag der Anmeldung:
**05.04.89 Patentblatt 89/14**

(45) Bekanntmachung des Hinweises auf die Patenterteilung:
**18.08.93 Patentblatt 93/33**

(84) Benannte Vertragsstaaten:
**AT BE CH DE ES FR GB GR IT LI LU NL SE**

(56) Entgegenhaltungen:
**EP-A- 0 068 250**

(73) Patentinhaber: **F. HOFFMANN-LA ROCHE AG**
**Postfach 3255**
**CH-4002 Basel(CH)**

(72) Erfinder: **Carson, Matthew**
**40 Brookfield Avenue**
**Nutley N.J. 07110(US)**
Erfinder: **Han, Ru-Jen Lee**
**17 Colonia Avenue**
**Princeton Junction N.J. 08550(US)**
Erfinder: **LeMahieu, Ronald Andrew**
**18 Spruce Road**
**North Caldwell N.J. 07006(US)**

(74) Vertreter: **Lederer, Franz, Dr. et al**
**Lederer, Keller & Riederer Patentanwälte**
**Lucile-Grahn-Strasse 22**
**D-81675 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 310 126 B1

## Beschreibung

Die vorliegende Erfindung betrifft Brenzcatechincarbonsäurederivate der Formel

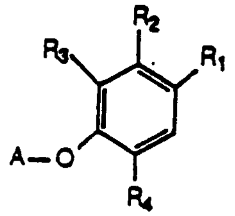

I

worin $R_1$ -C(O)OR, Wasserstoff, Acetyl, Hydroxy oder Alkanoyloxy; $R_2$ -C(O)OR, Hydroxy, Wasserstoff oder Alkanoyloxy; R Wasserstoff, nieder-Alkyl oder $-(CH_2)_nN$ (nieder-Alkyl)$_2$; $R_3$ Wasserstoff, nieder-Alkyl oder Amino; $R_4$ Wasserstoff, nieder-Alkyl, Halogen oder Amino darstellt, A eine Gruppe der Formel

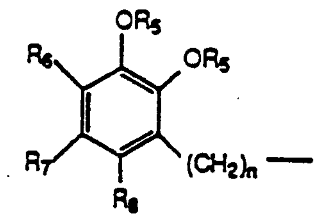

A'

darstellt, worin $R_5$ Wasserstoff oder Acyl; $R_6$ Wasserstoff, Halogen, nieder-Alkyl, Aryl oder Cycloalkyl; und $R_7$ und $R_8$ unabhängig voneinander Wasserstoff, nieder-Alkyl oder Halogen darstellen und n eine ganze Zahl von 2-10 ist, oder A eine Gruppe der Formel

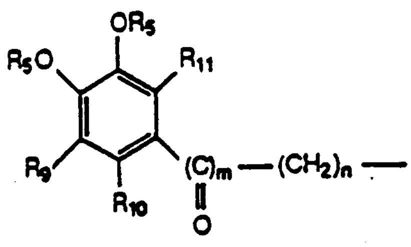

A"

darstellt, worin $R_5$ Wasserstoff oder Acyl; $R_9$ Wasserstoff oder nieder-Alkyl; $R_{10}$ Wasserstoff, nieder-Alkyl oder Halogen; $R_{11}$ Wasserstoff, nieder-Alkyl, Cycloalkyl oder Halogen darstellt, m 0 oder 1 und n eine ganze Zahl von 2-10 ist; vorausgesetzt, dass nicht mehr als ein Rest $R_1$ oder $R_2$ Hydroxy, Alkanoyloxy oder -C(O)OR ist,
und, falls R Wasserstoff ist, Salze davon mit pharmazeutisch akzeptablen Basen, und, falls R $(CH_2)_n$-N-(nieder-Alkyl)$_2$ ist, Säureadditionssalze davon mit pharmazeutisch akzeptablen Säuren.

Die Verbindungen der Formel I sind als Mittel zur Behandlung von entzündlichen Erkrankungen wie Arthritis, entzündlichen Darmerkrankungen wie Colitis, kardiovaskulären Erkrankungen wie Myocardinfarkt, Hauterkrankungen wie Psoriasis (bei topischer Verabreichung) und bronchopulmonärer Erkrankungen wie Asthma von Wert.

Die hier verwendete Ausduck "nieder-Alkyl" bezeichnet einen geradkettigen oder verzweigten gesättigten Kohlenwasserstoffrest mit 1-7 C-Atomen, beispielsweise Methyl, Aethyl, Propyl, Isopropyl, Butyl, t-Butyl, Neopentyl, Pentyl, Heptyl. Verzweigte gesättigte Kohlenwasserstoffreste sind für $R_6$, $R_9$ und $R_{11}$ bevorzugt. Der Ausdruck "Halogen" bezieht sich auf alle Halogene, d.h. Brom, Chlor, Fluor und Jod. Der Ausdruck "Aryl" bezeichnet Phenyl oder Phenyl mit einem oder zwei Substituenten aus der Gruppe Halogen, Trifluormethyl, nieder-Alkyl, nieder-Alkoxy, Nitro, Amino, nieder-Alkylamin und Di-nieder-Alkylamino. Der Ausdruck "Acyl" bezeichnet eine von einer aliphatischen Carbonsäure mit 1-7 C-Atomen abgeleitete Alkanoylgruppe, beispielsweise Formyl, Acetyl, Propionyl; eine Aroylgruppe leitet sich von einer aromatischen Carbonsäure ab, ein Beispiel ist Benzoyl. Der Ausdruck "Alkanoyloxy" bezeichnet eine von einer aliphatischen Carbonsäure mit 1-7 C-Atomen abgeleiteten Rest, beispielsweise Formyloxy, Acetoxy und Propionyloxy. Der Ausdruck "Cycloalkyl" bezeichnet vorzugsweise einen cyclischen Kohlenwasserstoffrest mit 3-6 C-Atomen, der unsubstituiert oder durch nieder-Alkyl substituiert sein kann und der vorzugsweise 5-6 C-Atome enthält, beispielsweise Cyclopropyl, Cyclopentyl und Cyclohexyl.

Der Verbindungen der Formel I können auch durch die Formel Ia und Ib dargestellt werden, je nach dem ob der Rest A ein Rest A′ oder A″ ist:

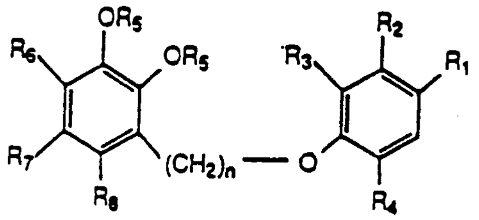

Ia

oder

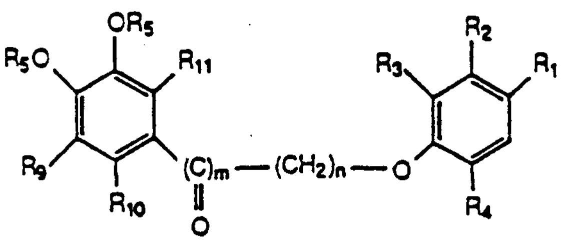

Ib

wobei $R_1$-$R_{11}$, m und n die obige Bedeutung haben.

Bevorzugte Verbindungen der Formel Ia sind diejenigen, in denen $R_1$ Carboxy oder Acetyl, $R_2$ Hydroxy, $R_3$ Wasserstoff oder Propyl, $R_4$ Wasserstoff oder Chlor, n eine ganze Zahl von 2-10, $R_5$ Wasserstoff oder Acetyl, $R_6$ Wasserstoff, nieder-Alkyl oder Aryl und $R_7$ und $R_8$ Wasserstoff sind.

Bevorzugte Verbindungen der Formel Ib sind diejenigen, in denen $R_1$ Carboxy oder Acetyl, $R_2$ Hydroxy, $R_3$ Wasserstoff oder Propyl, $R_4$ Wasserstoff oder Chlor, m 0 oder 1, n eine ganze Zahl von 2-10, $R_5$ Wasserstoff oder Acetyl, $R_9$ und $R_{10}$ Wasserstoff und $R_{11}$ Wasserstoff oder Chlor sind.

Mehr bevorzugte Verbindungen der Formel Ia sind diejenigen, in denen $R_1$ Carboxy oder Acetyl, $R_2$ Hydroxy, $R_3$ Wasserstoff oder Propyl, $R_4$ Wasserstoff oder Chlor, n eine ganze Zahl von 4-8, $R_5$ Wasserstoff oder Acetyl, $R_6$ Wasserstoff oder nieder-Alkyl und $R_7$ und $R_8$ Wasserstoff sind.

Mehr bevorzugte Verbindungen der Formel Ib sind diejenigen, in denen $R_1$ Carboxy oder Acetyl, $R_2$ Hydroxy, $R_3$ n-Propyl, $R_4$ Wasserstoff, m 0, n eine ganze Zahl von 4-6, $R_5$ Wasserstoff oder Acetyl, $R_9$ und $R_{10}$ Wasserstoff und $R_{11}$ Wasserstoff oder Chlor sind.

Am meisten bevorzugte Verbindungen der Formel Ia sind diejenigen, in denen $R_1$ Carboxy oder Acetyl, $R_2$ Hydroxy, $R_3$ n-Propyl, $R_4$ Wasserstoff, n eine ganze Zahl von 4-8, $R_5$ Wasserstoff, $R_6$ Wasserstoff oder $\alpha$-verzweigtes nieder-Alkyl und $R_7$ und $R_8$ Wasserstoff sind.

Am meisten bevorzugte Verbindungen der Formel Ib sind diejenigen, in denen $R_1$ Carboxy oder Acetyl, $R_2$ Hydroxy, $R_3$ n-Propyl, $R_4$ Wasserstoff, m 0, n eine ganze Zahl von 4-6 und $R_5$, $R_9$, $R_{10}$ und $R_{11}$ Wasserstoff sind.

Die bevorzugten erfindungsgemässen Verbindungen sind:

4-[6-(2,3-Dihydroxyphenyl)hexyloxy]-2-hydroxybenzoesäure;
4-[6-(2,3-Dihydroxyphenyl)hexyloxy]benzoesäure;
4-[6-(3,4-Dihydroxy-2,5-dimethylphenyl)hexyloxy]-2-hydroxy-3-propylbenzoesäure;
4-[5-(2,3,4-Trichlor-5,6-dihydroxyphenyl)pentyloxy]-2-hydroxy-3-propylbenzoesäure;
4-[4-(2,3-Dihydroxyphenyl)butoxy]-2-hydroxy-3-propylbenzoesäure;
4-[6-(2,3-Dihydroxyphenyl)hexyloxy]-2-hydroxy-3-propylbenzoesäure;
4-[8-(2,3-Dihydroxyphenyl)octyloxy]-2-hydroxy-3-propylbenzoesäure;
4-[6-[2,3-Bis(acetyloxy)phenyl]hexyloxy]-2-hydroxy-3-propylbenzoesäure;

4-[6-[2,3-Dihydroxy-4-(1-methyläthyl)phenyl]hexyloxy]-2-hydroxy-3-propylbenzoesäure;
4-[3-(3,4-Dihydroxyphenyl)propoxy]-2-hydroxy-3-propylbenzoesäure;
4-[6-(3,4-Dihydroxyphenyl)hexyloxy]-2-hydroxy-3-propylbenzoesäure;
4-[[6-(3,4-Dihydroxyphenyl)-6-oxohexyl]oxy]-2-hydroxy-3-propylbenzoesäure und
1-[2-Hydroxy-4-[4-(2,3-dihydroxyphenyl)butoxy]-3-propyl-phenyl]äthanon.

Verbindungen der Formel I können erfindungsgemäss dadurch hergestellt werden, dass man eine Verbindung der Formel

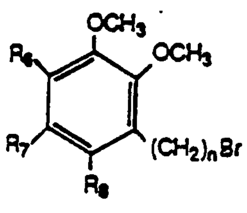

III

oder

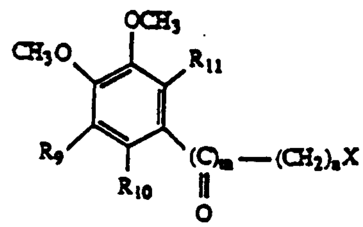

XXXVI

mit einer Verbindung der Formel

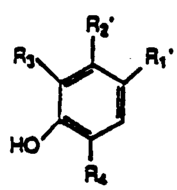

XXXIII

zu einer Verbindung der Formeln

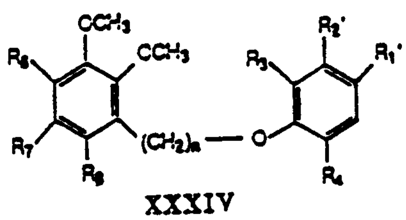

XXXIV

oder

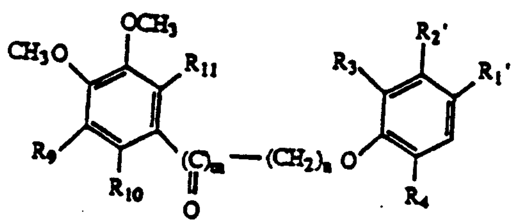

XXXVII

umsetzt oder
eine Verbindung der Formel

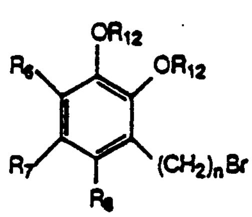

XXXIX

mit einer Verbindung der Formel

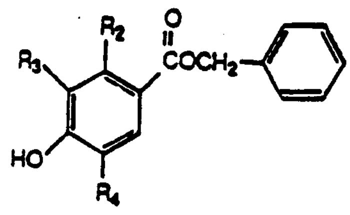

XXXX

zu einer Verbindung der Formel

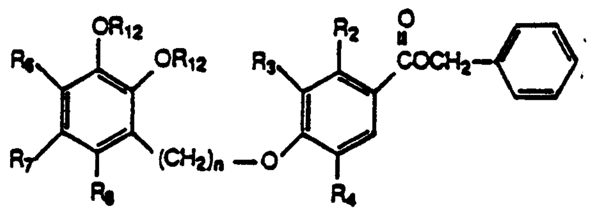

XXXXI

umsetzt,
Wobei $R_1'$ Wasserstoff, nieder-Alkoxycarbonyl oder Acetyl; $R_2'$ Wasserstoff oder nieder-Alkoxycarbonyl; $R_{12}$ Benzyl oder Acyl; X Brom oder Methansulfonyloxy darstellen und die restlichen Symbole die oben angegebene Bedeutung haben, vorausgesetzt, dass nicht mehr als ein Rest $R_1'$ oder $R_2'$ nieder-Alkoxycarbonyl ist,
worauf man die Methoxygruppen in einer Verbindung der Formel XXXIV oder XXXVII oder den Benzylester und eine gegebenenfalls anwesende Benzyläthergruppe $R_{12}$ in einer Verbindung der Formel XXXXI abspaltet, gewünschtenfalls eine Hydroxygruppe $R_2$ verestert und gewünschtenfalls eine Carboxygruppe $R_1$ oder $R_2$ verestert oder in ein Salz mit einer pharmazeutisch akzeptablen Base überführt oder eine die nieder-Alkyl-substituierte Aminogruppe in ein Salz mit einer pharmazeutisch akzeptablen Säure überführt.

Die Herstellung von Verbindungen der Formel I und Zwischenprodukte dafür ist ausführlicher in den folgenden Reaktionsschemata I-XII dargestellt.

Reaktionsschema I

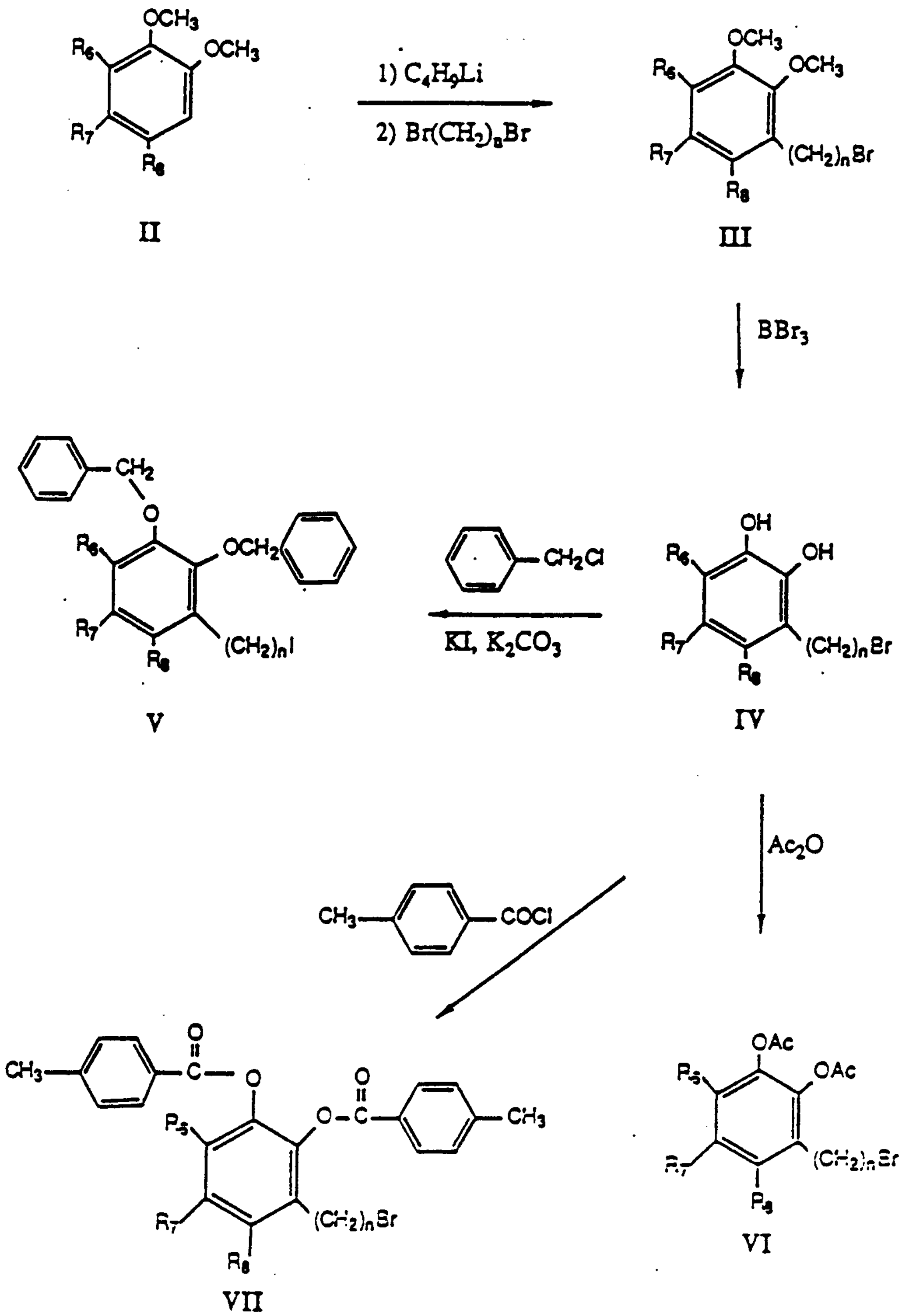


$R_6$, $R_7$, $R_8$ und n haben die obige Bedeutung, Ac ist Acetyl.

Im Reaktionsschema I wird eine Verbindung der Formel II, die eine bekannte Verbindung darstellt oder nach an sich bekannten Methoden hergestellt werden kann, in die entsprechende bekannte Verbindung der Formel III, wie in J. Chem. Soc. Perkin I, 2331 (1980) beschrieben, umgewandelt. Eine Verbindung der Formel II wird dabei mit einem Alkyllithium vorzugsweise Butyllithium in Gegenwart eines Lösungsmittels wie Diäthyläther oder Tetrahydrofuran bei einer Temperatur im Bereich von etwa -75° bis 0°C in das

entsprechende Lithiumsalz übergeführt, das man in situ mit einem Ueberschuss eines Dibromalkans bei einer Temperatur im Bereich von etwa 0-50 °C umsetzt.

Eine Verbindung der Formel III kann in die entsprechende Verbindung der Formel IV beispielsweise durch Behandlung mit Bortribromid in einem halogenierten Kohlenwasserstoff beispielsweise Chloroform oder 1,2-Dichloräthan oder vorzugsweise Methylenchlorid bei einer Temperatur im Bereich von etwa -75° bis etwa 25 °C umgewandelt werden.

Die erhaltene Verbindung der Formel IV kann in die entsprechende Verbindung der Formel V in Gegenwart von Benzylchlorid oder Benzylbromid, Kaliumjodid oder Natriumjodid und einem Alkalimetallcarbonat, beispielsweise Natrium- oder Kaliumcarbonat in einem Lösungsmittel wie Aceton oder Methyläthylketon bei Rückflusstemperatur oder mit Dimethylformamid bei einer Temperatur im Bereich von etwa 50-100 °C umgewandelt werden.

Die Verbindung der Formel IV kann in eine entsprechende Verbindung der Formel VI durch Behandlung mit Acetanhydrid und einem Säurekatalysator, beispielsweise Perchlorsäure in einem Lösungsmittel wie Aethylacetat bei einer Temperatur im Bereich von 0° bis etwa 25 °C umgewandelt werden.

Alternativ kann die erhaltene Verbindung der Formel IV in eine entsprechende Verbindung der Formel VII durch Umsetzung mit 4-Methylbenzoylchlorid in einer organischen tertiären Aminbase wie Triäthylamin in einem Lösungsmittel wie Tetrahydrofuran, Dioxan oder Aethyläther bei einer Temperatur im Bereich von 0° bis etwa 25 °C übergeführt werden.

Reaktionsschema II

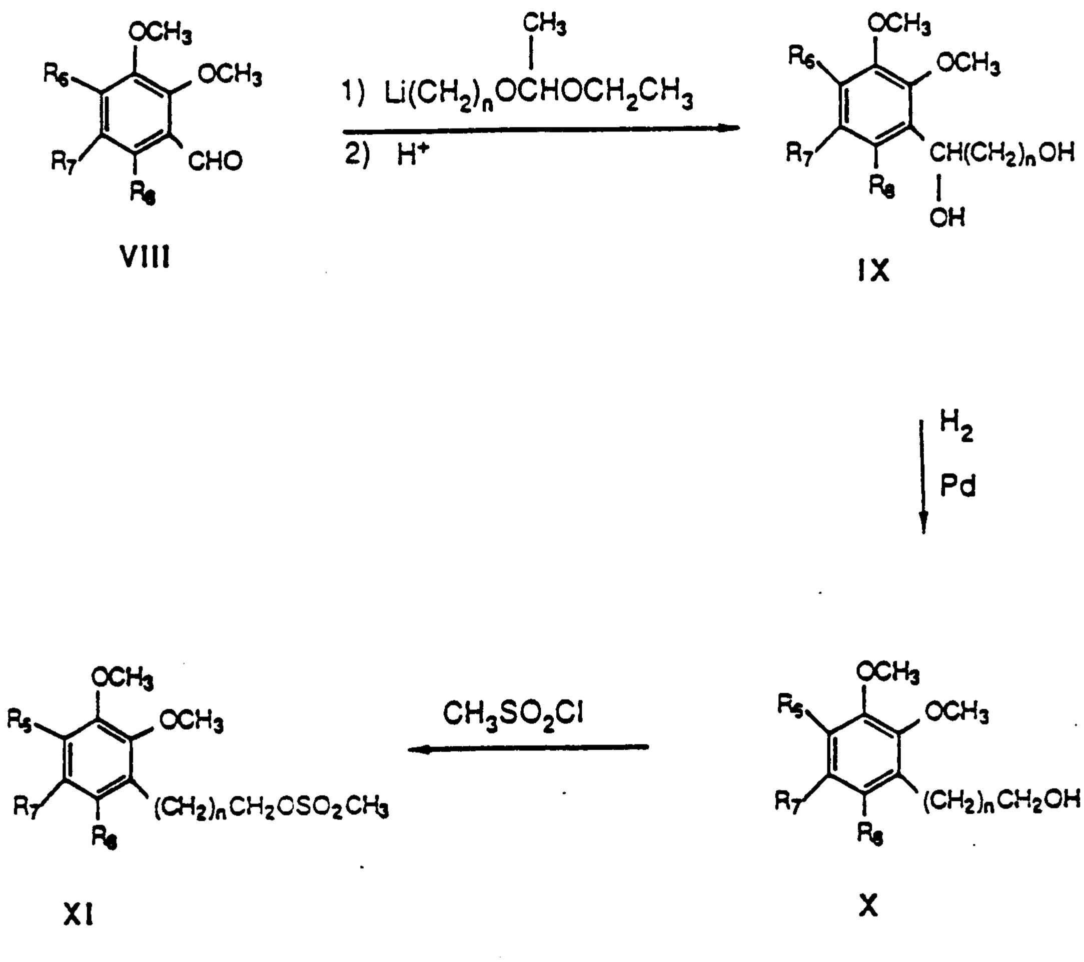


VIII IX X XI

$R_6$, $R_7$ und $R_8$ und n haben die obige Bedeutung.

Im Reaktionsschema II wird ein Aldehyd der Formel VIII, der eine bekannte Verbindung darstellt oder nach an sich bekannten Methoden hergestellt werden kann, in die entsprechende Verbindung der Formel IX, wie in Chem. Ind. 526 (1974) beschrieben, umgewandelt. Der Aldehyd der Formel VIII wird mit einem

Lithiumreagens in einem Lösungsmittel wie einem Aethyläther oder Tetrahydrofuran bei einer Temperatur im Bereich von etwa -20° bis etwa 35°C umgesetzt. Die Alkoholschutzgruppe kann aus dem Produkt durch Behandlung mit verdünnter Salzsäure bei 25°C entfernt werden, wobei man das Diol der Formel IX erhält.

Hydrogenolyse der Verbindung der Formel IX gibt die entsprechende Verbindung der Formel X. Die Hydrogenolyse kann durch Schütteln unter Wasserstoffatmosphäre mit einem Druck von etwa 2,7-4,1 bar in Gegenwart eines Palladiumkatalysators im Temperaturbereich bei etwa 25° bis etwa 50°C in einem Lösungsmittel wie Aethylacetat, Aethanol oder Tetrahydrofuran durchgeführt werden.

Die Umwandlung einer Verbindung der Formel X in das entsprechende Mesylat XI kann unter konventionellen Bedingungen, beispielsweise durch Umsetzung mit Methansulfonylchlorid und Triäthylamin in einem Lösungsmittel wie Methylenchlorid im Temperaturbereich von etwa -20° bis 25°C durchgeführt werden.

Reaktionsschema III

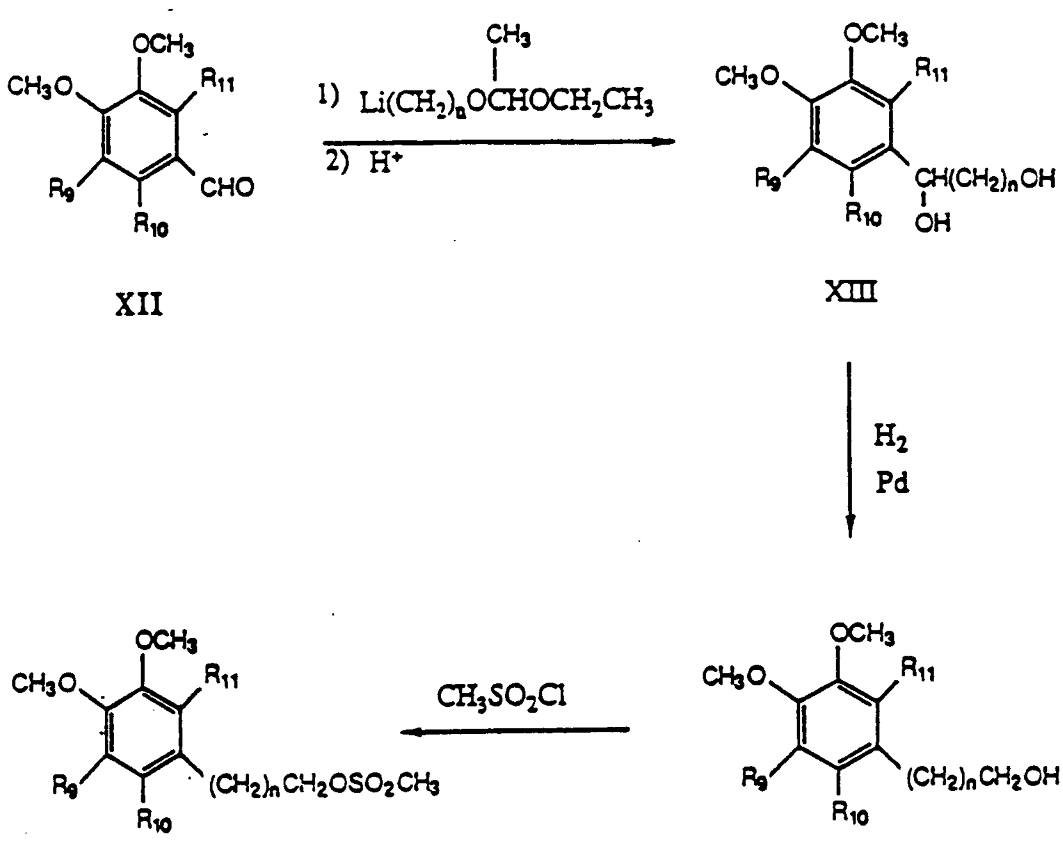


XII XIII XIV XV

$R_9$, $R_{10}$, $R_{11}$ und n haben die obige Bedeutung.

Im Reaktionsschema III wird eine Verbindung der Formel XII in die entsprechende Verbindung der Formel XIII umgewandelt, die wiederum in die entsprechenden Verbindungen der Formel XIV und XV unter den im Reaktionsschema II angegebenen Reaktionsbedingungen übergeführt werden können.

Reaktionsschema IV

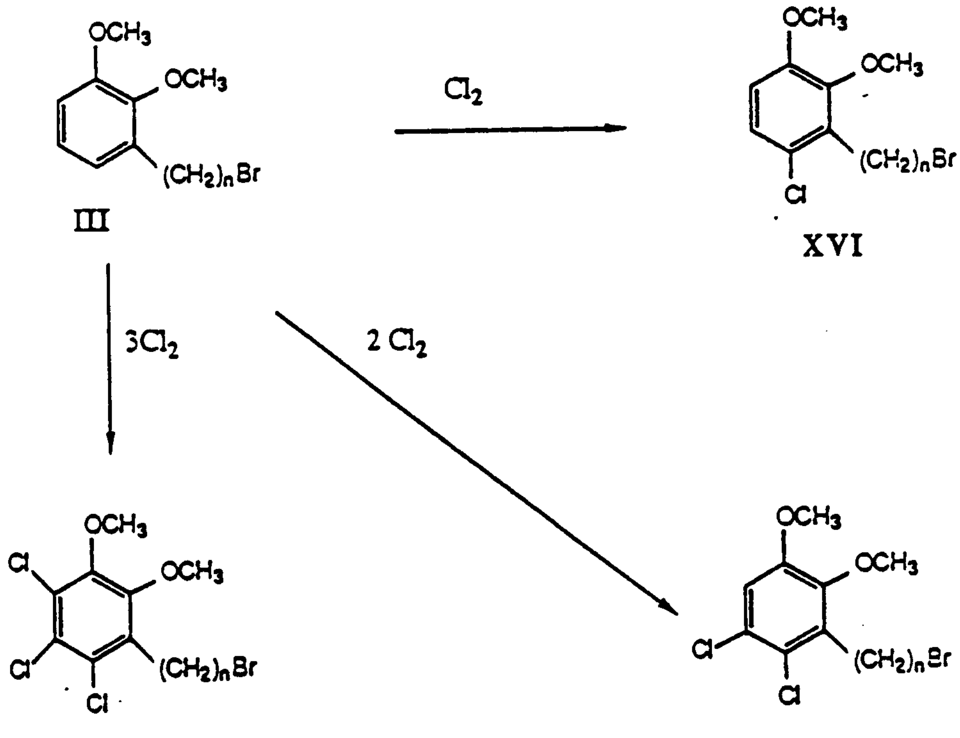


III

XVI

XVIII

XVII

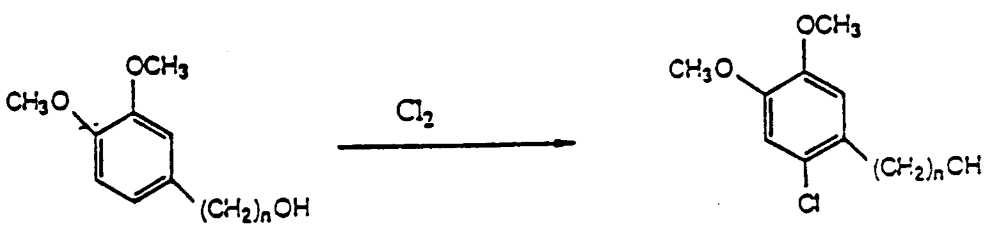


XIX

XX

n hat die obige Bedeutung.

Im Reaktionsschema IV wird eine Verbindung der Formel III, die eine bekannte Verbindung darstellt oder nach an sich bekannten Methoden hergestellt werden kann, in die entsprechenden Monochlorverbindungen der Formel XVI, die Dichlorverbindungen der Formel XVII und die Trichlorverbindungen der Formel XVIII durch Behandlung mit einer entsprechenden Menge Chlor in einem inerten Lösungsmittel wie einem chlorierten Kohlenwasserstoff, beispielsweise Methylenchlorid, Chloroform oder 1,2-Dichlormethan im Temperaturbereich von etwa -20° bis 25°C umgesetzt.

Die Umwandlung einer Verbindung der Formel XIX in die entsprechende Verbindung der Formel XX kann unter den weiter oben beschriebenen Bedingungen durchgeführt werden.

Reaktionsschema V

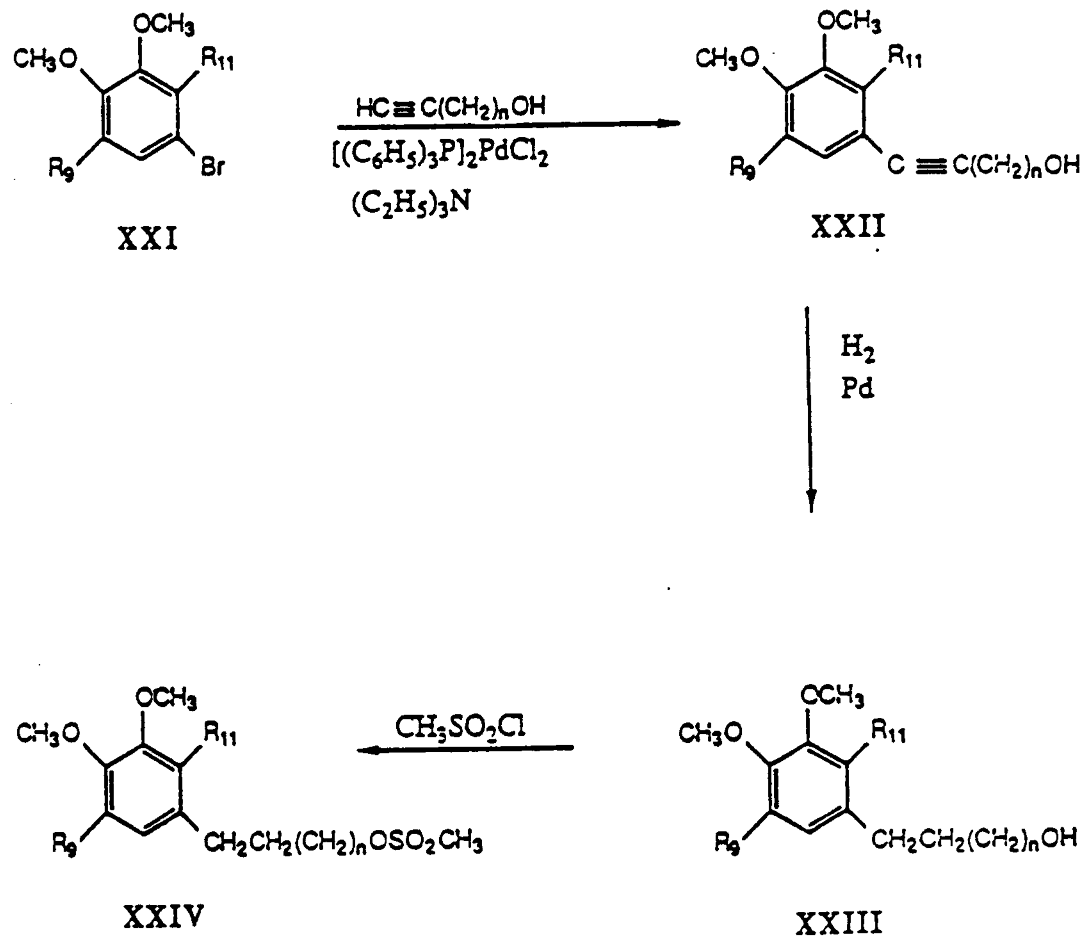


XXI

XXII

XXIII

XXIV

$R_9$, $R_{11}$ und n haben die obige Bedeutung.

Im Reaktionsschema V wird eine Verbindung der Formel XXI in einem acetylenischen Alkohol der Formel XXII durch Umsetzung mit einem acetylenischen Alkohol in Gegenwart von Bis(triphenylphosphin)-palladium-dichlorid, Kupfer(I)-jodid und einem organischen Amin (Triäthylamin), wie in Tet. Letters 4467 (1975) beschrieben, übergeführt.

Die Reaktion wird einem Lösungsmittel, beispielsweise einem halogenierten Kohlenwasserstoff wie Methylenchlorid, Chloroform oder 1,2-Dichloräthan im Temperaturbereich von 25° bis etwa 50°C durchgeführt.

Die erhaltene Verbindung der Formel XXII wird in eine Verbindung der Formel XXIII unter konventionellen Bedingungen beispielsweise katalytische Hydrierung unter Atmosphärendruck und bei Raumtemperatur durchgeführt.

Eine erhaltene Verbindung der Formel XXIII kann in eine Verbindung der Formel XXIV unter konventionellen Bedingungen beispielsweise durch Umsetzung mit Methansulfonylchlorid und Triäthylamin in Methylenchlorid im Temperaturbereich von -20° bis etwa 25°C umgewandelt werden.

Reaktionsschema VI

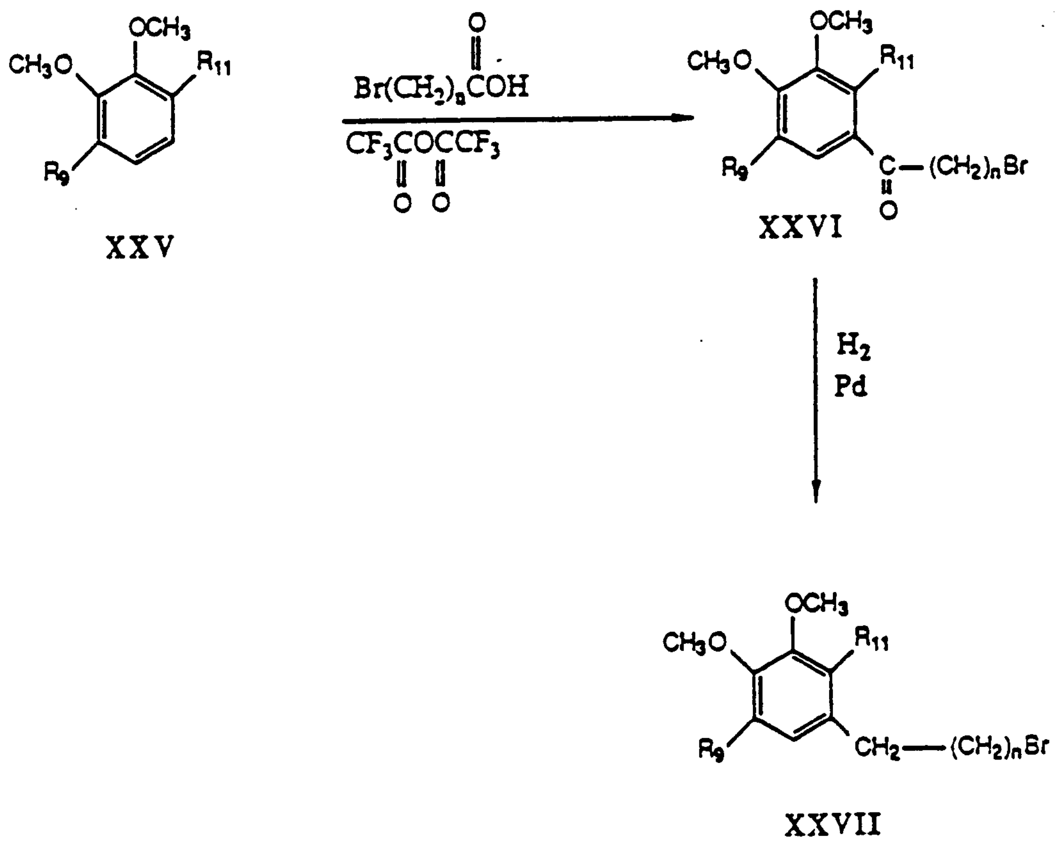


$R_9$, $R_{11}$ und n haben die obige Bedeutung.

Im Reaktionsschema VI wird eine Verbindung der Formel XXV, die eine bekannte Verbindung ist oder nach an sich bekannten Methoden hergestellt werden kann, in eine Verbindung der Formel XXVI mittels üblicher Acylierungsmittel übergeführt, beispielsweise durch Behandlung mit einer Bromsäure und Trifluoressigsäureanhydrid im Temperaturbereich von etwa 25-40 °C ohne Verwendung eines Lösungsmittels oder in Gegenwart eines Lösungsmittels wie Methylenchlorid oder 1,2-Dichloräthan. Alternativ kann ein Bromsäurechlorid und Aluminiumchlorid in einem Lösungsmittel wie Methylenchlorid oder 1,2-Dichloräthan im Temperaturbereich von 0-40 °C eingesetzt werden.

Die Reduktion einer Verbindung der Formel XXVI zur entsprechenden Verbindung der Formel XXVII kann durch Hydrierung bei etwa 3,4-4,1 bar in Gegenwart eines Palladiumkatalysators in einem Lösungsmittel wie Aethanol, Aethylacetat oder Tetrahydrofuran im Temperaturbereich von etwa 25-70 °C durchgeführt werden, wobei zusätzlich ein Mineralsäurekatalysator zugesetzt werden kann.

Reaktionsschema VII

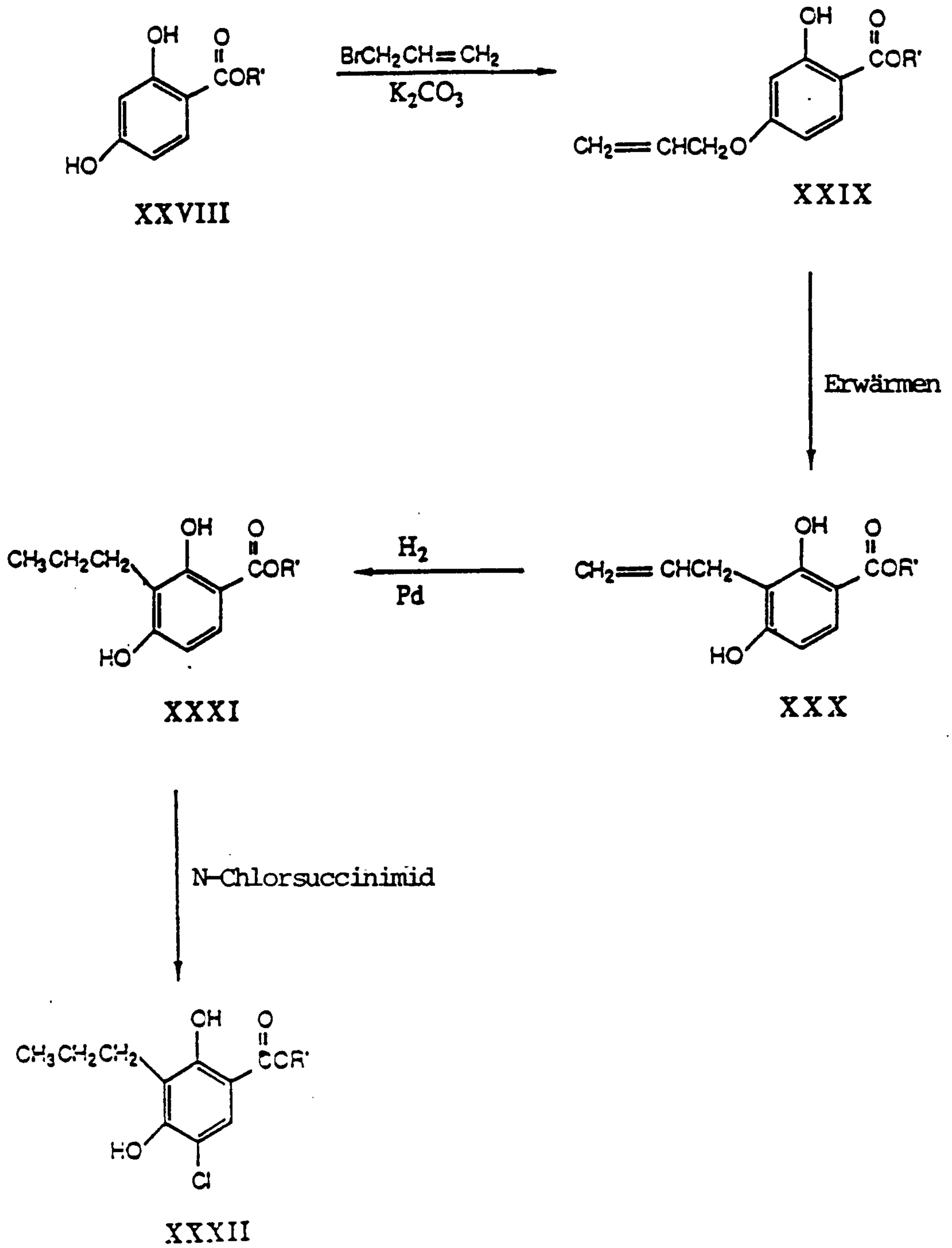


XXVIII

XXIX

XXX

XXXI

XXXII

R′ ist nieder-Alkyl.

Im Reaktionsschema VII wird eine Verbindung der Formel XXVIII, die eine bekannte Verbindung darstellt oder nach an sich bekannen Methoden hergestellt werden kann, in die entsprechende Verbindung der Formel XXIX mittels konventioneller Alkylierungsverfahren, beispielsweise mit Allylbromid oder Chlorid, einem Alkalimetallcarbonat wie Natriumcarbonat oder vorzugsweise Kaliumcarbonat in einem Lösungsmittel wie Methyläthylketon, Dimethylformamid oder vorzugsweise Aceton im Temperaturbereich von etwa 40-60 ° C umgesetzt.

Die Umlagerung einer Verbindung der Formel XXIX in eine Verbindung der Formel XXX wird durch Erwärmen unter Inertgas im Temperaturbereich von etwa 175 ° bis etwa 200 ° C durchgeführt.

Die Hydrierung einer Verbindung de Formel XXX in die entsprechende Verbindung der Formel XXXI kann mittels üblicher katalytische Hydrierverfahren beispielsweise bei Atmosphärendruck oder bei etwa 3,4 bar in einem Lösungsmittel wie Aethylacetat, Tetrahydrofuran oder Aethanol im Temperaturbereich von etwa 25-50 °C durchgeführt werden.

Die Chlorierung einer Verbindung der Formel XXXI zur entsprechenden Verbindung der Formel XXXII kann mittels üblicher Chloriermethoden beispielsweise mit N-Chlorsuccinimid in einem Lösungsmittel wie Tetrachlorkohlenstoff oder Chloroform bei Rückflusstemperatur durchgeführt werden.

Reaktionsschema VIII

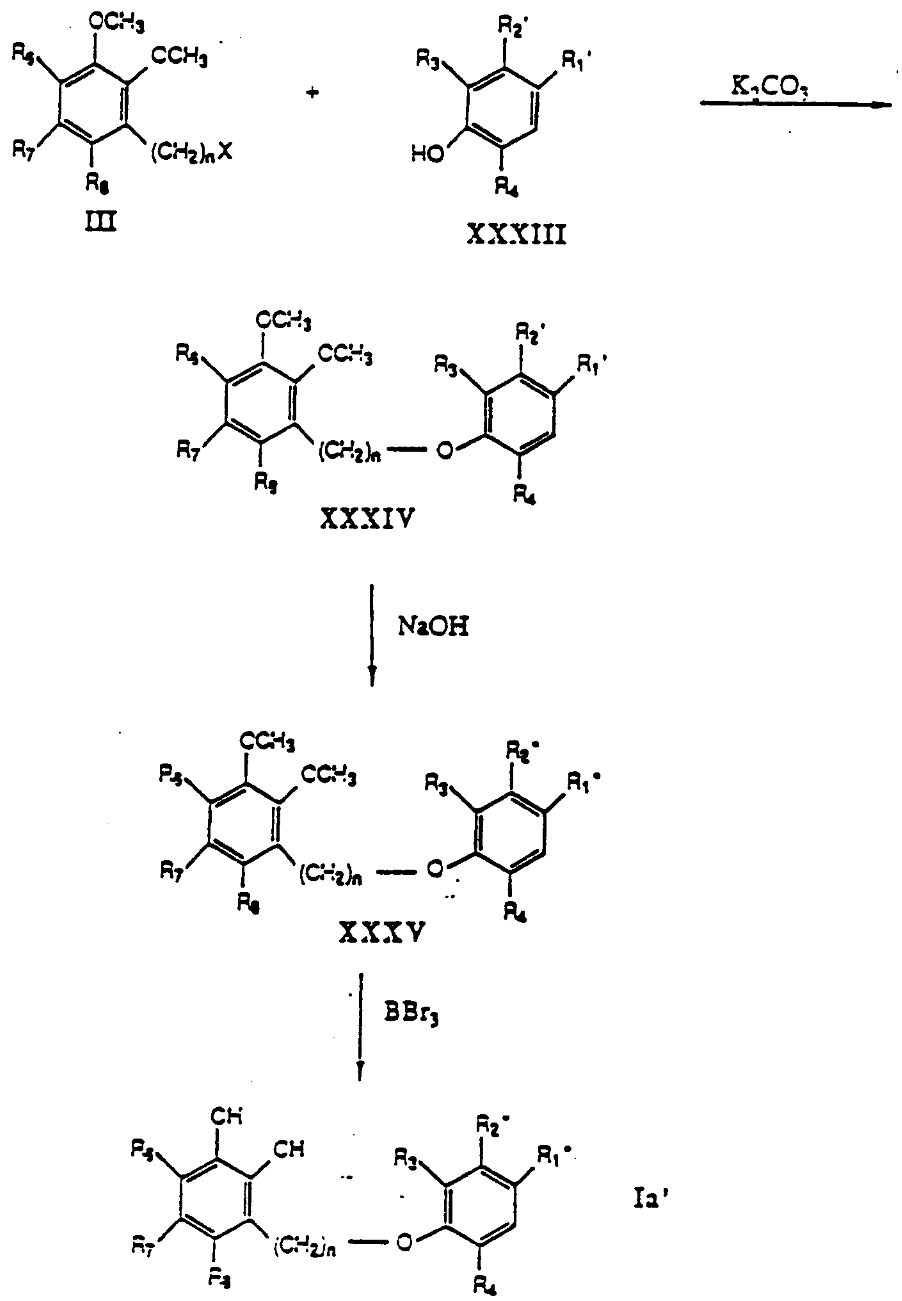


$R_1'$ ist -COOR', Acetyl, Wasserstoff oder Hydroxy, $R_2'$ ist -COOR', Wasserstoff oder Hydroxy, R' ist nieder-Alkyl, $R_1''$ ist Carboxy, Acetyl, Wasserstoff oder Hydroxy, $R_2''$ ist Carboxy, Wasserstoff oder Hydroxy, X ist Brom oder Methansulfonyloxy, vorausgesetzt, dass nur ein Rest $R_1'$ und $R_2'$ -COOR' sein kann und dass nur ein Rest $R_1''$ und $R_2''$ Carboxy sein kann und wobei $R_3$, $R_4$, $R_6$, $R_7$ und $R_8$ und n die obige Bedeutung haben.

Im Reaktionsschema VIII wird eine Verbindung der Formel III mit einer Verbindung der Formel XXXIII zur entsprechenden Verbindung de Formel XXXIV umgesetzt. Die Reaktion wird in Gegenwart eines Alkalimetallcarbonats als Base beispielsweise Natriumcarbonat und vorzugsweise Kaliumcarbonat unter Zusatz von Natrium- oder Kaliumjodid in einem Lösungsmittel wie Aceton, Methyläthylketon, Dimethylformamid oder Toluol im Temperaturbereich von etwa 40-70°C durchgeführt. Als Fest-Flüssig-Phasentransferkatalysator kann Tris[2-(2-methoxyäthoxy)äthyl]amin eingesetzt werden, wenn Toluol als Lösungsmittel verwendet wird.

Die Hydrolyse einer Verbindung der Formel XXXIV zur entsprechenden Verbindung der Formel XXXV kann unter üblichen Bedingungen beispielsweise mittels eines Alkalimetallhydroxids wie Natrium- oder Kaliumhydroxid in einem Lösungsmittel wie Methanol oder Aethanol gegebenenfalls unter Zusatz von Dioxan zur Verbesserung der Löslichkeit im Temperaturbereich von etwa 25-65°C durchgeführt werden.

Die Umwandlung einer Verbindung der Formel XXXV in die entsprechende Verbindung der Formel Ia′ kann durch Umsetzung mit Bortribromid in einem Lösungsmittel wie Methylenchlorid, Chloroform oder 1,2-Dichloräthan im Temperaturbereich von -70° bis 25°C durchgeführt werden. Die erhaltene Verbindung der Formel Ia′ wird abgetrennt und mittels konventioneller Verfahren beispielsweise durch Ausfällung, Kristallisation oder Chromatographie gereinigt.

Reaktionsschema IX

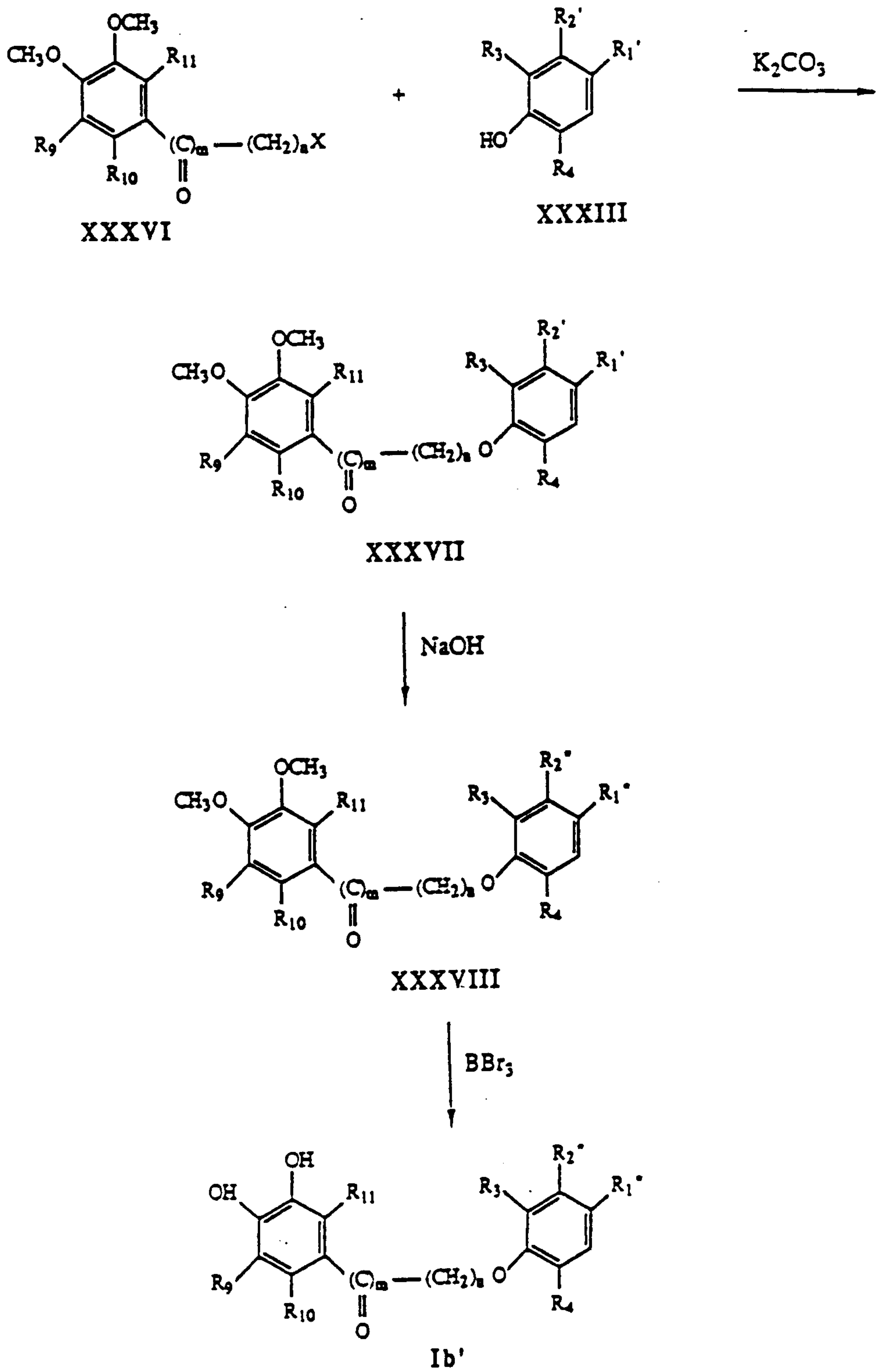


XXXVI

XXXIII

XXXVII

XXXVIII

Ib'

$R_3$, $R_4$, $R_9$, $R_{10}$, $R_{11}$, $R_2'$, $R_1'$, $R_2''$, $R_1''$, m, n und X haben die obige Bedeutung, vorausgesetzt, dass nur ein Rest $R_1'$ und $R_2'$ Hydroxy oder -COOR' ist, und dass nicht mehr als ein Rest $R_1''$ und $R_2''$ Carboxy oder Hydroxy ist.

Im Reaktionsschema IX wird eine Verbindung der Formel XXVI mit einer Verbindung der Formel XXXIII zur entsprechenden Verbindung der Formel XXXVII umgesetzt. Die Reaktion wird unter Verwendung eines Alkalimetallcarbonats wie Natrium- oder vorzugsweise Kaliumcarbonat in einem Lösungmittel wie Aceton,

Methyläthylketon oder Dimethylformamid im Temperaturbereich von etwa 40-70 ° C vorgenommen.

Die Umwandlung einer Verbindung der Formel XXXVII in die entsprechende Verbindung der Formel XXXVIII kann beispielsweise durch Behandlung mit einem Alkalimetallhydroxid wie Natrium- oder Kaliumhydroxid in einem Lösungsmittel wie Methanol oder Aethanol gegebenenfalls unter Zusatz von Dioxan zur Verbesserung der Löslichkeit im Temperaturbereich von etwa 25-65 ° C vorgenommen werden.

Die Umwandlung einer Verbindung der Formel XXXVIII in die entsprechende Verbindung der Formel Ib′ kann beispielsweise durch Behandlung mit Bortribromid in einem Lösungsmittel wie Methylenchlorid, Chloroform oder 1,2-Dichloräthan im Temperaturbereich von etwa -70 ° bis 25 ° C durchgeführt werden. Die erhaltene Verbindung der Formel Ib′ wird mit üblichen Verfahren beispielsweise Ausfällung, Kristallisation und Chromatographie abgetrennt und gereinigt.

Gewünschtenfalls kann eine Verbindung der Formel XXXVII, XXXVIII oder Ib′, worin m = 1 ist in die entsprechenden Verbindungen, in denen m = 0 ist und die verbindende Kette n + 1 Methylengruppen enthält durch Schütteln unter Wasserstoffatmosphäre umgewandelt werden. Diese Umwandlung kann in Gegenwart von Katalysatoren wie Palladium in Lösungsmitteln wie Aethanol, Aethylacetat oder Tetrahydrofuran unter einem Wasserstoffdruck von 2,7-4,1 bar ausgeführt werden. Dabei können kleine Mengen Säuren wie konzentrierte Schwefelsäure zugesetzt werden, um die Reaktion zu beschleunigen.

Reaktionsschema X

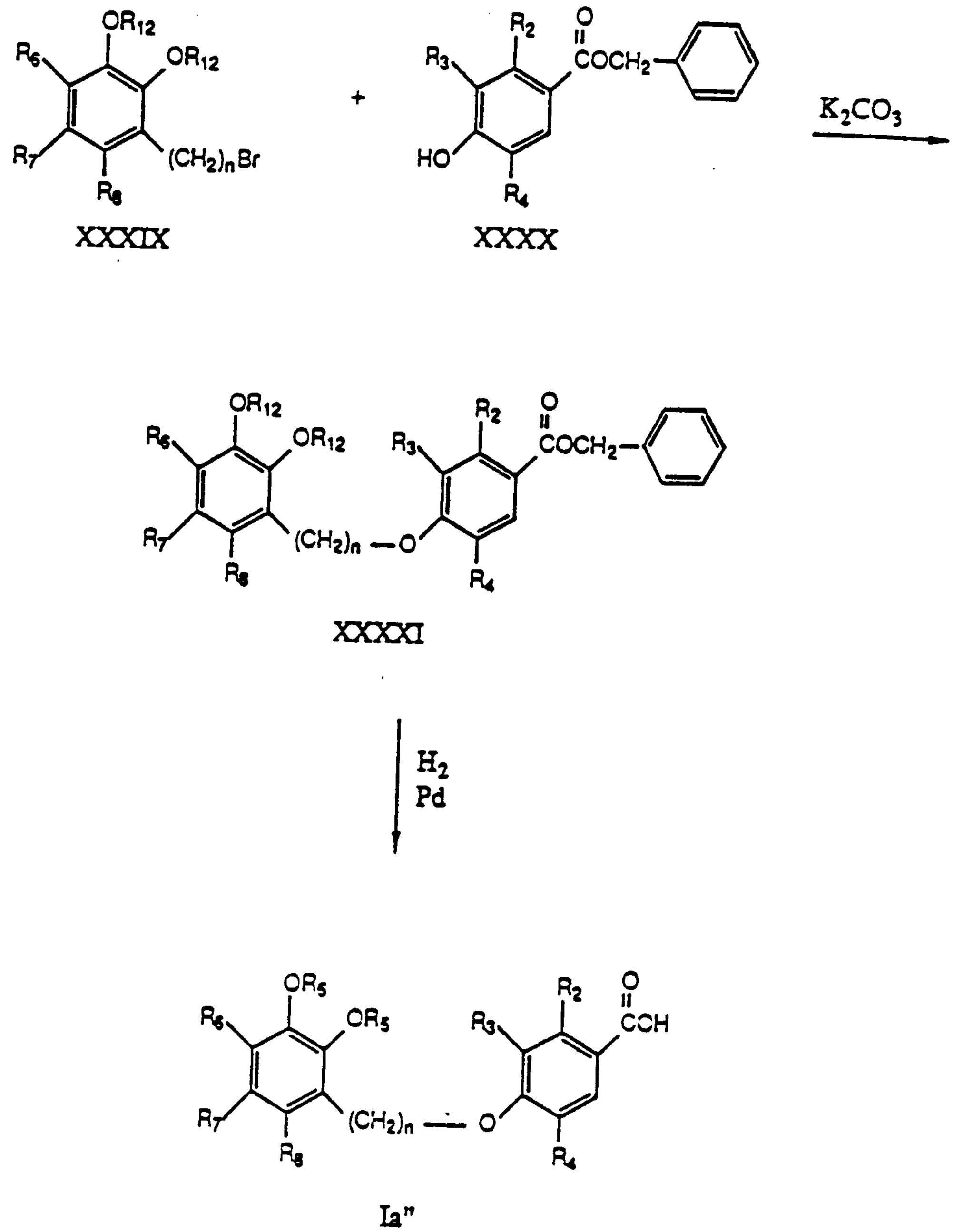


XXXIX XXXX

XXXXI

Ia″

$R_2$, $R_3$, $R_4$, $R_6$, $R_7$, $R_8$ und n haben die obige Bedeutung, $R_{12}$ ist Benzyl oder Acyl.

Im Reaktionsschema X wird eine Verbindung der Formel XXXIX mit einer Verbindung der Formel XXXX zu einer entsprechenden Verbindung der Formel XXXXI umgesetzt. Die Reaktion wird in Gegenwart eines Alkalimetallcarbonats wie Natrium- oder Kaliumcarbonat oder Natriumhydrid in einem Lösungsmittel wie Aceton, Methyläthylketon oder Dimethylformamid im Temperaturbereich von etwa 25-70 °C vorgenommen. Die Hydrogenolyse einer Verbindung der Formel XXXXI in die entsprechende Verbindung der Formel Ia″ kann mittels üblicher katalytischer Hydrogenolyseverfahren beispielsweise bei Atmosphärendruck oder unter einem Wasserstoffdruck von bis zu 3,4 bar im Temperaturbereich von etwa 25-50 °C in einem

Lösungsmittel wie Aethylacetat oder Tetrahydrofuran und in Gegenwart eines Katalysators wie Palladium erfolgen. Die erhaltene Verbindung der Formel Ia″ wird mittels konventioneller Verfahren beispielsweise Ausfällung, Kristallisation oder Chromatographie abgetrennt und gereinigt.

Reaktionsschema XI

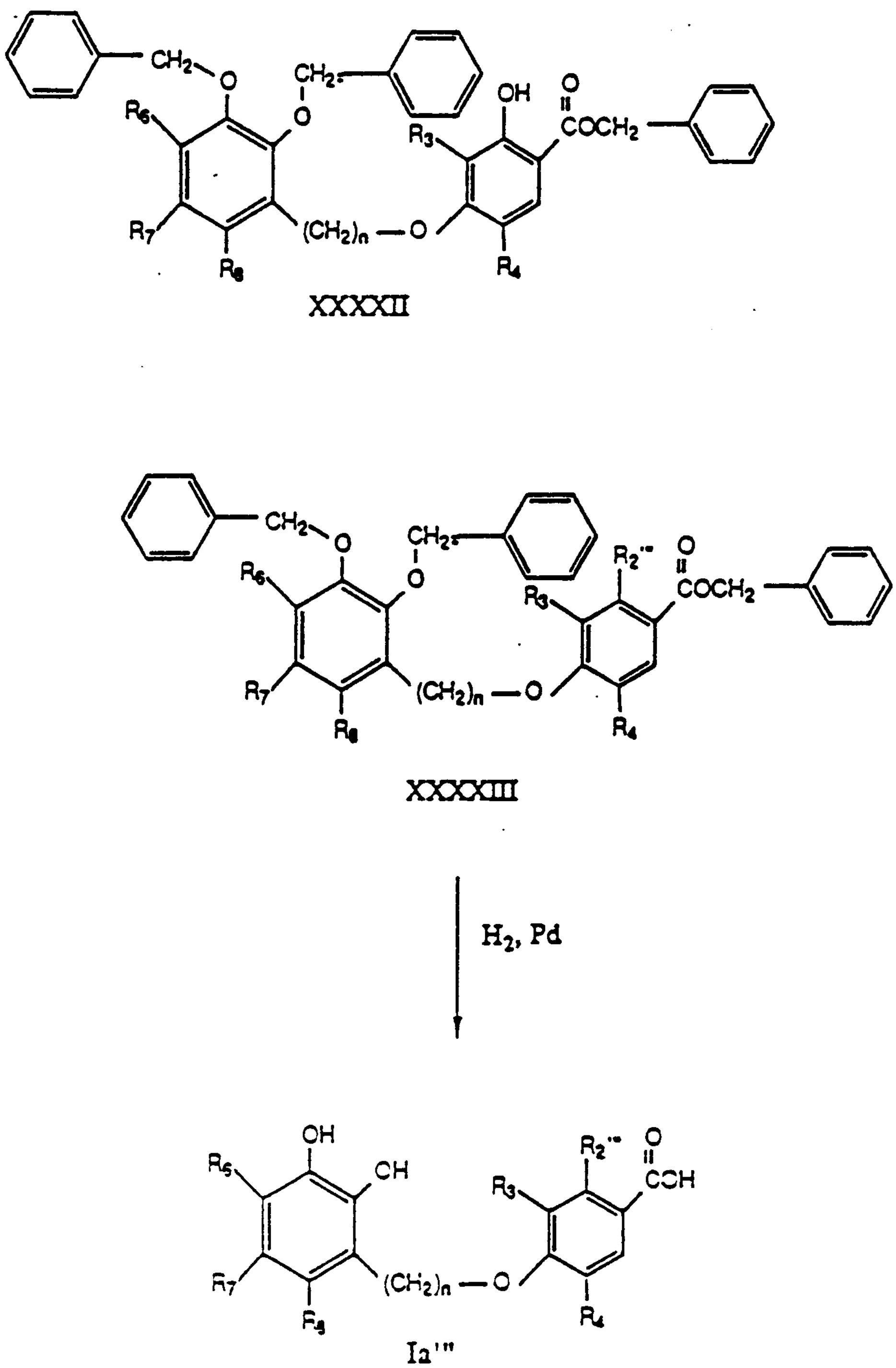


XXXXII

XXXXIII

Ia'''

$R_3$, $R_4$, $R_6$, $R_7$, $R_8$ und n haben die obige Bedeutung, $R_2''$ ist Alkanoyloxy.

Im Reaktionsschema XI wird eine Verbindung der Formel XXXXII in das entsprechende Alkanoylderivat der Formel XXXXIII durch Behandlung mit einem nieder-Alkylcarbonsäureanhydrid in Gegenwart einer organischen Base wie Pyridin im Temperaturbereich von etwa 25-70 °C übergeführt.

Die erhaltene Verbindung der Formel XXXXIII kann in eine Verbindung der Formel Ia''' durch Hydrogenolyse beispielsweise durch Schütteln unter Wasserstoffatmosphäre unter Druck oder unter Atmosphärendruck bei einer Temperatur im Bereich von etwa 25-70 °C in Gegenwart eines Katalysators wie Palladium in einem Lösungsmittel wie Aethylacetat oder Tetrahydrofuran umgewandelt werden.

Reaktionsschema XII

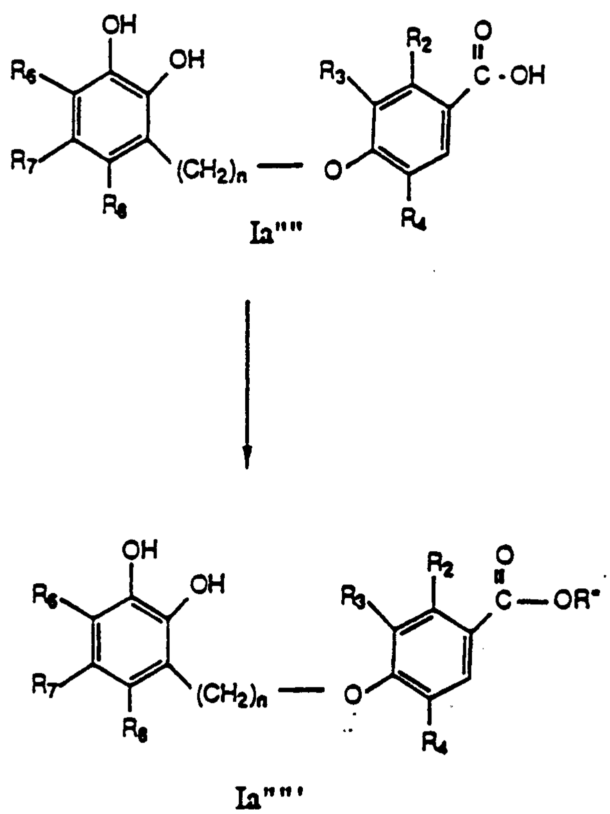

Ia''''

Ia'''''

$R_2$, $R_3$, $R_4$, $R_6$, $R_7$, $R_8$ und n haben die obige Bedeutung, R'' ist nieder-Alkyl oder $-(CH_2)_n-N-(\text{nieder-Alkyl})_2$, vorausgesetzt, dass $R_2$ von Carboxy verschieden ist.

Ein nieder-Alkylester oder ein basischer Ester der Formel Ia''''' und die entsprechende Verbindung der Formel Ib, worin $R_5$ Wasserstoff ist, können gemäss Reaktionsschema XII hergestellt werden.

Um einen nieder-Alkylester der Formel Ia''''' herzustellen, wird eine Verbindung der Formel Ia, worin $R_1$ oder $R_2$ -C(O)OH ist, d.h. eine Verbindung der Formel Ia'''' mit einem nieder-Alkyljodid in einem Lösungsmittel wie Aceton oder Dimethylformamid in Gegenwart eines Alkalimetallcarbonats wie Natrium- oder Kaliumcarbonat im Temperaturbereich von etwa 30-70 °C umgestzt.

Zur Herstellung eines basischen Esters der Formel Ia''''' wird eine Verbindung der Formel Ia, worin $R_1$ oder $R_2$ -C(O)OH ist, d.h. eine Verbindung der Formel Ia'''' mit einem di-nieder-Alkylamino-nieder-alkylchlorid in einem Lösungsmittel wie Dimethylformamid oder Tetrahydofuran in Gegenwart eines Alkalimetallbicarbonats wie Natrium- oder Kaliumbicarbonat im Temperaturbereich von etwa 30-70 °C umgesetzt.

Die in den Reaktionsschemata I-XII auftretenden Zwischenprodukte werden mittels an sich bekannter Verfahren, z.B. Ausfällung, Kristallisation oder Chromatographie vor dem nächsten Reaktionsschritt abgetrennt und gereinigt. Die Endprodukte der Formel I werden mit ähnlichen an sich bekannten Verfahren abgetrennt.

Die Erfindung betrifft weiterhin Salze von Verbindungen der Formel I, falls R Wasserstoff ist, wobei diese Salze durch Reaktion der Säure mit einer Base, die ein nicht-toxisches, pharmakologisch akzeptables

Kation enthalten, hergestellt werden. Im allgemeinen kommt hierfür jede Base, die ein Salz mit einer Carbonsäure bildet und deren pharmakologischen Eigenschaften keine unerwünschten physiologischen Effekte aufweisen erfindungsgemäss in Betracht. Geeignete Basen sind somit beispielsweise Alkalimetall- und Erdalkalimetallhydroxide und -carbonate, beispielsweise Calciumhydroxid, Natriumhydroxid, Kaliumcarbonat, Natriumcarbonat, Ammoniak, primäre, sekundäre und tertiäre Amine wie Monoalkylamine, Dialkylamine und Trialkylamine, beispielsweise Methylamin, Diäthylamin und Triäthylamin, stickstoffhaltige heterocyclische Amine, beispielsweise Piperidin. Ein so hergestelltes Salz ist das funktionelle Aequivalent der entsprechenden Verbindung der Formel I, worin R Wasserstoff ist, wobei die Auswahl der erfindungsgemässen in Betracht kommenden Salze allein durch das Kriterium limitiert ist, dass die angewandte Base sowohl nicht-toxisch als auch physiologisch akzeptabel ist.

Die Erfindung betrifft ebenfalls Additionssalze von Verbindungen der Formel I, in denen R $-(CH_2)_n-N-(\text{nieder-Alkyl})_2$ ist, welche Salze durch Umsetzung der genannten Amine mit einer nicht-toxischen, pharmakologisch oder pharmazeutisch akzeptablen Säure hergestellt werden. Im allgemeinen bilden die Verbindungen der Formel I pharmazeutisch akzeptable Additionssalze mit pharmazeutisch anwendbaren organischen und anorganischen Säuren wie Essigsäure, Bernsteinsäure, Ameisensäure, Methansulfonsäure, p-Toluolsulfonsäure, Salzsäure, Salpetersäure, Phosphorsäure und Schwefelsäure.

Es ist bekannt, dass der oxidative Stoffwechsel von Arachidonsäure über den $\Delta^5$-Lipoxygenase ($\Delta^5$-LO) Weg zu Peptidoleukotrienen ($LTC_4$ und $LTD_4$) und Leukotrien $B_4$ ($LTB_4$) führt. $LTC_4$ und $LTD_4$ sind wirksame Bronchokonstriktoren der menschlichen Bronchien und tragen bei zur Bildung von Oedemen bei einigen Spezies durch eine Erhöhung der kapillaren Permeabilität. $LTB_4$ ist ein wirksamer chemotaktischer Faktor für Entzündungszellen. $LTB_4$ ist auch in Synovialflüssigkeit von Patienten mit rheumatoider Arthritis und Gicht gefunden worden und kann ein Mediator entzündlicher Vorgänge und Gelenkzerstörung bei diesen Krankheiten sein. Infolgedessen können $\Delta^5$-LO-Hemmer einen therapeutischen Wert bei der Behandlung von Asthma und entzündlichen Erkrankungen haben.

Weiterhin sind Produkte des $\Delta^5$-LO-Weges ($LTB_4$, $LTC_4$, $LTD_4$) in erhöhten Mengen bei Hautläsionen von Patienten mit Psoriasis und atopischer Dermatitis vorhanden und können Mediatoren dieser Hauterkrankungen darstellen. Die intrakutane Verabreichung von $LTB_4$ in die menschliche Haut führt zu einer Quatelreaktion gefolgt von einer Infiltration neutrophiler Zellen an die Applikationsstelle. Ein Einströmen neutrophiler Zellen wird auch im Verlauf von entzündlichen Reaktionen bei psoriatischen Läsionen beobachtet. Die topische Verabreichung von $LTB_4$ auf der menschlichen Haut verursacht Abszesse ähnlich denen bei pustulärer Psoriasis.

Freie am Sauerstoffatom gebildete Radikale und deren Metaboliten können zu irreversiblen Schäden beitragen, die bei der Neudurchblutung von zuvor ischämischen Myocardgewebe auftreten. Eine Therapie, die darauf gerichtet ist, toxische Effekte dieser freien Radikale durch Verabreichung von Radikalfänger-Arzneimitteln zu vermeiden können Schutz gegen solche Schäden bewirken.

Die Verbindungen der Formel I sind als Antioxidantien und $\Delta^5$-Lipoxygenasehemmer wirksam. Die nützlichen pharmakologischen Eigenschaften der Verbindung der Formel I können durch die nachstehend ausgeführten Testanordnungen gezeigt werden.

Die Verbindungen der Formel I sind als Mittel zur Behandlung von entzündlichen Erkrankungen wie Arthritis, entzündliche Darmerkrankungen wie Colitis, kardiovaskuläre Erkrankungen wie Myocard-Ischämie, als anti-inflammatorische Mittel in der topischen therapeutischen Behandlung von Leukotrien-verursachten dermalen Entzündungen einschliesslich Psoriasis und bei bronchopulmonaren Erkrankungen wie Asthma von Wert.

Entzündliche Darmerkrankungen schliessen eine Vielzahl von Krankheiten des Gastrointestinaltrakts ein wie Morbus Crohn im Colon und Ileum, ulcerative Colitis und Pseudomembran-Colitis. Gemeinsame Symptome dieser Krankheiten sind die Entzündung des betroffenen Gebietes der gastrointestinalen Mucosa, Ulceration der Mucosa, Oedeme, Infiltration der Muscosa mit Entzündungszellen und schwere Diarrhoe. Arachidonsäuremetaboliten aus dem $\Delta^5$-LO-Weg werden als Mediatoren von entzündlichen Darmerkrankungen angenommen.

In vitro Test für $\Delta^5$-Lipoxygenasehemmer

Verbindungen der Formel I wurden auf ihre Wirksamkeit auf die $\Delta^5$-Lipoxygenase von RBL-1 Zellen geprüft. Ein Lipoxygenase (5-LO)-Präparat wurde durch Aufbrechen der Zellen mittels eines Homogenisators und Abzentrifugieren der Zelltrümmer gewonnen. Das Enzympräparat wurde mit der Testverbindung oder dem Vehikel 10 Minuten bei 30 °C inkubiert, danach wurden die Proben bei 37 °C mit Arachidonsäure versetzt, um die 5-LO-Aktivität in Gang zu setzen. Die Werte der durch Enzymreaktion gebildeten Menge von 5-HETE wurde durch einen Radioimmunoassay gemessen. In der Tabelle I sind als $IC_{50}$-Werte

diejenigen Konzentrationen der Testverbindung angegeben, die zu einer 50%igen Hemmung der Bildung von 5-HETE im Vergleich zu den Kontrollwerten führte.

Carrageenan-Pleuritistest in vivo

Die Verbindungen wurden weiterhin im Carrageenan-Pleuritistest an menschlichen Ratten geprüft. Dabei wird in den Versuchstieren eine Pleuritis durch Injektion von 1% lambda Carrageenan in die rechte Pleuralhöhle verursacht. Die Testverbindungen wurden 1 Stunde zuvor (bei einer Gesamttestdauer von 5 Stunden) oder 1 Stunde zuvor und 5 Stunden danach (bei einer 24-Stunden-Testdauer) durch Intubation verabreicht. Die Auswertung des Tests wurde durch Messung des aus der Pleuralhöhle gewonnenen Exsudats vorgenommen. Die mit den erfindungsgemässen Verbindungen erhaltenen Versuchswerte sind in Tabelle I angegeben.

Oedemtest am Mäuseohr in vivo

Die Verbindungen wurden weiterhin im Oedemtest am Mäuseohr getestet. Dabei wird durch Verarbreichung von Arachidonsäure in das Ohr ein Oedem induziert; siehe hierzu J. Invest. Dermatol. 80:48 (1983) und Advances in Inflammation Research, 11:57 (1986). Der Versuch wurde so geführt, dass eine Kontrollgruppe weder Arachidonsäure noch Testverbindungen erhielt, eine zweite Gruppe nur Arachidonsäure enthielt und eine dritte Gruppe zunächst die Testverbindung und danach Arachidonsäure erhielt. Die Testverbindungen wurden in Aceton gelöst, auf die dorsale Oberfläche des Ohrs aufgebracht. Die Arachidonsäure wurde in der gleichen Weise topisch verabreicht. Die Oedembildung wurde durch Auswägen von Gewebeproben ermittelt. Die prozentuale Hemmung der Oedembildung wurde nach der folgenden Formel berechnet:

$$\frac{\text{Probengewicht der Arachidonsäuregruppe - Probengewicht der Testgruppe}}{\text{Probengewicht der Arachidonsäuregruppe - Probengewicht der Kontrollgruppe}} \times 100$$

Die Versuchsdaten sind in Tabelle I angegeben.

Tabelle I

| Verbindung | Hemmung der $\Delta^5$-Lipoxygenase $IC_{50}$ (µM) | % Hemmung des Exsudat-Volumens bei 100 mg/kg p.o. | % Hemmung des Oedems mit 1 mg (topisch) |
|---|---|---|---|
| 4-[4-(2,3-Dihydroxyphenyl)butoxy]-2-hydroxy-3-propylbenzoesäure | 0,09 | 76 | 17 |
| 4-[5-(2,3-Dihydroxyphenyl)pentyloxy]-2-hydroxy-3-propylbenzoesäure | 0,006 | 63 | 0 |
| 4-[6-(2,3-Dihydroxyphenyl)hexyloxy]-2-hydroxy-3-propylbenzoesäure | 0,02 | 68 | 46 |
| 4-[7-(2,3-Dihydroxyphenyl)heptyloxy]-2-hydroxy-3-propylbenzoesäure | 0,005 | 57 | 57 |
| 4-[8-(2,3-Dihydroxyphenyl)octyloxy]-2-hydroxy-3-propylbenzoesäure | 62% mit 0,1 µM | 66 | 78 |
| 4-[10-(2,3-Dihydroxyphenyl)decyloxy]-2-hydroxy-3-propylbenzoesäure | 0,02 | 61 | 49 |
| 4-[6-(2,3-Dihydroxpyhenyl)hexyloxy]-2-hydroxy-3-propylbenzoesäure-äthylester | 100% mit 1 µM | 44 | 64 |

Tabelle I (Forts.)

| Verbindung | Hemmung der $\Delta^5$-Lipoxygenase $IC_{50}$ (µM) | % Hemmung des Exsudat-Volumens bei 100 mg/kg p.o. | % Hemmung des Oedems mit 1 mg (topisch) |
|---|---|---|---|
| 4-[6-(2,3-Dihydroxyphenyl)hexyloxy]-2-hydroxy-3-propylbenzoesäure [2-(diäthylamino)äthyl]ester | 0,67 | 42 | 48 |
| 2-Acetyloxy-4-[6-(2,3-dihydroxyphenyl)-hexyloxy]-3-propylbenzoesäure | 0,19 | 42 | nicht geprüft |
| 4-[6-[2,3-Bis(acetyloxy)phenyl]hexyloxy]-2-hydroxy-3-propylbenzoesäure | 0,15 | 43 (75 mg/kg) | nicht geprüft |
| 4-[6-[2,3-Dihydroxy-4-(1-methyläthyl)-phenyl]hexyloxy]-2-hydroxy-3-propyl-benzoesäure | 0,01 | 47 (75 mg/kg) | 45 |
| 4-[6-(2,3-Dihydroxyphenyl)hexyloxy]-benzoesäure | 0,17 | 46 | 48 |
| 4-[6-(2,3-Dihydroxyphenyl)hexyloxy]-2-hydroxybenzoesäure | 0,32 | 0 | 50 |
| 4-[6-(2,3-Dihydroxyphenyl)hexyloxy]-3-propylbenzoesäure | 0,02 | 0 | 52 |
| 4-[6-(2,3-Dihydroxyphenyl)hexyloxy]-3,5-dipropylbenzoesäure | 0,46 | nicht geprüft | 59 |

Tabelle I (Forts.)

| Verbindung | Hemmung der $\Delta^5$-Lipoxygenase $IC_{50}$ (µM) | % Hemmung des Exsudat-Volumens bei 100 mg/kg p.o. | % Hemmung des Oedems mit 1 mg (topisch) |
|---|---|---|---|
| 4-[3-(3,4-Dihydroxyphenyl)propoxy]-2-hydroxy-3-propylbenzoesäure | 97% mit 1 µM | 55 | 32 |
| 4-[6-(3,4-Dihydroxyphenyl)hexyloxy]-2-hydroxy-3-propylbenzoesäure | 0,005 | 0 | 21 |
| 4-[[6-(3,4-Dihydroxyphenyl)-6-oxohexyl]-oxy]-2-hydroxy-3-propylbenzoesäure | 51% mit 1 µM | nicht geprüft | nicht geprüft |
| 1-[2-Hydroxy-4-[6-(2,3-dihydroxyphenyl)-hexyloxy]-3-propylphenyl]äthanon | 0,03 | 51 | 21 |
| 1-[2-Hydroxy-4-[4-(2,3-dihydroxyphenyl)-butoxy]-3-propylphenyl]äthanon | 48% mit 0,01 µM | 53 | nicht geprüft |
| 1-[2-Hydroxy-4-[8-(2,3-dihydroxyphenyl)-octyloxy]-3-propylphenyl]äthanon | 95% mit 0,1 µM | 0 | 0 |
| 1-[2-Hydroxy-4-[6-[2,3-dihydroxy-4-(1-methyläthyl)phenyl]hexyloxy]-3-propylphenyl]äthanon | 35% mit 0,1 µM | 44 | 64 |
| 5-Chlor-4-[6-(2,3-dihydroxyphenyl)-hexyloxy]-2-hydroxy-3-propylbenzoesäure | 48% mit 0,1 µM | 45 (50 mg/kg) | 51 |

Durch Essigsäure induzierte Colitis bei Ratten in vivo

Die Verbindungen wurden weiterhin in einer Testanordnung geprüft, bei der bei Ratten durch Essigsäure eine Colitis verursacht wird, vgl. Amer. J. Proc. Gastro. Col. Rec. Surg. 31: 11-18 (1980) und Gastroenterology 88: 55-63 (1985) und 86: 453-460 (1984). Die durch Essigsäure induzierte Colitis ist

dadurch charakterisiert, dass inflammatorische Zellen in das Colon einwandern, wobei die Anzahl dieser Zellen durch die Myeloperoxidase-Aktivität gemessen wird. Eine positive Aktivität der Testverbindung zeigt sich in einer Verminderung der hohen Spiegel von Myeloperoxidase. Die Versuchstiere (männliche Ratten) wurden zwei Tage lang zweimal täglich mit entweder dem Vehikel oder der in Wasser suspendierten oder in Dimethylsulfoxid gelösten, oral verabreichten Testverbindung vorbehandelt. Am dritten Tage wurden die Tiere in gleicher Weise behandelt, danach wurden unter Anästhesie 2 ml 2,5%ige Essigsäure in das Colonlumen injiziert, worauf unmittelbar danach mit 3 ml Luft und 3 ml Phosphat-gepufferter Kochsalzlösung nachgespült wurde. Nach 16 Stunden erhielten die Tiere eine weitere Dosis der Testverbindung. Nach 24 Stunden wurde die Colon-Mucosa entfernt und die Myeloperoxidase-Aktivität im Gewebeshomogenat gemessen (siehe The Enzyme Linked Immunosorbent Assay Zoological Soc., London [1979], Seiten 29-30). Die erhaltene Versuchswerte sind in Tabelle II angegeben.

Antibiotika-induzierte Colitis bei Hamstern

In diesem Test erhalten männliche syrisiche Hamster eine Einmaldosis von 175 mg/kg Clindamycin-phosphat oder Clindamycin-hydrochlorid intraperitoneal verabreicht, wobei eine Colitis induziert wird. Etwa 7 Stunden nach der Injektion erhalten die Tiere die Testverbindung oral oder intraperitoneal. Die Therapie wird zweimal täglich während 4 Tagen fortgesetzt. Der Therapieeffekt wird bestimmt durch das Verhältnis der Mortalität der mit der Testverbindung behandelten Tiere zur Mortalität der Tiere, die keine Testverbindung erhalten hatten (Hazard Ratio). Ein Hazard-Ratio von 1,0 bedeutet, dass die Therapie nicht wirksamer war als die Behandlung mit dem Vehikel allein (Kontrollwert). Während ein Hazard-Ratio grösser als 1,0 bedeutet, dass die Ueberlebenszeit im Vergleich zur Kontrollgruppe verlängert wird (vgl. Amer. J. Vet. Res. 39: 1525-1530 [1978]).

Die mit repräsentativen erfindungsgemässen Verbindungen erhaltenen Werte sind in Tabelle II angegeben.

Tabelle II

| Verbindung | Essigsäure-Colitis Dosis (mg/kg p.o.) | Essigsäure-Colitis % Hemmung der Myeloperoxidase | Antibiotika-Colitis Dosis (mg/kg p.o.) | Antibiotika-Colitis Hazard Ratio |
|---|---|---|---|---|
| 4-[4-(2,3-Dihydroxyphenyl)butoxy]-2-hydroxy-3-propylbenzoesäure | 10 | 73±10 | 10 | 1,35 |
| | 30 | 71±11 | 100 | 0,1 |
| 4-[6-(2,3-Dihydroxyphenyl)hexyloxy]-2-hydroxy-3-propylbenzoesäure | 10 | 42± 2 | 10 | 2,26** |
| | 30 | 67±15 | 100 | 4,22** |
| 4-[8-(2,3-Dihydroxyphenyl)octyloxy]-2-hydroxy-3-propylbenzoesäure | 10 | 35± 7 | | |
| | 100 | 27± 3 | | |
| 4-[6-[2,3-Dihydroxy-4-(1-methyläthyl)-phenyl]hexyloxy]-2-hydroxy-3-propylbenzoesäure | 10 | 53±12 | | |
| | 100 | 86± 9 | | |
| 1-[2-Hydroxy-4-[4-(2,3-dihydroxyphenyl)-butoxy]-3-propylphenyl]äthanon | 10 | 46± 7 | | |
| | 30 | 89±11 | | |
| 4-[6-(2,3-Dihydroxyphenyl)hexyloxy]-2-hydroxybenzoesäure | 1 | 82± 9 | | |
| | 3 | 81±15 | | |
| 4-[6-(2,3-Dihydroxyphenyl)hexyloxy]-benzoesäure | 3 | 79± 8 | | |
| | 10 | 89±28 | | |

** $p<0,01$

Tabelle II (Forts.)

Essigsäure-Colitis

| Verbindung | Dosis (mg/kg p.o.) | % Hemmung der Myeloperoxidase |
|---|---|---|
| 4-[6-(2,3-Dihydroxyphenyl) hexyloxy]-2-hydroxy-3-propylbenzoesäure-äthylester | 1<br>3 | 40± 9<br>67±11 |
| 4-[6-(3,4-Dihydroxy-2,5-dimethylphenyl)-hexyloxy]-2-hydroxy-3-propylbenzoesäure | 3<br>10 | 37± 6<br>85±13 |
| 4-[3-(2,3-Dihydroxyphenyl)propoxy]-2-hydroxy-3-propylbenzoesäure | 10 | 54± 5 |
| 4-[5-(2,3-Dihydroxyphenyl)pentyloxy]-2-hydroxy-3-propylbenzoesäure | 1 | 96±13 |
| 5-Chlor-4-[6-(2,3-dihydroxyphenyl)hexyloxy]-2-hydroxy-3-propylbenzoesäure | 30 | 44± 6 |
| 4-[3-(3,4-Dihydroxyphenyl)propoxy]-2-hydroxy-3-propylbenzoesäure | 30 | 39± 5 |
| 4-[2-(3,4-Dihydroxyphenyl))äthoxy]-2-hydroxy-3-propylbenzoesäure | 10 | 56± 6 |
| 4-[[6-(2-Fluor-4,5-dihydroxphenyl)-6-oxohexyl]oxy]-2-hydroxy-3-propylbenzoesäure | 10 | 40± 5 |

**Tabelle II (Forts.)**

Essigsäure-Colitis

| Verbindung | Dosis (mg/kg p.o.) | % Hemmung der Myeloperoxidase |
|---|---|---|
| 1-[4-[6-(2,3-Dihydroxy-4-(1-methyläthyl)-phenyl]hexyloxy-2-hydroxy-3-propylphenyl]äthanon | 10 | 65±10 |
| 1-[4-[5-(3,4-Dihydroxyphenyl)pentyloxy]-2-hydroxy-3-propyl]äthanon | 10 | 52± 6 |
| 4-[5-(2-Chlor-5,6-dihydroxyphenyl)pentyloxy]-2-hydroxy-3-propylbenzoesäure | 3 | 54± 9 |
| 4-[5-(2,3-Dichlor-5,6-dihydroxyphenyl)pentyloxy]-2-hydroxy-3-propylbenzoesäure | 1 | 73± 5 |
| 4-[5-(2,3,4-Trichlor-5,6-dihydroxyphenyl)-pentyloxy]-2-hydroxy-3-propylbenzoesäure | 1 | 115±21 |
| 3-Amino-4-[6-(2,3-dihydroxyphenyl)hexyloxy]-benzoesäure | 1 | 55± 7 |

Antiperoxidative Wirkung in vitro

Diese Versuchsanordnung beruht auf der Anwendung von Hypoxanthin-Xanthin-Oxidase als Generator von freiem Radikal und gereinigten nativen Rattenherz-Membran-Phosphoglycerid als Substrat. Gemessen wird die durch Lipidperoxidation gebildete Menge von Malondialdehyd. Die Versuchsresultate sind in Tabelle III als $IC_{50}$ angegeben, die wie folgt berechnet wurde:

$$\%\ \text{Hemmung der Peroxidation} = 1 - \frac{\text{Drug}_{60'} - \text{Drug}_{0'}}{T_{60'} - T_{0'}} \times 100$$

| | |
|---|---|
| $\text{Drug}_{60'}$ = | Aequivalente Malondialdehyd, die nach 60 Minuten in Gegenwart des freien Radikalbildners und der Testsubstanz gebildet wurden. |
| $\text{Drug}_{0'}$ = | Aequivalente Malondialdehyd, die nach 0 Minuten in Gegenwart des freien Radikalbilders und der Testsubstanz vorhanden waren. |
| $T_{60'}$ = | Aequivalente Malondialdehyd, die in Abwesenheit der Testverbindungen nach 60 Minuten gebildet wurden. |
| $T_{0'}$ = | Endogene Thiobarbitursäure-Reaktivität des Reaktionsgemisches zum Zeitpunkt null. |

Test als Antioxidans im Myocard-Ischämie-Modell bei der Ratte

Bei diesem Testsystem wird ein freie Radikale erzeugendes System (Purin, Xanthinoxidase und Eisenbeladenes Transferrin) in die Coronar-Arterie spontan hypertensiver Ratten infundiert. 24 Stunden nach Verabreichung der Testsubstanzen wurden die Herzen der Versuchstiere isoliert und seziert. Die Infarktgebiete aus dem linken Ventrikel wurden gemessen und als Prozentsatz des gesamten linken ventrikulären Volumens quantifiziert. Versuchswerte aus diesem Versuch sind in der Tabelle III angegeben.

Tabelle III

| Verbindung | Hemmung der Lipid-Peroxidation $IC_{50}$ (µM) | Ischämie Modell 10 mg/kg iv: Infarkte Total | Ischämie Modell 10 mg/kg iv: % Infarktgebiet von Ventrikel |
|---|---|---|---|
| 4-[4-(2,3-Dihydroxyphenyl)butoxy]-2-hydroxy-3-propylbenzoesäure | 0,5 | 2/5 | 8 |
| 4-[5-(2,3-Dihydroxyphenyl)pentyloxy]-2-hydroxy-3-propylbenzoesäure | 0,4 | 4/5 | 15 |
| 4-[6-(2,3-Dihydroxyphenyl)hexyloxy]-2—hydroxy-3-propylbenzoesäure | 0,3 | 4/5 | 8 |
| 4-[7-(2,3-Dihydroxyphenyl)heptyloxy]-2-hydroxy-3-propylbenzoesäure | 0,5 | 3/5 | 18 |
| 4-[8-(2,3-Dihydroxyphenyl)octyloxy]-2-hydroxy-3-propylbenzoesäure | 0,3 | 1/5 | 8 |
| 4-[10-(2,3-Dihydroxyphenyl)decyloxy]-2-hydroxy-3-propylbenzoesäure | 0,4 | 3/3 | 13 |
| 4-[6-[2,3-Dihydroxy-4-(1-methyläthyl)phenyl]-hexyloxy]-2-hydroxy-3-propylbenzoesäure | 0,7 | 1/5 | 5 |

Tabelle III (Forts.)

| Verbindung | Hemmung der Lipid-Peroxidation $IC_{50}$ (µM) | Ischämie Modell 10 mg/kg iv: Infarkte Total | Ischämie Modell 10 mg/kg iv: % Infarktgebiet von Ventrikel |
|---|---|---|---|
| 4-[6-(2,3-Dihydroxyphenyl)hexyloxy]-benzoesäure | > 1 | 2/5 | 19 |
| 4-[6-(2,3-Dihydroxyphenyl)hexyloxy]-2-hydroxybenzoesäure | > 1 | 3/5 | 10 |
| 4-[6-(3,4-Dihydroxyphenyl)hexyloxy]-2-hydroxy-3-propylbenzoesäure | 0,3 | 1/5 (5 mg/kg iv) | 11 |
| 1-[2-Hydroxy-4-[6-(2,3-dihydroxyphenyl)-hexyloxy]-3-propylphenyl]äthanon | 0,7 | 4/4 | 24 |
| 1-[2-Hydroxy-4-[4-(2,3-dihydroxyphenyl)-butoxy]-3-propylphenyl]äthanon | > 1 | 1/5 | 12 |
| 1-[2-Hydroxy-4-[6-[2,3-dihydroxy-4-(1-methyläthyl)phenyl]hexyloxy]-3-propylphenyl]äthanon | 0,7 | 2/5 | 11 |

Die Verbindungen der Formel I oder deren Salze können in an sich bekannter Weise verabreicht werden. Die Verbindungen der Formel I können einzeln oder mit anderen Pharmazeutika oral, parenteral, rektal oder durch Inhalierung, beispielsweise in Form eines Aerosols oder eines mikronisierten Pulvers verabreicht werden. Die orale Verabreichung kann in Form von Tabletten, Kapseln, beispielsweise im Gemisch mit Talk, Stärke, Milchzucker oder anderen inerten Ingredienzien, d.h. pharmazeutisch akzeptablen

Trägern erfolgen, oder in Form von wässrigen Lösungen, Suspensionen oder wässrig-alkoholischen Lösungen, beispielsweise im Gemisch mit Zucker oder Süßstoffen, Geschmackstoffen, Farbstoffen, Verdickungsmitteln und anderen konventionellen pharmazeutischen Excipientien oder in Form von Kügelchen für die orale Verabreichung. Für die parenterale Verabreichung können die Verbindungen als Lösungen oder Suspensionen, beispielsweise als wässrige Lösung oder in Lösung oder Suspension mit Erdnussöl unter Verwendung von Excipientien und üblichen Trägern angewandt werden. Für die Verabreichung als Aerosole können sie in einem geeigneten pharmazeutisch anwendbaren Lösungsmittel, beispielsweise Aethylalkohol oder Kombinationen mischbarer Lösungsmittel und im Gemisch mit einem pharmazeutisch akzeptablen Treibmittel angewandt werden. Solche Aerosole können in einem Druckbehälter mit Zerstäuberventil abgepackt werden. Für die rektale Verabreichung können die Verbindungen als Suppositorium unter Verwendung eines inerten Trägers wie Kakaobutter eingesetzt werden. Für die topische Verabreichung können die Verbindungen der Formel I in Salben, Crèmes, Lotionen and Gele eingearbeitet werden. Lösungen, Salben und Crèmes enthalten im allgemeinen absorbierbare wasser-lösliche oder emulsionsartige Grundlagen wie Petrolatum, Lanolin und Polyäthylenglykol. Lösungen können die Verbindungen der Formel I in einem pharmazeutisch akzeptablen Lösungsmittel wie Polyäthylenglykol enthalten. Geeignete Lotionen sind echte Lösungen, wässrige oder hydroalkoholische Formulierungen, die fein verteilte Partikel enthalten. Lotionen können Suspendierungs- oder Dispergiermittel wie Cellulosederivate, beispielsweise Methylcellulose und Aethylcellulose enthalten. Gele sind halbfeste Präparate, die aus einer Lösung oder Suspension einer Verbindung der Formel I in einem geeigneten wässrigen oder wasserfreien Vehikel unter Verwendung eines Geliermittels wie Carboxypolymethylen erhalten werden können und die danach durch Zusatz von Alkalimetallhydroxiden wie Natriumhydroxiden und Aminen, beispielsweise Polyäthylenkokosamin auf die geeignete Konsistenz gebracht werden. Pharmazeutische Präparate zur topischen Verabreichung einer Verbindung der Formel I können auch konventionelle Inhaltsstoffe wie Konservierungsmittel, Stabilisatoren, Befeuchtungsmittel, Emulgatoren und Puffer in üblichen Mengen enthalten, die den jeweiligen Erfordernissen angepasst sind und durch den Fachmann ohne weiteres ermittelt werden können.

Für die Verabreichung der Verbindung der Formel I in oralen Dosen kommen Dosierungen von 25-1000 mg pro Tag in Betracht, vorzugsweise 25-250 mg entweder als Einzeldosis oder in unterteilten Dosen.

Die nachfolgenden Beispiele erläutern die Erfindung weiter.

## Beispiel 1

Eine Lösung von 195 ml 1,55M Butyllithium in Hexan wurde tropfenweise im Verlauf von 30 Minuten unter Rühren zu 41,4 g 1,2-Dimethoxybenzol in 700 ml wasserfreiem Tetrahydrofuran bei Raumtemperatur und unter Argon gegeben. Das Reaktionsgemisch wurde 4 Stunden unter Rühren auf 40° erwärmt und dann auf -70° gekühlt. Danach wurde eine Lösung von 46 ml 1,6-Dibromhexan in 250 ml wasserfreiem Tetrahydrofuran tropfenweise im Verlauf von 30 Minuten zugegeben. Das Kühlbad wurde entfernt und das Reaktionsgemisch 1 Stunde gerührt und dann 4 Stunden auf 40° erwärmt. Der Hauptteil des Lösungsmittels wurde entfernt, 90 ml 3N Salzsäure zugesetzt und das Produkt mit Hexan extrahiert. Der Extrakt wurde mit Natriumbicarbonatlösung gewaschen, getrocknet und unter vermindertem Druck zu einem Oel eingedampft. Destillation lieferte 1-(6-Bromhexyl)-2,3-dimethoxybenzol als gelbes Oel, Ausbeute 29%, Siedepunkt 125-140°/20 Pa.

Dieses Verfahren wurde für die Herstellung von allen Brom-Zwischenprodukten mit n = 3-10 verwendet.

## Beispiel 2

266 ml 1M Bortribromid in Methylenchlorid wurden tropfenweise im Verlaufe einer Stunde zu einer auf -65° gekühlten Lösung von 40 g 1-(6-Bromhexyl)-2,3-dimethoxybenzol in 800 ml wasserfreiem Methylenchlorid unter Rühren und Argonatmosphäre gegeben. Das Kühlbad wurde dann entfernt und das Reaktionsgemisch 1,5 Stunden gerührt. Nach Kühlen im Eisbad wurden 100 ml Wasser und 50 ml 3N Salzsäure zugesetzt und das Gemisch 2 Stunden gerührt. Die organische Phase wurde abgetrennt, getrocknet und unter vermindertem Druck zu einem Oel eingedampft, das mittels HPLC unter Verwendung von 5% Methanol-Chloroform gereinigt wurde und 34,7 g 1-(6-Bromhexyl)-2,3-dihydroxybenzol als Oel lieferte. Zu diesem Oel wurden 32 ml Benzylchlorid, 46 g Kaliumjodid, 122 g Kaliumcarbonat und 700 ml wasserfreies Aceton gegeben und das Reaktionsgemisch wurde unter Rückfluss 72 Stunden gerührt. Der Feststoff wurde abfiltriert und das Filtrat unter vermindertem Druck zu einem Oel eingedampft, das durch HPLC mit 1% Aethylacetat-Hexan gereinigt wurde und 47 g 1-(6-Jodhexyl)-2,3-bis(phenylmethoxy)benzol als Oel lieferte.

Beispiel 3

Zu 20 g 1,2-Dimethoxybenzol in 300 ml wasserfreiem Tetrahydrofuran wurden bei Raumtemperatur und Argonatmosphäre unter Rühren 90 ml 1,6M Butyllithium in Hexan im Verlauf von 30 Minuten gegeben. Das Reaktionsgemisch wurde unter Rühren auf 40° 4 Stunden erwärmt und dann in einem Eisbad gekühlt. In das durch Eis gekühlte Reaktionsgemisch wurden im Verlauf von 45 Minuten 14 ml Aethylenoxid eindestilliert. Das Reaktionsgemisch wurde dann unter Kühlung weitere 1,5 Stunden gerührt und dann 17 Stunden bei Raumtemperatur. Der Hauptteil des Lösungsmittels wurde unter vermindertem Druck entfernt und der Rückstand mit Wasser versetzt. Das Produkt wurde mit Aether extrahiert und getrocknet und unter vermindertem Druck zu einem Oel eingedampft. Das verbleibende 1,2-Dimethoxybenzol wurde abdestilliert und der Rückstand durch HPLC mit 20% Aethylacetat-Toluol gereinigt, wobei 5 g 1-(2-Hydroxyäthyl)-2,3-dimethoxybenzol erhalten wurden. 5 g dieses Zwischenproduktes wurden in 100 ml wasserfreiem Methylenchlorid gelöst und die Lösung in einem Eisbad gekühlt. Danach wurden nacheinander 7,7 ml Triäthylamin und 2,6 ml Methansulfonylchlorid tropfenweise zugesetzt. Das Reaktionsgemisch wurde unter Eiskühlung 2 Stunden gerührt und dann mit Wasser und Natriumbicarbonatlösung gewaschen, getrocknet und unter vermindertem Druck eingedampft. Man erhielt 7,2 g 1-[(2-Methansulfonyloxy)äthyl]-2,3-dimethoxybenzol als Oel, das ohne weitere Reinigung verwendet wurde.

Beispiel 4

Zu 9 g 1-(6-Bromhexyl)-2,3-dihydroxybenzol in 200 ml wasserfreiem Tetrahydrofuran und 13,7 ml Triäthylamin wurde unter Eiskühlung und Rühren 10,9 ml 4-Methylbenzoylchlorid tropfenweise im Verlauf von 30 Minuten zugesetzt. Nach 30 Minuten wurde das Bad entfernt und es wurde bei Raumtemperatur weitere 2,5 Stunden gerührt. Das Reaktionsgemisch wurde unter vermindertem Druck konzentriert, der Rückstand mit Natriumbicarbont behandelt und das Produkt mit Aether extrahiert. Der getrocknete Extrakt wurde zu einem Oel konzentriert, das durch HPLC mit 10% Aethylacetat-Hexan gereinigt wurde und 15,2 g 1-(6-Bromhexyl)-2,3-bis[(4-methylbenzoyl)oxy]benzol als Oel lieferte.

Beispiel 5

Zu 1 g 1-(6-Bromhexyl)-2,3-dihydroxybenzol in 150 ml Aethylacetat und 15 ml Acetanhydrid wurden 0,03 ml 70%ige Perchlorsäure gegeben. Die Lösung wurde 1,5 Stunden bei Raumtemperatur belassen und dann mit Natriumbicarbonatlösung gewaschen. Nach Trocknen wurde die organische Phase konzentriert und lieferte 1,3 g 1-(6-Bromhexyl)-2,3-bis(acetyloxy)benzol als Oel.

Beispiel 6

Zu 3,3 g 3,4-Dimethoxyphenäthylalkohol in 50 ml Methylenchlorid und 4,2 ml Triäthylamin wurde unter Kühlen in einem Eisbad 1,6 ml Methansulfonylchlorid unter Rühren zugesetzt. Das Reaktionsgemisch wurde 75 Minuten gerührt und dann nacheinander mit Wasser, 1N Salzsäure und Natriumbicarbonatlösung gewaschen. Nach Trocknen wurde der Extrakt unter vermindertem Druck konzentriert und lieferte 1-[(2-Methansulfonyloxy)äthyl]-2,3-dimethoxybenzol als Oel.

Beispiel 7

Ein Gemisch von 1 ml 1,2-Dimethoxybenzol und 2 g 6-Bromhexansäure wurde kurz bis zur Homogenität gewärmt und gerührt, wobei 1,7 ml Trifluoracetanhydrid zugesetzt wurden. Das Reaktionsgemisch wurde 17 Stunden bei Raumtemperatur gerührt und dann in Natriumbicarbonatlösung gegossen. Das Produkt wurde mit Aethylacetat extrahiert und der getrocknete Extrakt zu einem Oel konzentriert, das durch Chromatographie an 150 g Silicagel gereinigt wurde. Elution mit 25% Aethylacetat-Hexan lieferte 1,6 g 1-(6-Brom-1-oxohexyl)-3,4-dimethoxybenzol.

Beispiel 8

Ein Gemisch von 10 g 1-Brom-3,4-dimethoxybenzol, 3,4 g 3-Butin-1-ol und 8 ml Triäthylamin in 20 ml Methylenchlorid wurde unter Begasung mit Argon gerührt. Das Gemisch wurde mit 0,12 g Kupfer-(I)-jodid und 0,3 g Bis(triphenylphosphin)palladiumdichlorid versetzt. Das Reaktionsgemisch wurde 4 Stunden bei Raumtemperatur gerührt und 16 Stunden unter Rückfluss. Nach Filtration wurde das Filtrat mit Wasser

gewaschen, getrocknet und eingedampft. Das Rohprodukt wurde durch HPLC mit 30% Aethylacetat-Toluol gereinigt und lieferte 3 g 4-(3,4-Dimethoxyphenyl)-3-butin-1-ol.

Beispiel 9

Ein Gemisch von 2 g 4-(3,4-Dimethoxyphenyl)-3-butin-1-ol und 0,2 g 10% Palladium/Kohle in 40 ml Aethanol wurde 4 Stunden unter Wasserstoffatmosphäre gerührt. Nach Filtration wurde das Filtrat unter vermindertem Druck konzentriert und lieferte 1,9 g 4-(3,4-Dimethoxyphenyl)-butan-1-ol als Oel.

Beispiel 10

Zu 0,8 g in kleine Stücke geschnittenem Lithiumband in 50 ml wasserfreiem Aether wurde unter Rühren bei Raumtemperatur und unter Argonatmosphäre 12 g 3-Brom-propan-1-ol-1-äthoxyäthyläther [J. Org. Chem. 37, 1947 (1972)] gegeben. Nach Zusatz von etwa 1 ml wurde das Reaktionsgemisch in einem Eis-Kochsalz-Bad gekühlt und der Rest der Bromverbindung tropfenweise im Verlauf von 35 Minuten zugesetzt. Danach wurde weitere 1,5 Stunden unter Kühlen gerührt. Dann wurden 7,5 g 2,3-Dimethoxybenzaldehyd in 45 ml wasserfreiem Aether tropfenweise im Verlauf von 30 Minuten zugegeben. Nach 1 Stunde wurde das Kühlbad entfernt und es wurde eine weitere Stunde bei Raumtemperatur gerührt. Das Reaktionsgemisch wurde in halbgesättigte Ammoniumsulfatlösung gegossen. Die Aetherschicht wurde abgetrennt, mit Natriumsulfat getrocknet und zu einem Oel konzentriert. Danach wurden 25 ml Aethanol, 25 ml Wasser und 2 ml konzentrierte Salzsäure zugesetzt und die Lösung bei Raumtemperatur 35 Minuten stehen gelassen. Danach wurde Kaliumcarbonat unter Rühren zugesetzt, bis das Gemisch basisch war. Das Aethanol wurde unter vermindertem Druck entfernt und das Produkt mit Aethylacetat extrahiert. Der getrocknete Extrakt wurde zu einem Oel konzentriert. Dieses Oel wurde in 150 ml Aethanol gelöst, mit 1 g 10% Palladium/Kohle versetzt und das Gemisch einem Parr-Hydrierapparat unter einem anfänglichen Wasserstoffdruck von 3,75 bar 5 Stunden geschüttelt. Das Reaktionsgemisch wurde durch Celit filtriert und das Filtrat zu einem Oel konzentriert. Reinigung durch HPLC mit 30% Aethylacetat-Hexan lieferte 7,45 g 4-(2,3-Dimethoxyphenyl)-butan-1-ol.

Beispiel 11

Ein Gemisch von 102 g Methyl-2,3-dihydroxybenzoat, 54 ml Allylbromid und 126 g wasserfreies Kaliumcarbonat in 300 ml wasserfreiem Aceton wurde unter Rückfluss 3 Stunden gerührt. Das Reaktionsgemisch wurde filtriert und der Feststoff mit Aceton gewaschen. Nach Entfernen des Acetons aus dem Filtrat unter vermindertem Druck wurde der Rückstand destilliert und lieferte 85 g 2-Hydroxy-4-(2-propenyloxy)-benzoesäure-methylester, Siedepunkt 106-108°/40 Pa.

Beispiel 12

81 g 2-Hydroxy-4-(2-propenyloxy)benzoesäure-methylester wurden in einem Oelbad unter Argon erwärmt, bis die Innentemperatur 180-185° erreichte. Die Temperatur wurde in diesem Bereich 1,5 Stunden gehalten und dann 1,5 Stunden auf 210° erhöht. Nach Kühlen kristallisierte das Oel und wurde aus Aether-Petroläther umkristallisiert, wobei 37 g 2,4-Dihydroxy-3-(2-propenyl)benzoesäure-methylester, Smp. 65-66° erhalten wurden.

Beispiel 13

Eine Lösung von 54 g 2,4-Dihydroxy-3-(2-propenyl)benzoesäure-methylester in 900 ml Aethanol und 3 g 10% Palladium/Kohle wurde 45 Minuten in einer Wasserstoffatmosphäre geschüttelt, bis die Aufnahme aufhörte. Der Katalysator wurde über Celit abfiltriert und das Filtrat konzentriert. Das zurückbleibende Oel verfestigte sich. Nach Rühren mit Hexan wurde das Produkt filtriert und lieferte 51 g 2,4-Dihydroxy-3-propylbenzoesäure-methylester, Smp. 66-68°.

Beispiel 14

Eine Lösung von 37 g 2,4-Dihydroxy-3-propylbenzoesäure-methylester in 750 ml Methanol und 415 ml 3N Natriumhydroxid wurde unter Rückfluss 3 Stunden gerührt. Das Methanol wurde unter vermindertem Druck entfernt und der Rückstand mit Wasser und 6N Salzsäure bis zur sauren Reaktion versetzt. Der

Feststoff wurde mit Aethylacetat extrahiert und der Extrakt getrocknet und zu einem braunen Rückstand konzentriert, der ohne weitere Reinigung verwendet wurde. Diese rohe Säure (35 g) wurde mit 23 ml Benzylchlorid und 17 g Natriumbicarbonat in 250 ml wasserfreiem Dimethylformamid 23 Stunden unter Rühren auf 60° erwärmt. Das Lösungsmittel wurde unter vermindertem Druck entfernt und der Rückstand mit gesättigter Natriumbicarbonatlösung versetzt und das Produkt mit Aethylacetat extrahiert. Der getrocknete Extrakt wurde unter vermindertem Druck eingedampft und das zurückbleibende Oel durch HPLC mit 15% Aethylacetat-Hexan gereinigt. Man erhielt 36 g 2,4-Dihydroxy-3-propylbenzoesäure-phenylmethylester, Smp. 86-88°.

Beispiel 15

Eine Lösung von 2,1 g 2,4-Dihydroxy-3-propylbenzoesäure-methylester und 1,6 g N-Chlorsuccinimid in 50 ml Tetrachlorkohlenstoff wurde unter Rühren 9,5 Stunden zum Rückfluss erhitzt. Weitere 1,6 g N-Chlorsuccinimid wurden zugesetzt und es wurde weitere 17 Stunden zum Rückfluss erhitzt. Danach wurden 0,8 g N-Chlorsuccinimid versetzt und es wurde weitere 8 Stunden am Rückfluss erhitzt. Nach Zusatz von Wasser wurde die organische Phase abgetrennt und mit Natriumthiosulfatlösung und Natriumbicarbonatlösung gewaschen, getrocknet und unter vermindertem Druck eingedampft. Man erhielt 5-Chlor-2,4-dihydroxy-3-propylbenzoesäure-methylester, Smp. 75-76° (aus Hexan).

Beispiel 16

Ein Gemisch von 29,2 g 1-(6-Bromhexyl)-2,3-dimethoxybenzol, 18,5 g 2,4-dihydroxy-3-propylbenzoesäure-methylester, 18,2 g wasserfreies Kaliumcarbonat und 21,9 g Kaliumjodid in 550 ml wasserfreiem Aceton wurde 22 Stunden unter Rühren zum Rückfluss erhitzt. Das Reaktionsgemisch wurde filtriert und das Filtrat unter vermindertem Druck zu einem Oel konzentriert, das durch HPLC mit 8% Aethylacetat-Hexan gereinigt wurde. Man erhielt 31,4 g 4-[6-(2,3-Dimethoxyphenyl)hexyloxy]-2-hydroxy-3-propylbenzoesäure-methylester als Oel.

In Analogie zu dem Verfahren von Beispiel 16 wurden die Verbindungen von Beispiel 17-23 hergestellt.

Beispiel 17

4-[6-(2,3-Dimethoxyphenyl)äthoxy]-2-hydroxy-3-propylbenzoesäure-methylester, Smp. 77-80°.

Beispiel 18

4-[6-(2,3-Dimethoxyphenyl)propoxy]-2-hydroxy-3-propylbenzoesäure-methylester, Smp. 51-53°.

Beispiel 19

4-[6-(2,3-Dimethoxyphenyl)butoxy]-2-hydroxy-3-propylbenzoesäure-methylester, Oel.

Beispiel 20

4-[6-(2,3-Dimethoxyphenyl)heptyloxy]-2-hydroxy-3-propylbenzoesäure-benzylester, Oel.

Beispiel 21

4-[6-(2,3-Dimethoxyphenyl)octyloxy]-2-hydroxy-3-propylbenzoesäure-methylester, Oel.

Beispiel 22

4-[6-(2,3-Dimethoxyphenyl)decyloxy]-2-hydroxy-3-propylbenzoesäure-benzylester, Oel.

Beispiel 23

4-[6-(2,3-Dimethoxy-4-isopropylphenyl)hexyloxy]-2-hydroxy-3-propylbenzoesäure-benzylester, Oel.

Beispiel 24

Eine Lösung von 31,4 g 4-[6-(2,3-Dimethoxyphenyl)hexyloxy]-2-hydroxy -3-propylbenzoesäure-methylester in 800 ml Methanol und 365 ml 1N Natriumhydroxid wurde 1,5 Stunden unter Rühren zum Rückfluss erhitzt. Das Methanol wurde unter vermindertem Druck entfernt, der Rückstand angesäuert und das Produkt mit Methylenchlorid extrahiert. Der getrocknete Extrakt wurde unter vermindertem Druck konzentriert und lieferte einen Feststoff, der aus Aether-hexan kristallisiert, 24,8 g 4-[6-(2,3-Dimethoxyphenyl)hexyloxy]-2-hydroxy-3-propylbenzoesäure, Smp. 115-118° lieferte.

In Analogie zu dem Verfahren von Beispiel 24 wurden die Verbindungen von Beispiel 25-30 hergestellt.

Beispiel 25

4-[6-(2,3-Dimethoxyphenyl)äthoxy]-2-hydroxy-3-propylbenzoesäure, Smp. 154-156°.

Beispiel 26

4-[6-(2,3-Dimethoxyphenyl)propoxy]-2-hydroxy-3-propylbenzoesäure, Smp. 133-134°.

Beispiel 27

4-[6-(2,3-Dimethoxyphenyl)butoxy]-2-hydroxy-3-propylbenzoesäure, Smp. 111-113°.

Beispiel 28

4-[6-(2,3-Dimethoxyphenyl)heptyloxy]-2-hydroxy-3-propylbenzoesäure, Smp. 98-100°.

Beispiel 29

4-[6-(2,3-Dimethoxyphenyl)octyloxy]-2-hydroxy-3-propylbenzoesäure, Smp. 90-92°.

Beispiel 30

4-[6-(2,3-Dimethoxyphenyl)decyloxy]-2-hydroxy-3-propylbenzoesäure, Smp. 77-78°.

Beispiel 31

Eine Lösung von 6,96 g 2-Hydroxy-4-[6-(2,3-dimethoxy-4-(1-methyläthyl)phenyl]hexyloxy] -3-propylbenzoesäure-phenylmethylester in 150 ml Aethylacetat und 1,4 g 10% Palladium/Kohle wurde in einer Wasserstoffatmosphäre 3 Stunden gerührt. Das Reaktionsgemisch wurde durch ein Celit-Filterbett filtriert und das Filtrat unter vermindertem Druck eingedampft. Man erhielt 5,45 g 2-Hydroxy-4-[6-[2,3-dimethoxy-4-(1-methyläthyl)phenyl]hexyloxy] -3-propylbenzoesäure, Smp. 106-108°.

Beispiel 32

Zu 5 g 4-[6-(2,3-Dimethoxyphenyl)hexyloxy]-2-hydroxy-3-propylbenzoesäure, die in 250 ml wasserfreiem Methlenchlorid suspendiert waren, wurde unter Kühlung bei -70° 36 ml 1M Bortribromid in Methylenchlorid tropfenweise im Verlauf von 30 Minuten gegeben. Das Reaktionsgemisch wurde 30 Minuten bei -70° gerührt und dann 17 Stunden bei -18° stehen gelassen. Danach wurden 150 ml Wasser unter Rühren tropfenweise zugesetzt und das Produkt wurde mit Aether extrahiert. Der Extrakt wurde unter vermindertem Druck konzentriert, der Rückstand in 500 ml Aether aufgenommen und kräftig mit 125 ml 1N Salzsäure geschüttelt. Der Extrakt wurde getrocknet und unter vermindertem Druck zu einem Feststoff eingedampft. Umkristallisation aus Aether-Hexan lieferte 3,7 g 4-[6-(2,3-Dihydroxpyhenyl)hexyloxy]-2-hydroxy -3-propylbenzoesäure Smp. 147-150°.

In Analogie zu dem Verfahren von Beispiel 32 wurden die Verbindungen von Beispiel 32-39 hergestellt.

Beispiel 33

4-[6-(2,3-Dihydroxyphenyl)äthoxy]-2-hydroxy-3-propylbenzoesäure, Smp. 184-189°.

Beispiel 34

4-[6-(2,3-Dihydroxyphenyl)propoxy]-2-hydroxy-3-propylbenzoesäure, Smp. 189-191°.

Beispiel 35

4-[6-(2,3-Dihydroxyphenyl)butoxy]-2-hydroxy-3-propylbenzoesäure, Smp. 160-162°.

Beispiel 36

4-[6-(2,3-Dihydroxyphenyl)heptyloxy]-2-hydroxy-3-propylbenzoesäure, Smp. 144-146°.

Beispiel 37

4-[6-(2,3-Dihydroxyphenyl)octyloxy]-2-hydroxy-3-propylbenzoesäure, Smp. 136-139°.

Beispiel 38

4-[6-(2,3-Dihydroxyphenyl)decyloxy]-2-hydroxy-3-propylbenzoesäure, Smp. 126-128°.

Beispiel 39

4-[6-(2,3-Dihydroxy-4-isopropylphenyl)hexyloxy]-2-hydroxy-3-propylbenzoesäure, Smp. 104-105°.

Beispiel 40

Ein Gemisch von 6,88 g 1-(6-Jodhexyl)-2,3-diphenylmethoxybenzol, 3,6 g 2,4-Dihydroxy-3-propylbenzoesäurephenylmethylester, 2,6 g wasserfreies Kaliumcarbonat und 3,1 g Kaliumjodid und 150 ml wasserfreiem Aceton wurde 42 Stunden unter Rühren zum Rückfluss erhitzt. Das Reaktionsgemisch wurde abfiltriert und das Filtrat unter vermindertem Druck konzentriert. Der Rückstand wurde durch HPLC mit 10% Aethylacetat-Hexan gereinigt und lieferte 7,37 g 2-Hydroxy-4-[5-[2,3-bis(phenylmethoxy)pentyloxy] -3-propylbenzoesäure-phenylmethylester als Oel, das sich verfestigte, Smp. 65-69°.

Eine Lösung von 7,2 g des vorstehenden Phenylmethylesters in 500 ml Tetrahydrofuran und 1,4 g 10% Palladium/Kohle wurde 25 Stunden unter Wasserstoffatmosphäre gerührt. Das Reaktionsgemisch wurde durch ein Celit-Filterbett filtriert und das Filtrat unter vermindertem Druck zu einem Feststoff konzentriert, der aus Aether-Hexan umkristallisiert wurde und 3,8 g 4-[5-(2,3-Dihydroxyphenyl)pentyloxy]-2-hydroxy-3-propylbenzoesäure, Smp. 155-157° lieferte.

Beispiel 41

Ein Gemisch von 1,4 g 1-(6-Bromhexyl)-2,3-dimethoxybenzol, 1 g 4-Hydroxy-3-propylbenzoesäureäthylester, 1,3 g Kaliumcarbonat und 0,72 g Natriumjodid in 35 ml Aceton wurde 47 Stunden zum Rückfluss erhitzt. Aufarbeitung und Reinigung wie in Beispiel 16 lieferte 2 g 4-[6-(2,3-Dimethoxyphenyl)hexyloxy] -3-propylbenzoesäure-äthylester als Oel.

Beispiel 42

Eine Lösung von 2 g 4-[6-(2,3-Dimethoxyphenyl)hexyloxy]-3-propylbenzoesäure-äthylester in 70 ml Methanol und 24 ml 1N Natriumhydroxid wurde 3 Stunden unter Rühren zum Rückfluss erhitzt. Aufarbeitung wie in Beispiel 24 lieferte 1,87 g 4-[6-(2,3-Dimethoxyphenyl)hexyloxy]-3-propylbenzoesäure vom Smp. 107-108°.

Beispiel 43

Zu 1,8 g 4-[6-(2,3-Dimethoxyphenyl)hexyloxy]-3-propylbenzoesäure in 125 ml Methylenchlorid wurden unter Kühlung auf -70° 14 ml 1M Bortribromid in Methylenchlorid gegeben. Nach 30 Minuten bei -70° und 5 Stunden bei -20° wurde das Reaktionsgemisch wie in Beispiel 32 aufgearbeitet und das Produkt aus Aether-Hexan umkristallisiert. Man erhielt 1,12 g 4-[6-(2,3-Dihydroxyphenyl)hexyloxy]-3-propylbenzoesäure

vom Smp. 123-124°.

Beispiel 44

Ein Gemisch von 1,20 g 1-(6-Bromhexyl)-2,3-dimethoxybenzol, 1 g 3,5-Dipropyl-4-hydroxybenzoesäure-äthylester, 1,1 g Kaliumcarbonat und 0,6 g Natriumjodid in 35 ml Aceton wurde 47 Stunden unter Rühren zum Rückfluss erhitzt. Aufarbeitung und Reinigung wie in Beispiel 16 lieferte 4-[6-(2,3-Dimethoxyphenyl)-hexyloxy] -3,5-dipropylbenzoesäure-äthylester als Oel.

Beispiel 45

Eine Lösung von 1,8 g 4-[6-(2,3-Dimethoxyphenyl)hexyloxy]-3,5-dipropylbenzoesäure-äthylester in 100 ml Methanol und 20 ml 1N Natriumhydroxid wurde 3 Stunden unter Rühren zum Rückfluss erhitzt. Aufarbeitung wie in Beispiel 24 lieferte 4-[6-(2,3-Dimethoxyphenyl)hexyloxy]-3,5-dipropylbenzoesäure, Smp. 61-65°.

Beispiel 46

Zu 1,7 g 4-[6-(2,3-Dimethoxyphenyl)hexyloxy]-3,5-dipropylbenzoesäure in 15 ml Methylenchlorid wurde bei -70° 14 ml 1M Bortribromid in Methylenchlorid gegeben. Nach 30 Minuten bei -70° und 5 Stunden bei -20° wurde das Reaktionsgemisch wie in Beispiel 32 aufgearbeitet und das Produkt aus Aether-Hexan umkristallisiert. Man erhielt 0,27 g 4-[6-(2,3-Dihydroxyphenyl)hexyloxy]-3,5-dipropylbenzoesäure vom Smp. 94-96°.

Beispiel 47

Ein Gemisch von 0,90 g 4-[6-(2,3-Dihydroxyphenyl)hexyloxy]-2-hydroxy -3-propylbenzoesäure, 1,9 ml Aethyljodid und 0,21 g Natriumbicarbonat in 10 ml wasserfreiem Dimethylformamid wurde 6 Stunden unter Rühren auf 50° erwärmt. Das Lösungsmittel wurde an der Oelpumpe entfernt, der Rückstand mit Natriumbicarbonatlösung versetzt und das Produkt mit Aethylacetat extrahiert. Der getrocknet Extrakt wurde konzentriert und der Rückstand an 35 g Silicagel chromatogrpahiert. Elution mit 10% Aethylacetat-Toluol lieferte 0,89 g eines Oels, das mit Hexan gerührt wurde und nach Filtration 0,71 g 4-[6-(2,3-Dihydroxyphenyl)hexyloxy]-2-hydroxy-3-propylbenzoesäure-äthylester, Smp. 54-57° lieferte.

Beispiel 48

Ein Gemisch von 1 g 4-[6-(2,3-Dihydroxyphenyl)hexyloxy]-2-hydroxy -3-propylbenzoesäure, 3,5 g 2-Diäthylaminoäthylchlorid und 0,24 g Natriumbicarbonat in 20 ml wasserfreiem Dimethylformamid wurde 1,5 Stunden unter Rühren auf 50° erwärmt. Das Lösungsmittel wurde an der Oelpumpe entfernt. Der Rückstand wurde mit Natriumbicarbonatlösung versetzt und das Produkt mit Aethylacetat extrahiert. Der getrocknete Extrakt wurde konzentriert und der Rückstand an 50 g Silicagel chromatographiert. Elution mit Methylenchlorid-95% Methanol: Ammoniumhydroxid (95:5:0,05) lieferte 1 g der freien Base 4-[6-(2,3-Dihydroxyphenyl)hexyloxy]-2-hydroxy-3-propylbenzoesäure [2-(diäthylamino)äthyl]ester. Diese wurde in Methylenchlorid gelöst und mit 3,2 ml 2M Salzsäure in Aethanol versetzt. Nach Einengen und Zusatz von Hexan wurden 0,91 g 4-[6-(2,3-Dihydroxyphenyl)hexyloxy]-2-hydroxy-3-propylbenzoesäure [2-(diäthylamino)äthyl]ester-hydrochlorid, Smp. 98-100° erhalten.

Beispiel 49

Ein Gemisch von 1,91 g 1-[6-Bromhexyl)-2,3-bis[(4-methylbenzoyl)oxy]benzol, 1,07 g 2,4-Dihydroxy-3-propylbenzoesäure-phenylmethylester, 0,94 g Kaliumjodid und 0,75 g Kaliumcarbonat in 40 ml Aceton wurde 26 Stunden unter Rühren zum Rückfluss erhitzt. Das Reaktionsgemisch wurde filtriert und das Filtrat unter vermindertem Druck eingeengt. Das Rohprodukt wurde durch HPLC mit 5% Aethylacetat-Hexan gereinigt und lieferte 0,85 g 2-Hydroxy-4-[6-[2,3-bis[(4-methylbenzoyl)oxy]phenyl]hexyloxy] -3-propylbenzoesäure-phenylmethylester als Oel.

Beispiel 50

Eine Lösung von 0,78 g 2-Hydroxy-4-[6-[2,3-bis[(4-methylbenzoyl)oxy]phenyl]hexyloxy] -3-propylbenzoesäure-phenylmethylester in 65 ml Tetrahydrofuran und 0,16 g 10% Palladium/Kohle wurde 3 Stunden unter Wasserstoffatmosphäre geschüttelt. Das Reaktionsgemisch wurde über Celit abfiltriert und das Filtrat unter vermindertem Druck zu einem Feststoff konzentriert, der aus Methylenchlorid-Hexan umkristallisiert wurde. Man erhielt 0,57 g 2-Hydroxy-4-[6-[2,3-bis[4-methylbenzoyl)oxy]phenyl]hexyloxy] -3-propylbenzoesäure vom Smp. 141-143°.

Beispiel 51

Zu einer Suspension von 0,14 g Natriumhydrid (60% in Oel) in 10 ml wasserfreiem Dimethylformamid wurde unter Rühren bei Raumtemperatur 0,88 g 2,4-Dihydroxy-3-propylbenzoesäure-phenylmethylester gegeben. Das Reaktionsgemisch wurde 2 Stunden gerührt und dann tropfenweise mit 1,09 g 1-(6-Bromhexyl)-2,3-bis(acetyloxy)benzol in 10 ml Dimethylformamid versetzt. Danach wurde 16 Stunden bei 50° weitergerührt und das Lösungsmittel dann an der Oelpumpe entfernt. Das Rohprodukt wurde durch HPLC mit 25% Aethylacetat-Hexan gereinigt und lieferte 0,55 g 4-[6-[2,3-Bis(acetyloxy)phenyl]hexyloxy]-2-hydroxy -3-propylbenzoesäure-phenylmethylester als Oel.

Beispiel 52

Eine Lösung von 0,53 g 4-[6-[2,3-Bis(acetyloxy)phenyl]hexyloxy]-2-hydroxy -3-propylbenzoesäure-phenylmethylester in 50 ml Tetrahydrofuran und 0,1 g 10% Palladium/Kohle wurde 3 Stunden unter Wasserstoffatmosphäre geschüttelt. Das Reaktionsgemisch wurde über Celit abfiltriert und das Filtrat zu einem Feststoff eingeengt, der nach Umkristallisation aus Aether-Hexan 0,35 g 4-[6-[2,3-Bis(acetyloxy)phenyl]-hexyloxy]-2-hydroxy -3-propylbenzoesäure, Smp. 120-122° lieferte.

Beispiel 53

Ein Gemisch von 1,31 g 1-(6-Jodhexyl)-2,3-bis(phenylmethoxy)benzol, 0,74 g 2,4-Dihydroxy-3-propylbenzoesäure-phenylmethylester und 0,54 g Kaliumcarbonat in 35 ml Aceton wurde 39 Stunden unter Rühren zum Rückfluss erhitzt. Das Reaktionsgemisch wurde filtriert und das Filtrat unter vermindertem Druck zu einem Oel eingeengt, das durch HPLC mit 8% Aethylacetat-Hexan gereinigt wurde. Man erhielt 1,03 g 2-Hydroxy-4-[6-[2,3-bis(phenylmethoxy)phenyl]hexyloxy] -3-propylbenzoesäure-phenylmethylester als Oel.

Beispiel 54

Eine Lösung von 1,15 g 2-Hydroxy-4-[6-[2,3-bis(phenylmethoxy)phenyl]hexyloxy] -3-propylbenzoesäure-phenylmethylester in 15 ml Pyridin und 15 ml Acetanhydrid wurde 15 Stunden unter Rühren zum Rückfluss erhitzt. Das Reaktionsgemisch wurde an der Oelpumpe konzentriert. Der Rückstand wurde in Aethylacetat gelöst, die Lösung mit Natriumbicarbonatlösung gewaschen, getrocknet und unter vermindertem Druck eingedampft, wobei 1,03 g 2-Acetyloxy-4-[6-[2,3-bis(phenylmethoxy)phenyl]hexyloxy] -3-propylbenzoesäure-phenylmethylester als Oel erhalten wurden.

Beispiel 55

Eine Lösung von 1,02 g 2-Acetyloxy-4-[6-[2,3-bis(phenylmethoxy)phenyl]hexyloxy] -3-propylbenzoesäure-phenylmethylester in 65 ml Tetrahydrofuran und 0,2 g 10% Palladium/Kohle wurde 24 Stunden unter Wasserstoffatmosphäre gerührt. Das Reaktionsgemisch wurde durch Celit filtriert und das Filtrat eingeengt. Der Rückstand wurde aus Aether-Hexan kristallisiert und lieferte 0.51 g 2-Acetyloxy-4-[6-(2,3-dihydroxyphenyl)hexyloxy] -3-propylbenzoesäure, Smp. 130-132°.

Beispiel 56

Ein Gemisch von 0,41 g 1-(6-Jodhexyl)-2,3-bis(phenylmethoxy)benzol, 0,18 g 5-Chlor-2,4-dihydroxypropylbenzoesäure-methylester und 0,22 g Kaliumcarbonat in 15 ml Aceton wurde 16 Stunden unter Rühren zum Rückfluss erhitzt. Das Reaktionsgemisch wurde filtriert und das Filtrat zu einem Oel

eingeengt. Chromatographie an 30 g Silicagel und Elution mit 10% Aethylacetat-Hexan lieferte 0,31 g 5-Chlor-2-hydroxy-4-[6-[2,3-bis(phenylmethoxy)phenyl]hexyloxy] -3-propylbenzoesäure-methylester als Oel.

Beispiel 57

Eine Lösung von 0,30 g 5-Chlor-2-hydroxy-4-[6-[2,3-bis(phenylmethoxy)phenyl]hexyloxy] -3-propylbenzoesäure-methylester in 10 ml Methanol, 5 ml Dioxan und 2,5 ml 1N Natronlauge wurde 3 Tage bei Raumtemperatur stehengelassen. Das Lösungsmittel wurde unter vermindertem Druck entfernt, der Rückstand angesäuert und mit Aethylacetat extrahiert. Der getrocknete Extrakt wurde konzentriert und an 30 g Silicagel mit Essigsäure:Aethylacetat:Toluol (1:25:75) chromatographiert. Man erhielt 0,21 g 5-Chlor-2-hydroxy-4-[6-[2,3-bis(phenylmethoxy)phenyl]hexyloxy] -3-propylbenzoesäure als Oel.

Beispiel 58

Eine Lösung von 0,21 g 5-Chlor-2-hydroxy-4-[6-[2,3-bis(phenylmethoxy)phenyl]hexyloxy] -3-propylbenzoesäure in 30 ml Aethylacetat und 0,073 g 10% Palladium/Kohle wurde 21 Stunden unter Wasserstoff geschüttelt (2-2,7 bar). Das Reaktionsgemisch wurde über Celit abfiltriert und das Filtrat zu einem Oel konzentriert. Chromatographie auf 20 g Silicagel und Elution mit Essigsäure:Aethylacetat:Toluol (5:20:75) lieferte 82 mg 5-Chlor-2-hydroxy-4-[6-(2,3-dihydroxyphenyl)hexyloxy] -3-propylbenzoesäure, Smp. 110-113°.

Beispiel 59

Ein Gemisch von 1 g 1-(6-Bromhexyl)-2,3-dimethoxybenzol, 0.55 g 2,4-Dihydroxybenzoesäure-methylester, 1,2 g Kaliumcarbonat und 0,75 g Kaliumjodid in 25 ml Aceton wurde 20 Stunden unter Rühren zum Rückfluss erhitzt. Aufarbeitung wie in Beispiel 16 lieferte 1,2 g 4-[6-(2,3-Dimethoxyphenyl)hexyloxy] -2-hydroxy-benzoesäure-methylester als Oel.

Beispiel 60

Eine Lösung von 0,45 g 4-[6-(2,3-Dimethoxyphenyl)hexyloxy] -2-hydroxy-benzoesäure-methylester in 25 ml Methanol und 8 ml 1N Natronlauge wurde 7 Stunden zum Rückfluss erhitzt. Aufarbeitung wie in Beispiel 24 und Umkristallisation aus Methanol lieferte 0,36 g 4-[6-(2,3-Dimethoxyphenyl)hexyloxy]-2-hydroxybenzoesäure, Smp. 115-116°.

Beispiel 61

Zu 0,35 g 4-[6-(2,3-Dimethoxyphenyl)hexyloxy]-2-hydroxybenzoesäure in 10 ml Methylenchlorid wurde unter Rühren und Kühlung auf -70° 3,5 ml 1M Bortribromid in Methylenchlorid gegeben. Das Reaktionsgemisch wurde 20 Minuten bei -70° gerührt und 6,5 Stunden bei -20°. Aufarbeitung wie in Beispiel 32 und Umkristallisation aus Methanol lieferte 0,2 g 4-[6-(2,3-Dihydroxyphenyl)hexyloxy]-2-hydroxy-benzoesäure, Smp. 170-180°.

Beispiel 62

Ein Gemisch von 1 g 1-(6-Jodhexyl)-2,3-bis(phenylmethoxy)benzol, 0,34 g 2,5-Dihydroxybenzoesäure-methylester und 1 g Kaliumcarbonat in 30 ml Aceton wurde 17 Stunden zum Rückfluss erhitzt. Aufarbeitung wie in Beispiel 16 und Chromatographie an 60 g Silicagel mit 1% Aethylacetat-Toluol lieferte 0,42 g 2-Hydroxy-5-[6 -[2,3-bis(phenylmethoxy)phenyl]hexyloxy]benzoesäure-methylester als Oel.

Beispiel 63

Eine Lösung von 0,42 g 2-Hydroxy-5-[6 -[2,3-bis(phenylmethoxy)phenyl]hexyloxy]benzoesäure-methylester in 12 ml Aethanol und 4 ml 1N Natronlauge wurde 1,5 Stunden unter Rühren zum Rückfluss erhitzt. Aufarbeitung wie in Beispiel 24 und Umkristallisation aus Aether-Hexan lieferte 0,25 g 2-Hydroxy-5-[6 -[2,3-bis(phenylmethoxy)phenyl]hexyloxy]benzoesäure, Smp. 97-100°.

Beispiel 64

Ein Gemisch von 0,22 g 2-Hydroxy-5-[6 -[2,3-bis(phenylmethoxy)phenyl]hexyloxy]benzoesäure und 30 mg 10% Palladium/Kohle in 10 ml Methanol wurde 5 Stunden unter Wasserstoffatmosphäre gerührt. Aufarbeitung wie in Beispiel 36 und Umkristallisation aus Aceton-Hexan lieferte 0,1 g 2-Hydroxy-5-[6-(2,3-dihydroxyphenyl)hexyloxy]benzoesäure, Smp. 159-161°.

Beispiel 65

Ein Gemisch von 1 g 1-(6-Bromhexyl)-2,3-dimethoxybenzol, 0,45 g 4-Hydroxybenzoesäure-methylester, 0,62 g Kaliumcarbonat und 0,75 g Kaliumjodid in 25 ml Aceton wurde 23 Stunden unter Rühren zum Rückfluss erhitzt. Aufarbeitung wie in Beispiel 16 und Reinigung durch HPLC mit 15% Aethylacetat-Hexan lieferte 1,1 g 4-[6-(2,3-Dimethoxyphenyl)hexyloxy]benzoesäure-methylester als Oel.

Beispiel 66

Zu 0,8 g 4-[6-(2,3-Dimethoxyphenyl)hexyloxy]benzoesäure-methylester in 25 ml Methylenchlorid wurden bei -70° unter Rühren 8 ml 1M Bortribromid in Methylenchlorid gegeben. Das Gemisch wurde 30 Minuten bei -70° gerührt und dann 7 Stunden bei -20°. Aufarbeitung wie in Beispiel 32 und Umkristallisation aus Aethylacetat-Hexan lieferte 0,3 g 4-[6-(2,3-Dihydroxyphenyl)hexyloxy]benzoesäure vom Smp. 170-172°.

Beispiel 67

Ein Gemisch von 0,58 g 1-(6-Jodhexyl)-2,3-bis(phenylmethoxy)benzol, 0,18 g 3-Hydroxybenzoesäure-methylester und 0,25 g Kaliumcarbonat in 15 ml Aceton wurde 18 Stunden unter Rühren zum Rückfluss erhitzt. Aufarbeitung wie in Beispiel 16 und Reinigung durch HPLC mit Toluol lieferte 0,4 g 3-[6-[2,3-Bis(phenylmethoxy)phenyl]hexyloxy]benzoesäure-methylester als Oel.

Beispiel 68

Eine Lösung von 0,5 g 3-[6-[2,3 -Bis(phenylmethoxy)phenyl]hexyloxy]benzoesäure-methylester in 15 ml Methanol und 5 ml 1N Natronlauge wurde 2 Stunden unter Rühren zum Rückfluss erhitzt. Aufarbeitung wie in Beispiel 24 und Kristallisation aus Methanol lieferte 0,34 g 3-[6-[2,3-Bis(phenylmethoxy)phenyl]hexyloxy]-benzoesäure, Smp. 72-74°.

Beispiel 69

Ein Gemisch von 0,33 g 3-[6-[2,3-Bis(phenylmethoxy)phenyl]hexyloxy]benzoesäure und 0,95 mg 10% Palladium/Kohle in 20 ml Aethylacetat und 5 ml Aethanol wurde 11 Stunden unter einer Wasserstoffatmosphäre gerührt. Das Reaktionsgemisch wurde über Celit filtriert und das Filtrat unter vermindertem Druck zu einem Oel eingeengt. Chromatographie an 10 g Silicagel und Elution mit 10% Methanol-Chloroform lieferte einen Feststoff, der nach Umkistallisation aus Aether-Hexan 0,14 g 3-[6-(2,3-Dihydroxyphenyl)hexyloxy]-benzoesäure, Smp. 123-125° lieferte.

Beispiel 70

Ein Gemisch von 5,6 g 1-(6-Jodhexyl)-2,3-bis(phenylmethoxy)benzol, 2,1 g 3-Chlor-4-hydroxybenzoesäure-methylester und 5 g Kaliumcarbonat in 50 ml Aceton wurde 20 Stunden unter Rühren zum Rückfluss erhitzt. Aufarbeitung wie in Beispiel 16, Chromatographie an 100 g Silicagel mit 15% Aethylacetat-Hexan und Kristallisation aus Aethylacetat-Hexan lieferte 3,7 g 3-Chlor-4-[6-[2,3 -bis-(phenylmethoxy)phenyl]hexyloxy]benzoesäure-methylester, Smp. 68-69°.

Beispiel 71

Eine Lösung von 3,6 g 3-Chlor-4-[6-[2,3 -bis(phenylmethoxy)phenyl]hexyloxy]benzoesäure-methylester in 90 ml Methanol und 30 ml 1N Natronlauge wurde 2 Stunden zum Rückfluss erhitzt. Aufarbeitung wie in Beispiel 24, Chromatographie an 70 g Silicagel und 50% Aethylacetat-Hexan und Umkristallisation aus

Aether-Hexan lieferte 1,3 g 3-Chlor-4-[6-[2,3 -bis(phenylmethoxy)phenyl]hexyloxy]benzoesäure, Smp. 87-89°.

Beispiel 72

Ein Gemisch von 0,6 g 3-Chlor-4-[6-[2,3 -bis(phenylmethoxy)phenyl]hexyloxy]benzoesäure und 60 mg 10% Palladium/Kohle in 20 ml Tetrahydrofuran wurde unter Wasserstoff 6 Stunden gerührt. Aufarbeitung wie in Beispiel 36 und Umkristallisation aus Aethylacetat-Hexan lieferte 0,24 g 3-Chlor-4-[6-[2,3-bis-(phenylmethoxy)hexyloxy]benzoesäure, Smp. 157-160°.

Beispiel 73

Ein Gemisch von 3,4 g 2,4-Dihydroxy-3-propylbenzoesäure-methylester, 4,7 g 1-[(2-Methansulfonyloxy)-äthyl]-2,3-dimethoxybenzol, 4,4 g Kaliumcarbonat und 2,7 g Natriumjodid in 90 ml Aceton wurde 18 Stunden unter Rühren zum Rückfluss erhitzt. Danach wurden 4,7 g 1-[(2-Methansulfonyloxy)äthyl]-2,3-dimethoxybenzol und 4,4 g Kaliumcarbonat zugesetzt und das Erhitzen wurde 41 Stunden fortgesetzt. Das Reaktionsgemisch wurde filtriert, das Filtrat konzentriert und das zurückbleibende Oel durch HPLC mit 10% Aethylacetat-Hexan und dann Aethylacetat gereinigt. Man erhielt 4,5 g Oel, das nach Rühren mit Hexan und Filtrieren 1,65 g 4-[2-(3,4-Dimethoxyphenyl)äthoxy]-2-hydroxy -3-propylbenzoesäure-methylester, Smp. 56-58° lieferte.

Beispiel 74

Ein Gemisch von 9,5 g 1-(3-Brompropyl)-3,4-dimethoxybenzol, 7 g 2,4-Dihydroxy-3-propylbenzoesäure-methylester, 6,9 g Kaliumcarbonat und 8,3 g Kaliumjodid in 250 ml Aceton wurde 24 Stunden zum Rückfluss erhitzt. Das Reaktionsgemisch wurde filtriert und das Filtrat unter vermindertem Druck zu einem Oel eingeengt, das durch HPLC mit 15% Aethylacetat-Hexan gereinigt wurde und 7,2 g 4-[3-(3,4-Dimethoxyphenyl)propoxy]-2-hydroxy -3-propylbenzoesäure-methylester als Oel lieferte.

In Analogie zu dem Verfahren von Beispiel 74 wurden die Verbindungen von Beispiel 75-77 hergestellt:

Beispiel 75

4-[3-(3,4-Dimethoxyphenyl)butoxy]-2-hydroxy-3-propylbenzoesäuremethylester, Ausbeute 49%.

Beispiel 76

4-[3-(3,4-Dimethoxyphenyl)pentyloxy]-2-hydroxy-3-propylbenzoesäurebenzylester, Ausbeute 80%.

Beispiel 77

4-[3-(3,4-Dimethoxyphenyl)hexyloxy]-2-hydroxy-3-propylbenzoesäurebenzylester, Ausbeute 77%.

Beispiel 78

Eine Lösung von 7,1 g 4-[3-(3,4-Dimethoxyphenyl)propoxy]-2-hydroxy-3-propylbenzoesäuremethylester in 180 ml Methanol und 90 ml 1N Natronlauge wurde 1 Stunde unter Rühren zum Rückfluss erhitzt. Das Lösungsmittel wurde unter vermindertem Druck entfernt, der Rückstand angesäuert und das Produkt mit Methylenchlorid extrahiert. Der mit Magnesiumsulfat getrocknete Extrakt wurde konzentriert und lieferte 6,5 g 4-[3-(3,4-Dimethoxyphenyl)propoxy]-2-hydroxy-3-propylbenzoesäure, Smp. 104-110°.

In Analogie zu dem Verfahren von Beispiel 78 wurden die Verbindungen von Beispiel 79-83 hergestellt:

Beispiel 79

4-[3-(3,4-Dimethoxyphenyl)äthoxy]-2-hydroxy-3-propylbenzoesäure, Smp. 156-157°.

Beispiel 80

4-[3-(3,4-Dimethoxyphenyl)butoxyl]-2-hydroxy-3-propylbenzoesäure, Smp. 125-127°.

Beispiel 81

4-[3-(3,4-Dimethoxyphenyl)pentyloxy)-2-hydroxy-3-propylbenzoesäure, Smp. 133-136°.

Beispiel 82

4-[3-(3,4-Dimethoxyphenyl)hexyloxy]-2-hydroxy-3-propylbenzoesäure, Smp. 99-101°.

Beispiel 83

Zu 3 g 4-[3-(3,4-Dimethoxyphenyl)propoxy]-2-hydroxy-3-propylbenzoesäure, in 250 ml Methylenchlorid suspendiert, wurden bei -70° 24 ml 1M Bortribromid in Methylenchlorid tropfenweise im Verlauf von 30 Minuten gegeben. Das Reaktionsgemisch wurde 1 Stunde bei -70° und 1,5 Stunden in einem Eisbad gerührt. Danach wurden 100 ml Wasser tropfenweise unter Rühren zugesetzt und das Produkt wurde mit Äther extrahiert. Der Extrakt wurde unter vermindertem Druck konzentriert und der Rückstand in 500 ml Aether aufgenommen und kräftig mit 100 ml 1N Salzsäure geschüttelt. Der getrocknete Extrakt wurde eingeengt und der Rückstand aus Aether-Hexan umkristallisiert. Man erhielt 2,2 g 4-[3-(3,4-Dihydroxyphenyl)propoxy]-2-hydroxy-3-propylbenzoesäure, Smp. 194-195°.

In Analogie zu dem Verfahren von Beispiel 83 wurden die Verbindungen von Beispiel 84-87 hergestellt:

Beispiel 84

4-[3-(3,4-Dihydroxyphenyl)äthoxy]-2-hydroxy-3-propylbenzoesäure, Smp. 164-165°.

Beispiel 85

4-[3-(3,4-Dihydroxyphenyl)butoxy]-2-hydroxy-3-propylbenzoesäure, Smp. 190-193°.

Beispiel 86

4-[3-(3,4-Dihydroxyphenyl)pentyloxy]-2-hydroxy-3-propylbenzoesäure, Smp. 159-162°.

Beispiel 87

4-[3-(3,4-Dihydroxyphenyl)hexyloxy]-2-hydroxy-3-propylbenzoesäure, Smp. 113-114°.

Beispiel 88

Ein Gemisch von 2,56 g 1-(3-Brompropyl)-3,4-dimethoxybenzol, 1,5 g 4-Hydroxybenzoesäureäthylester, 1,38 g Kaliumcarbonat und 1,66 g Kaliumjodid in 50 ml Aceton wurde 22 Stunden unter Rühren zum Rückfluss erhitzt. Aufarbeitung wie in Beispiel 16, Reinigung durch HPLC mit Methylenchlorid und Umkristallisation aus 2-Propanol lieferte 1,23 g 4-[3-(3,4-Dimethoxyphenyl)propoxy]benzoesäureäthylester, Smp. 70-71°.

Beispiel 89

Eine Lösung von 1,2 g 4-[3-(3,4-Dimethoxyphenyl)propoxy] benzoesäureäthylester in 40 ml Methanol und 18 ml 1N Natronlauge wurde 1 Stunde unter Rühren zum Rückfluss erhitzt. Aufarbeitung wie in Beispiel 24 lieferte 1,1 g 4-[3-(3,4-Dimethoxyphenyl)propoxy]benzoesäure, Smp. 150-154°.

Beispiel 90

Zu 1,08 g 4-[3-(3,4-Dimethoxyphenyl)propoxy]benzoesäure, in 60 ml Methylenchlorid suspendiert, wurden bei -70° 10 ml 1M Bortribromid in Methylenchlorid gegeben. Das Reaktionsgemisch wurde 2,5 Stunden bei -70° gerührt und dann wie in Beispiel 32 aufgearbeitet. Umkristallisation aus Aether-Hexan lieferte 0,4 g 4-[3-(3,4-Dihydroxyphenyl)propoxy]benzoesäure, Smp. 180-185°.

Beispiel 91

Ein Gemisch von 1,6 g 6-Brom-1-(3,4-dimethoxyphenyl)-1-hexanon, 0,95 g 2,4-Dihydroxy-3-propylbenzoesäuremethylester, 1,38 g Kaliumcarbonat und 0,75 g Natriumjodid in 40 ml Aceton wurde 45 Stunden unter Rühren zum Rückfluss erhitzt. Das Reaktionsgemisch wurde filtriert und das Filtrat konzentriert und mit Wasser versetzt. Das Produkt wurde abfiltriert und aus Methylenchlorid-Methanol umkristallisiert. Man erhielt 1,4 g 4-[[6-(3,4-Dimethoxyphenyl)-6-oxohexyl]oxy]-2-hydroxy -3-propylbenzoesäuremethylester, Smp. 117-119°.

Beispiel 92

Eine Lösung von 1,36 g 4-[[6-(3,4-Dimethoxyphenyl)-6-oxo-hexyl]oxy]-2-hydroxy-3-propylbenzoesäuremethylester in 35 ml Methanol und 13 ml 1N Natronlauge wurde 8,5 Stunden unter Rühren zum Rückfluss erhitzt. Das Lösungsmittel wurde unter vermindertem Druck entfernt, der Rückstand angesäuert und das Produkt abfiltriert. Umkristallisation aus Aethylacetat-Hexan lieferte 0,94 g 4-[[6-(3,4-Dimethoxyphenyl)-6-oxohexyl]oxy]-2-hydroxy-3-propyl-benzoesäure, Smp. 114-117°.

Beispiel 93

Zu 0,93 g 4-[[6-(3,4-Dimethoxyphenyl)-6-oxohexyl]oxy]-2-hydroxy-3-propylbenzoesäure in 60 ml Methylenchlorid suspendiert, wurden bei -70° 7 ml 1N Bortribromid in Methylenchlorid gegeben. Das Reaktionsgemisch wurde 1 Stunde bei -70° und 20 Stunden bei -80° gerührt. Aufarbeitung wie in Beispiel 32 und Chromatographie des Rohprodukts an 100 g Silicagel mit Essigsäure:Aethylacetat:Toluol (5:25:70) und anschliessende Umkristallisation aus Aethylacetat-Hexan lieferte 0,42 g 4-[[6-(3,4-Dihydroxyphenyl)-6-oxohexyl]oxy]-2-hydroxy-3-propylbenzoesäure, Smp. 188-191°.

Beispiel 94

Ein Gemisch von 4,36 g 1-(4-Brombutyl)-2,3-dimethoxybenzol, 3,1 g 1-(2,4-Dihydroxy-3-propylphenyl)-äthanon, 4,4 g Kaliumcarbonat und 2,4 g Natriumjodid in 100 ml Aceton wurde 30 Stunden unter Rühren zum Rückfluss erhitzt. Das Reaktionsgemisch wurde filtriert und das Filtrat unter vermindertem Druck konzentriert. Reinigung durch HPLC mit 20% Aethylacetat-Hexan lieferte 5,07 g 1-[2-Hydroxy-4-[4-(2,3-dimethoxyphenyl)butoxy] -3-propylphenyl]äthanon als Oel.

In Analogie zu Beispiel 94 wurden die Verbindungen von Beispiel 95-97 hergestellt:

Beispiel 95

1-[2-Hydroxy-4-[4-(2,3-dimethoxyphenyl)hexyloxy]-3-propylphenyl]äthanon, Oel.

Beispiel 96

1-[2-Hydroxy-4-[4-(2,3-dimethoxyphenyl)octyloxy]-3-propylphenyl]äthanon, Oel.

Beispiel 97

1-[2-Hydroxy-4-[4-(2,3-dimethoxy-4-isopropylphenyl)hexyloxy]-3-propylphenyl]äthanon, Oel.

Beispiel 98

Zu 5,02 g 1-[2-Hydroxy-4-[4-(2,3-dimethoxyphenyl)butoxy]-3-propylphenyl]äthanon in 300 ml Methylenchlorid wurden bei -70° unter Rühren neue 30 ml 1M Bortribromid in Methylenchlorid gegeben. Das Reaktionsgemisch wurde 30 Minuten bei -70° gerührt und 5,5 Stunden bei -20° gehalten. Aufarbeitung wie in Beispiel 32 und Umkristallisation aus Aceton-Hexan lieferten 3,81 g 1-[2-Hydroxy -4-[4-(2,3-dihydroxyphenyl)butoxy]-3-propylphenyl]-äthanon, Smp. 103-105°.

In Analogie zu Beispiel 98 wurden die Verbindungen von Beispiel 99-101 hergestellt:

Beispiel 99

1-[2-Hydroxy-4-[4-(2,3-dihydroxyphenyl)hexyloxy]-3-propylphenyl]äthanon, Smp. 106-108°.

Beispiel 100

1-[2-Hydroxy-4-[4-(2,3-dihydroxyphenyl)octyloxy]-3-propylphenyl]äthanon, Smp. 116-118°.

Beispiel 101

1-[2-Hydroxy-4-[4-(2,3-dihydroxy-4-isopropylphenyl)hexyloxy]-3-propylphenyl]äthanon, als Oel.

Beispiel 102

3 g 5-(3,4-Dimethoxyphenyl)pentan-1-ol wurde wie in Beispiel 6 beschrieben in das Mesylat übergeführt. Ein Gemisch dieses Mesylats, 2,6 g 1-(2,4-Dihydroxy-3-propylphenyl)äthanon, 2,8 g Kaliumcarbonat und 0,28 ml Tris [2-(2-Methoxyäthoxy)äthyl]amin (TDA-1) in 75 ml Toluol wurde 6,5 Stunden unter Rühren zum Rückfluss erhitzt. Das Reaktionsgemisch wurde mit halbgesättigter Kochsalzlösung und dann mit 1N Natronlauge gewaschen, getrocknet und unter vermindertem Druck zu einem Oel eingeengt. Reinigung durch HPLC mit 25% Aethylacetat-Hexan lieferte 4,05 g 1-[2-Hydroxy-4-[5-(3,4-dimethoxyphenyl)pentyloxy]-3-propylphenyl]-1-äthanon, Smp. 72-75°.

Beispiel 103

Zu 3,95 g 1-[2-Hydroxy-4-[5-(3,4-dimethoxyphenyl)pentyloxy)-3-propylphenyl]-1-äthanon in 80 ml Methylenchlorid wurden bei -70° 30 ml 1M Bortribromid in Methylenchorid gegeben. Nach 30 Minuten bei -70° und 6 Stunden bei -20° wurde das Reaktionsgemisch wie in Beispiel 32 aufgearbeitet und das Produkt aus Aether-Hexan umkristallisiert. Man erhielt 3,24 g 1-[4-[5-(3,4-Dihydroxyphenyl)pentyloxy]-2-hydroxy-3-propylphenyl]-1-äthanon, Smp. 126-127°.

Beispiel 104

Eine Lösung von 16 ml 2,5M Butyllithium in Hexan wurde im Verlauf von 15 Minuten tropfenweise zu einer gerührten Lösung von 8,3 g 2,3-Dimethoxybiphenyl in 160 ml wasserfreiem Tetrahydrofuran bei 0° und unter Argon gegeben. Das Reaktionsgemisch wurde 2,5 Stunden bei 0° gerührt, dann 30 Minuten zum Rückfluss erhitzt. Nach Kühlen auf 5° wurden 6,3 ml 1,6-Dibromhexan zugesetzt. Danach wurde das Reaktionsgemisch 30 Minuten bei 5° und 30 Minuten bei 25° und 20 Stunden unter Erhitzen zum Rückfluss gerührt. Aufarbeitung wie in Beispiel 1 lieferte ein Oel. Reinigung durch HPLC mit 3% Aethylacetat-Hexan lieferte 5,7 g nicht umgesetztes 2,3-Dimethoxybiphenyl und 3,3 g 4-(6-Bromhexyl)-2,3-dimethoxy-1,1′-biphenyl als Oel.

Beispiel 105

Ein Gemisch von 3,3 g 4-(6-Bromhexyl)-2,3-dimethoxy-1,1′-biphenyl, 2,5 g 2,4-Dihydroxy-3-propylbenzoesäurephenylmethylester, 1,8 g Kaliumcarbonat und 0,2 ml Tris[2-(2-Methoxyäthoxy)äthyl]amin in 65 ml wasserfreiem Toluol wurde 30 Stunden unter Rühren zum Rückfluss erhitzt. Das Reaktionsgemisch wurde mit halbgesättigter Kochsalzlösung und dann mit 1N Natronlauge gewaschen. Nach Trocknen der organischen Schicht wurde das Lösungsmittel unter vermindertem Druck entfernt und das erhaltene Oel durch Chromatographie an 100 g Silicagel gereinigt. Elution mit 10% Aethylacetat-Hexan lieferte 3,7 g 2-Hydroxy-4-[[6-(2,3-dimethoxy-1,1′-biphenyl)-4-yl]hexyl]-oxy]-3-propylbenzoesäurephenylmethylester.

Beispiel 106

Ein Gemisch von 3,76 g 2-Hydroxy-4-[[6-(2,3-dimethoxy-1,1′-biphenyl)-4-yl]hexyl]oxy]-3-propylbenzoesäurephenylmethylester und 0,3 g 10% Palladium/Kohle in 80 ml Tetrahydrofuran wurde unter Wasserstoffatmosphäre 17 Stunden gerührt. Aufarbeitung wie in Beispiel 36 und Umkristalliation aus Hexan lieferte 2,4 g 2-Hydroxy-4-[[6-(2,3-dimethoxy-1,1′-biphenyl)-4-yl]hexyl]oxy]-3-propylbenzoesäure, Smp. 78-80°.

Beispiel 107

Zu 1,72 g 2-Hydroxy-4-[[6-(2,3-dimethoxy-1,1'-biphenyl)-4-hexyl]oxy]-3-propylbenzoesäure, in 150 ml Methylenchlorid suspendiert, wurden bei -70° 10,5 ml 1M Bortribromid in Methylenchlorid gegeben. Das Reaktionsgemisch wurde 20 Minuten bei -70° gerührt und dann 17 Stunden bei -18° gehalten. Aufarbeitung wie in Beispiel 32 und Umkristallisation aus Methylenchlorid lieferte 1,03 g 2-Hydroxy-4-[[6-(2,3-dihydroxy-1,1'-biphenyl)-4-yl]hexyl]oxy]-3-propylbenzoesäure, Smp. 151-155°.

Beispiel 108

Zu 0,6 g in kleine Stücke geschnittenem Lithiumband in 40 ml wasserfreiem Aether wurde unter Rühren und Argonatmosphäre bei Raumtemperatur 0,5 g 5-Brompentanol-2-äthoxyäthyläther gegeben. Nachdem etwa 1 ml zugesetzt war wurde das Reaktionsgemisch auf -5° gekühlt und die restliche Bromverbindung tropfenweise zugesetzt. Danach wurde noch 1 Stunde bei -5° gerührt und dann wurden 6,0 g 2-Chlor-3,4-dimethoxybenzaldehyd in 50 ml Aether und 20 ml Tetrahydrofuran tropfenweise im Verlauf einer Stunde zugesetzt. Das Kühlbad wurde entfernt und es wurde noch 1 Stunde gerührt. Das Reaktionsgemisch wurde wie in Beispiel 10 aufgearbeitet und das erhaltene Oel in 25 ml Aethanol gelöst. Danach wurden 20 ml Wasser und 2 ml konzentrierte Salzsäure zugesetzt. Die Lösung wurde 45 Minuten bei 25° stehen gelassen. Danach wurde Kaliumcarbonat unter Rühren zugesetzt bis das Gemisch basisch war. Das Aethanol wurde unter vermindertem Druck entfernt und das Produkt mit Aethylacetat extrahiert. Der getrocknete Extrakt wurde zu einem Oel eingeengt. Das Oel wurde durch HPLC mit 60% Aethylacetat-Hexan gereinigt und lieferte 2,9 g 6-(2-Chlor-3,4-dimethoxybenzol)-6-hydroxy-hexanol, Smp. 65-70°. Dieses Produkt wurde in 50 ml Aethanol gelöst und nach Zusatz von 0,3 g 10% Palladium/Kohle 21 Stunden unter einem anfänglichen Wasserstoffdruck von 54 psi geschüttelt. Das Reaktionsgemisch wurde über Celit abfiltriert und das Filtrat unter vermindertem Druck eingeengt. Reinigung durch HPLC mit 15% Aethylacetat-Toluol lieferte 1,74 g 2-Chlor-3,4-dimethoxybenzolhexanol als Oel.

Beispiel 109

Zu 1,74 g 2-Chlor-3,4-dimethoxybenzolhexanol in 25 ml Methylenchlorid wurde unter Kühlung durch ein Eisbad 1,8 ml Triäthylamin gefolgt von 0,65 ml Methansulfonylchlorid gegeben. Das Reaktionsgemisch wurde 80 Minuten unter Kühlung im Eisbad gerührt und dann wie in Beispiel 3 aufgearbeitet. Das erhaltene Mesylat, 1,24 g 2,4-Dihydroxy-3-propylbenzoesäuremethylester, 1,8 g Kaliumcarbonat und 0,2 ml tris[2-(2-Methoxyäthoxy)äthyl]amin wurden in 40 ml Toluol 19 Stunden unter Rühren zum Rückfluss erhitzt. Das Reaktionsgemisch wurde dann filtriert und das Filtrat eingeengt. Das Rohprodukt wurde durch HPLC mit 7% Aethylacetat-Hexan gereinigt und lieferte 2,11 g 4-[6-(2-Chlor-3,4-dimethoxyphenyl)hexyloxy]-2-hydroxy-3-propylbenzoesäuremethylester als Oel.

Beispiel 110

Eine Lösung von 2,1 g 4-[6-(2-Chloro-3,4-dimethoxyphenyl)hexyloxy]-2-hydroxy-3-propylbenzoesäuremethylester in 50 ml Methanol und 15 ml Dioxan und 15 ml 1N Natronlauge wurde 8 Stunden zum Rückfluss erhitzt. Die Lösungsmittel wurden unter vermindertem Druck abgedampft, der Rückstand angesäuert und das Produkt mit Aethylacetat extrahiert. Der getrocknete Extrakt wurde eingeengt und der Rückstand aus Aethylacetat-Hexan umkristallisiert. Man erhielt 1,86 g 4-[6-(2-Chlor-3,4-dimethoxyphenyl)-hexyloxy]-2-hydroxy-3-propylbenzoesäure, Smp. 107-108°.

Beispiel 111

Zu 1,8 g 4-[6-2-Chlor-3,4-dimethoxyphenyl)hexyloxy]-2-hydroxy-3-propylbenzoesäure, in 120 ml Methylenchlorid suspendiert, wurden bei -70° 14 ml 1M Bortribromid in Methylenchlorid gegeben. Das Reaktionsgemisch wurde 15 Minuten bei -70° gerührt und 6 Stunden bei -18° gehalten. Aufarbeitung wie in Beispiel 32 und Umkristallisation aus Aethylacetat-Hexan lieferte 1,03 g 4-[6-(2-Chlor-3,4-dihydroxyphenyl)-hexyloxy]-2-hydroxy -3-propylbenzoesäure, Smp. 145-146°.

Beispiel 112

Zu 4 g 1-(5-Brompentyl)-2,3-dimethoxybenzol in 50 ml Methylenchlorid wurde unter Eisbadkühlung 18 ml 0,8M Chlor in Methylenchlorid gegeben. Das Reaktionsgemisch wurde 3 Stunden bei 0° gehalten und dann unter vermindertem Druck zu einem Oel eingeengt. Reinigung durch HPLC mit 30% Toluol-Hexan lieferte 2,6 g 1-(5-Brompentyl)-6-chlor-2,3-dimethoxybenzol.

Beispiel 113

Zu 4,0 g 1-(5-Brompentyl)-2,3-dimethoxybenzol in 50 ml Methylenchlorid wurde unter Eisbadkühlung 18 ml 0,8M Chlor in Methylenchlorid gegeben. Nach 15 Minuten wurden 17 ml 0,88M Chlor in Methylenchlorid zugesetzt. Das Reaktionsgemisch wurde 3 Stunden bei 0° gehalten und dann unter vermindertem Druck zu einem Oel eingeengt. Reinigung durch HPLC mit 30% Toluol-Hexan lieferte 2,03 g 1-(5-Brompentyl)-5,6-dichlor-2,3-dimethoxybenzol.

Beispiel 114

Zu 1,9 g 1-(5-Brompentyl)-2,3-dimethoxybenzol in 25 ml Methylenchlorid wurde unter Eisbadkühlung 23 ml 0,88M Chlor in Methylenchlorid gegeben. Nach 1,5 Stunden bei 0° wurden 5 ml 1,35M Chlor in Methylenchlorid zugesetzt. Das Reaktionsgemisch wurde 17 Stunden bei 0° gehalten und dann unter vermindertem Druck zu einem Oel eingeengt. Reinigung durch HPLC mit 25% Toluol-Hexan lieferte 1,48 g 1-(5-Brompentyl)-2,3-dimethoxy-4,5,6-trichlorbenzol.

Beispiel 115

Zu 1,4 g 6-(3,4-Dimethoxyphenyl)hexan-1-ol in 25 ml Methylenchlorid wurde unter Aethanol Trockeneiskühlung 4,6 ml 1,35M Chlor in Methylenchlorid gegeben. Das Reaktionsgemisch wurde 1,5 Stunden bei -75°, 16 Stunden bei -18° und 24 Stunden bei 0° gehalten. Nach Einengen unter vermindertem Druck wurde das Rohprodukt durch HPLC gereinigt, wobei 6-(6-Chlor-3,4-dimethoxyphenyl)hexan-1-ol als Oel erhalten wurde.

Beispiel 116

Eine Lösung von 5 g 3-(1-Methyläthyl)-1,2-dimethoxybenzol in 5 ml Methylenchlorid wurde zu einer eisgekühlten Mischung von 4,4 g Aluminiumchlorid und 7 g 6-Bromhexanoylchlorid in 50 ml Methylenchlorid gegeben. Das Gemisch wurde 18 Stunden bei 0° gehalten. Danach wurde Wasser zugesetzt, die organische Phase abgetrennt und mit Natriumbicarbonatlösung gewaschen. Der getrocknete Extrakt wurde under vermindertem Druck zu einem Oel eingeengt das durch HPLC mit 5% Aethylacetat-Hexan gereinigt wurde. Man erhielt 8,1 g 6-Brom-1-[3,4-dimethoxy-5-(1-methyläthyl)phenyl]-1-hexanon als Oel.

Beispiel 117

Eine Lösung von 0,227 g 3,6-Dimethylveratrol in 1 ml Methylenchlorid wurde zu einer eisgekühlten Mischung von 0,245 g Aluminiumchlorid und 0,416 g 6-Bromhexanoylchlorid in 3 ml Methylenchlorid gegeben. Das Reaktionsgemisch wurde 19 Stunden bei 0° gehalten. Danach wurde Wasser zugesetzt und die organische Phase abgetrennt und mit Natriumbicarbonatlösung gewaschen. Der trockene Extrakt wurde eingeengt und das erhaltene Oel an 60 g Silicagel mit 10% Aethylacetat-Hexan chromatographiert. Man erhiert 0,235 g 6-Brom-1-(3,4-dimethoxy-2,5-dimethylphenyl)-1-hexanon als Oel.

Beispiel 118

Eine Lösung von 0,1056 g 4-Fluorveratrol in 1 ml Methylenchlorid wurde zu einem Gemisch von 0,2289 g Aluminiumchlorid und 0,09 ml 4-Chlorbutyrylchlorid in 2 ml Methylenchlorid bei 25° gegeben. Das Reaktionsgemisch wurde 22 Stunde bei 25° gerührt. Danach wurde Wasser zugesetzt und die organische Phase abgetrennt und mit Natriumbicarbonatlösung gewaschen. Der getrocknete Extrakt wurde unter vermindertem Druck zu einem Oel eingeengt, das an 10 g Silicagel mit 25% Aethylacetat-Hexan chromatographiert wurde und 0,041 g 4-Chlor-[3,4-dimethoxy-6-fluorphenyl]-1-hexanon, Smp. 81-82° lieferte.

Beispiel 119

Ein Gemisch von 2,6 g 1-(5-Brompentyl)-6-chlor-2,3-dimethoxybenzol, 1,65 g 2,4-Dihydroxy-3-propylbenzoesäuremethylester und 5,0 g wasserfreies Kaliumcarbonat in 60 ml Aceton und 6 ml DMF wurde 24 Stunden unter Rühren zum Rückfluss erhitzt. Das Reaktionsgemisch wurde filtriert und das Filtrat unter vermindertem Druck eingeengt. Kristallisation aus Hexan lieferte 2,95 g 4-[5-(2-Chlor-5,6-dimethoxyphenyl)-pentyloxy]-2-hydroxy-3-propylbenzoesäure-methylester, Smp. 53-55°.

Beispiel 120

Eine Lösung von 2,95 g 4-[5-(2-Chlor-5,6-dimethoxyphenyl)pentyloxy]-2-hydroxy-3-propylbenzoesäure-methylester in 80 ml und 20 ml 1N Natronlauge wurde 4 Stunden unter Rühren zum Rückfluss erhitzt. Aufarbeitung wie in Beispiel 24 und Umkristallisation aus Aether-Hexan lieferte 2,7 g 4-[5-(2-Chlor-5,6-dimethoxyphenyl)pentyloxy]-2-hydroxy-3-propylbenzoesäure, Smp. 140-142°.

Beispiel 121

Zu 2,7 g 4-[5-(2-Chlor-5,6-dimethoxyphenyl)pentyloxy]-2-hydroxy-3-propylbenzoesäure, in 250 ml Methylenchlorid suspendiert, wurde unter Rühren bei -60° 18,6 ml 1M Bortribromid in Methylenchlorid gegeben. Das Reaktionsgemisch wurde 20 Minuten bei -60° gerührt und 19 Stunden bei -20° gehalten. Nach Zusatz von Wasser wurde das Produkt mit Aether extrahiert. Der Extrakt wurde under vermindertem Druck eingeengt und der Rückstand in 50 ml Aether gelöst und mit 50 ml 1N Salzsäure 20 Minuten kräftig geschüttelt. Der getrocknete Extrakt wurde unter vermindertem Druck eingedampft und der Rückstand aus Aether-Chloroform kristallisiert. Man erhielt 1,1 g 4-[5-(2-Chlor-5,6-dihydroxy-phenyl)pentyloxy]-2-hydroxy-3-propylbenzoesäure, Smp. 178-181°.

Beispiel 122

Ein Gemisch von 2,03 g 1-(5-Brompentyl)-2,3-dichlor-5,6-dimethoxybenzol, 1,15 g 2,4-Dihydroxy-3-propylbenzoesäuremethylester und 3,5 g Kaliumcarbonat in 50 ml Aceton und 5 ml DMF wurde 24 Stunden unter Rühren zum Rückfluss erhitzt. Das Reaktionsgemisch wurde filtriert und das Filtrat unter vermindertem Druck eingeengt. Kristallisation aus Aether-Hexan lieferte 1,5 g 4-[5-(2,3-Dichlor-5,6-dimethoxyphenyl)-pentyloxy]-2-hydroxy-3-propylbenzoesäuremethylester, Smp. 113-115°.

Beispiel 123

Eine Lösung von 1,5 g 4-[5-(2,3-Dichlor-5,6-dimethoxyphenyl)pentyloxy]-2-hydroxy-3-propylbenzoesäuremethylester in 40 ml Methanol und 10 ml 1N Natronlauge wurde 5 Stunden unter Rühren zum Rückfluss erhitzt. Aufarbeitung wie in Beispiel 24 und Umkristallisation aus Aether-Hexan lieferte 1,2 g 4-[5-(2,3-Dichlor-5,6-dimethoxyphenyl)pentyloxy]-2-hydroxy-3-propylbenzoesäure, Smp. 152-154°.

Beispiel 124

Zu 1,2 g 4-[5-(2,3-Dichlor-5,6-dimethoxyphenyl)pentyloxy]-2-hydroxy-3-propylbenzoesäure, in 100 ml Methylenchlorid suspendiert, wurde unter Rühren bei -60° 7,5 ml 1M Bortribromid in Methylenchlorid gegeben. Nach 30 Minuten Rühren bei -60° wurde das Reaktionsgemisch 20 Stunden bei -20° gehalten. Aufarbeitung wie in Beispiel 24 lieferte einen festen Rückstand der aus Aether/Chloroform umkristallisiert, 0,42 g 4-[5-(2,3-Dichlor-5,6-dihydroxyphenyl)pentyloxy]-2-hydroxy-3-propylbenzoesäure, Smp. 159-164° lieferte.

Beispiel 125

Ein Gemisch von 1,48 g 1-(5-Brompentyl)-5,6-dimethoxy-2,3,4-trichlorbenzol, 0,76 g 2,4-Dihydroxy-3-propylbenzoesäuremethylester und 4,6 g Kaliumcarbonat in 30 ml Aceton und 3 ml DMF wurde 23 Stunden unter Rühren zum Rückfluss erhitzt. Das Reaktionsgemisch wurde filtriert und das Filtrat unter vermindertem Druck eingeengt. Kristallisation aus Aether-Hexan lieferte 1,5 g 4-[5-(2,3,4-Trichlor-5,6-dimethoxyphenyl)pentyloxy]-2-hydroxy-3-propylbenzoesäuremethylester, Smp. 84-87°.

Beispiel 126

Eine Lösung von 1,5 g 4-[5-(2,3,4-Trichlor-5,6-dimethoxyphenyl)pentyloxy]-2-hydroxy-3-propylbenzoesäuremethylester in 40 ml Methanol und 8,7 ml 1N Natronlauge wurde 5 Stunden unter Rühren zum Rückfluss erhitzt. Aufarbeitung wie in Beispiel 24 und Umkristallisation aus Aethylacetat-Hexan lieferte 1,3 g 4-[5-(2,3,4-Trichlor-5,6-dimethoxyphenyl)pentyloxy]-2-hydroxy-3-propylbenzoesäure Smp. 148-150°

Beispiel 127

Zu 1,3 g 4-[5-(2,3,4-Trichlor-5,6-dimethoxyphenyl)pentyloxy-2-hydroxy-3-propylbenzoesäure, suspendiert in 110 ml Methylenchlorid wurde, unter Rühren bei -60° 8 ml 1M Bortribromid in Methylenchlorid gegeben. Das Reaktionsgemisch wurde 1 Stunde bei -60° gerührt und dann 18 Stunden bei -20° gehalten. Aufarbeitung wie in Beispiel 24 lieferte einen Feststoff, der nach Umkristallisation aus Aether-Hexan 0,9 g 4-[5-(2,3,4-Trichlor-5,6-dihydroxyphenyl)pentyloxy]-2-hydroxy-3-propylbenzoesäure, Smp. 193-196° lieferte.

Beispiel 128

Ein Gemisch von 2,5 g 6-Brom-1-(3,4-dimethoxy-2,5-dimethylphenyl)-1-hexanon, 1,53 g 2,4-Dihydroxy-3-propylbenzoesäuremethylester und 3,3 g Kaliumcarbonat in 50 ml Aceton und 5 ml DMF wurde 26 Stunden unter Rühren zum Rückfluss erhitzt. Nach Aufarbeitung wie in Beispiel 16 wurde das Rohprodukt durch HPLC mit 10% Aethylacetat-Hexan gereinigt und lieferte 3,4 g 4-[[6-(3,4-Dimethoxy2,5-dimethylphenyl)-6-oxohexyl]oxy]-2-hydroxy-3-propylbenzoesäuremethylester als Oel.

Beispiel 129

Eine Lösung von 3,4 g 4-[[6-(3,4-Dimethoxy-2,5-dimethylphenyl)-6-oxohexyl]oxy]-2-hydroxy-3-propylbenzoesäuremethylester in 90 ml Methanol und 30 ml 1N Natronlauge wurde 5 Stunden unter Rühren zum Rückfluss erhitzt. Aufarbeitung wie in Beispiel 24 und Umkristallisation des Rohprodukts aus Aether-Hexan lieferte 3 g 4-[[6-(3,4-Dimethoxy-2,5-dimethylphenyl)-6-oxohexyl]oxy]-2-hydroxy-3-propylbenzoesäure, Smp. 92-94°.

Beispiel 130

Zu 1 g 4-[[6-(3,4-Dimethoxy-2,5-dimethylphenyl)-6-oxohexyl]oxy]-2-hydroxy-3-propylbenzoesäure in 100 ml Methylenchlorid wurden 6,6 ml 1M Bortribromid in Methylenchlorid bei -70° gegeben. Das Gemisch wurde 1 Stunde bei -70° gerührt und dann 16 Stunden bei-20° gehalten. Aufarbeitung wie in Beispiel 32 und Umkristallisation des Rohprodukts aus Aether-Hexan lieferte 0,6 g 4-[[6-(3,4-Dihydroxy-2,5-dimethylphenyl)-6-oxohexyl]oxy]-2-hydroxy-3-propylbenzoesäure, Smp. 121-125°.

Beispiel 131

Ein Gemisch von 6 g 6-Brom-1-[3,4-dimethoxy-5-(1-methyläthyl)phenyl]-1-hexanon, 3,39 g 2,4-Dihydroxy-3-propylbenzoesäuremethylester und 7 g Kaliumcarbonat in 120 ml Aceton und 12 ml DMF wurde 19 Stunden unter Rühren zum Rückfluss erhitzt. Nach Aufarbeitung wie in Beispiel 16 wurde das Rohprodukt durch HPLC mit 16% Aethylacetat-Hexan gereinigt. Man erhielt 7,7 g 4-[[6-[3,4-Dimethoxy-5-(1-methyläthyl)phenyl)-6-oxohexyl]oxy]-2-hydroxy-3-propylbenzoesäuremethylester als Oel.

Beispiel 132

Eine Lösung von 7,7 g 4-[[6-[3,4-Dimethoxy-5-(1-methyläthyl-phenyl)-6-oxohexyl]oxy]-2-hydroxy-3-propylbenzoesäuremethylester in 80 ml Methanol und 60 ml 1N Natronlauge wurde 5 Stunden zum Rückfluss erhitzt. Aufarbeitung wie in Beispiel 24 und Umkristallisation des Rohprodukts aus Aethylacetat-Hexan lieferte 6,6 g 4-[[6-[3,4-Dimethoxy-5-(1-methyläthyl)phenyl]-6-oxohexyl]oxy]-2-hydroxy-3-propylbenzoesäure, Smp. 113-115°.

Beispiel 133

Zu 2 g 4-[[6-[3,4-Dimethoxy-5-(1-methyläthyl)phenyl]-6-oxohexyl]oxy]-2-hydroxy-3-propylbenzoesäure in 200 ml Methylenchlorid wurde unter Kühlung auf -65° 13 ml 1M Bortribromid in Methylenchlorid gegeben. Die Suspension wurde 2 Stunden bei -50° gerührt und dann 16 Stunden bei -20° gehalten. Aufarbeitung wie in Beispiel 32 und Umkristallisation des Rohprodukts aus Aether-Hexan lieferte 1,5 g 4-[[6-[3,4-Dihydroxy-5-(1-methyläthyl)phenyl] -6-oxohexyl]oxy]-2-hydroxy-3-propylbenzoesäure, Smp. 169-171°.

Beispiel 134

Ein Gemisch von 2,5 g 4-[[6-[3,4-Dimethoxy-5-(1-methyläthyl)phenyl]-6-oxohexyl]oxy]-2-hydroxy-3-propylbenzoesäure in 50 ml Tetrahydrofuran die 2 Tropfen konzentrierte Schwefelsäure enthielten und 0,5 g 10% Palladium/Kohle wurde in einem Parr-Hydrierapparat unter einem Anfangswasserstoffdruck von 3,6 bar 20 Stunden geschüttelt. Das Reaktionsgemisch wurde über ein Celitfilterbett filtriert und das Filtrat unter vermindertem Druck eingeengt. Der Rückstand wurde in Aether gelöst und mit Wasser gewaschen. Der Extrakt wurde getrocknet und zu einem Feststoff eingeengt der nach Umkristallisation aus Hexan 1,4 g 4-[-[6-[3,4-Dimethoxy-5-(1-methyläthyl)phenyl]hexyloxy]-2-hydroxy-3-propylbenzoesäure, Smp. 106-108° lieferte.

Beispiel 135

Zu 2,43 g 4-[[6-(3,4-Dimethoxy-5-(1-methyläthyl)phenyl]hexyloxy]-2-hydroxy-3-propylbenzoesäure in 250 ml Methylenchlorid wurden bei -75° 16 ml 1M Bortribromid in Methylenchlorid gegeben. Das Reaktionsgemisch wurde 1 Stunde bei -75° gerührt und dann 17 Stunden bei -20° erhalten. Aufarbeitung wie in Beispiel 32 und Umkristallisation aus Aether-Hexan lieferte 1,2 g 4-[[6-(3,4-Dimethoxy-5-(1-methyläthyl)phenyl]hexyloxy]-2-hydroxy-3-propylbenzoesäure, Smp. 138-140°.

Beispiel 136

Ein Gemisch von 1,45 g 4-[[6-(3,4-Dimethoxy-2,5-dimethylphenyl)-6-oxohexyl]oxy]-2-hydroxy-3-propylbenzoesäure in 40 ml Tetrahydrofuran die 2 Tropfen konzentrierte Schwefelsäure enthielten und 0,4 g 10% Palladium/Kohle wurde in einem Parr-Hydrierapparat unter einem Wasserstoffanfangsdruck von 3,6 bar 20 Stunden hydriert. Das Reaktionsgemisch wurde wie in Beispiel 134 aufgearbeitet und das Produkt aus Aether-Hexan umkristallisiert. Man erhielt 1,2 g 4-[[6-(3,4-Dimethoxy-2,5- -dimethylphenyl)hexyloxy]-2-hydroxy-3-propylbenzoesäure, Smp. 109-112°.

Beispiel 137

Zu 1,2 g 4-[[6-(3,4-Dimethoxy-2,5-dimethylphenyl)hexyloxy]-2-hydroxy-3-propylbenzoesäure in 120 ml Methylenchlorid wurden bei -75° 8 ml 1M Bortribromid in Methylenchlorid gegeben. Das Reaktionsgemisch wurde 1 Stunde bei -75° gerührt und 17 Stunden bei -20° gehalten. Aufarbeitung wie in Beispiel 32 und Umkristallisation des Rohprodukts aus Aether-Hexan lieferte 0,6 g 4-[[6-(3,4-Dimethoxy-2,5-dimethylphenyl)-hexyloxy]-2-hydroxy-3-propylbenzoesäure, Smp. 170-171°.

Beispiel 138

Eine Lösung von 5,3 g 1,2-Dimethoxy-4-fluorbenzol in 25 ml Methylenchlorid wurde unter Eisbadkühlung zu einer Lösung von 5,4 g Aluminiumchlorid und 8,7 g Bromhexanoylchlorid in 60 ml Methylenchlorid gegeben. Die erhaltene Lösung wurde 5 Stunden bei 0° gehalten und dann wie in Beispiel 117 aufgearbeitet. Das Rohprodukt wurde aus Methylenchlorid-Aether kristallisiert und lieferte 6,98 g 6-Brom-1-(2-fluor-4,5-dimethoxyphenyl)-1-hexanon, Smp. 81-83°

Beispiel 139

Ein Gemisch von 4 g 6-Brom-1-(2-fluor-4,5-dimethoxyphenyl)-1-hexanon,2,5 g 2,4-Dihydroxy-3-propylbenzoesäuremethylester und 5 g Kaliumcarbonat in 80 ml Aceton und 8 ml DMF wurde 86 Stunden unter Rühren zum Rückfluss erhitzt. Danach wurden 12 ml DMF und 3 g Kaliumcarbonat zugesetzt und es wurde weitere 18 Stunden zum Rückfluss erhitzt. Aufarbeitung wie in Beispiel 16 und Umkristallisation des

Rohprodukts aus Aethylacetat lieferte 4,3 g 4-[[6-(2-Fluor-4,5-dimethoxyphenyl)-6-oxohexyl]oxy]-2-hydroxy-3-propylbenzoesäuremethylester, Smp. 127-129°.

Beispiel 140

Eine Lösung von 4,3 g 4-[[6-(2-Fluor-4,5-dimethoxy-6-oxohexyl]oxy]-2-hydroxy-3-propylbenzoesäuremethylester in 135 ml Methanol und 45 ml 1N Natronlauge wurde 5 Stunden unter Rühren zum Rückfluss erhitzt. Aufarbeitung wie in Beispiel 24 und Umkristallisation des Rohprodukts aus Aethylacetat-Hexan lieferte 3,7 g 4-[[6-(2-Fluor-4,5-dimethoxyphenyl)-6-oxohexyl] oxy]-2-hydroxy-3-propylbenzoesäure, Smp. 146-147°.

Beispiel 141

Zu 2 g 4-[[6-(2-Fluor-4,5-dimethoxyphenyl)-6-oxohexyl]oxy]-2-hydroxy-3-propylbenzoesäure in 200 ml Methylenchlorid wurden bei -75° 13 ml 1M Bortribromid in Methylenchlorid gegeben. Das Reaktionsgemisch wurde 1 Stunde bei -75° gerührt und 17 Stunden bei -20° gehalten. Danach wurden weitere 7,5 ml Bortribromid zugesetzt und das Gemisch wurde 5 Stunden bei -5° gerührt. Aufarbeitung wie in Beispiel 32 und Umkristallisation des Rohprodukts aus Aether-Hexan lieferte 0,84 g 4-[[6-(2-Fluor-4,5-dihydroxyphenyl)-6-oxohexyl]oxy]-2-hydroxy-3-propylbenzoesäure, Smp. 193-195°.

Beispiel 142

Ein Gemisch von 0,4 g 4-[[6-(2-Fluor-4,5-dimethoxyphenyl)-6-oxohexyl]oxy]-2-hydroxy-3-propylbenzoesäure und 0,15 g 10% Palladium/Kohle in 20 ml Tetrahydrofuran die 2 Tropfen konzentrierte Schwefelsäure enthielten wurde unter einem Anfangswasserstoffdruck von 3,5 bar in einem Parr-Hydrierapparat 20 Stunden geschüttelt. Das Reaktionsgemisch wurde über ein Celitfilterbett filtriert und das Filtrat unter vermindertem Druck zu einem Feststoff eingeengt. Umkristallisation aus Aethylacetat-Hexan lieferte 0,37 g 4-[6-(2-Fluor-4,5-dimethoxyphenyl)-hexyloxy]-2-hydroxy-3-propylbenzoesäure, Smp. 127-130°.

Beispiel 143

Zu 0,36 g 4-[6-(2-Fluor-4,5-dimethoxyphenyl)hexyloxy]-2-hydroxy-3-propylbenzoesäure in 60 ml Methylenchlorid wurden bei -75° 2,8 ml 1M Bortribromid in Methylenchlorid gegeben. Das Reaktionsgemisch wurde 30 Minuten bei -75° gerührt und dann 20 Stunden bei -20° gehalten. Aufarbeitung wie in Beispiel 32 und Umkristallisation des Rohprodukts aus Aether-Hexan lieferte 0,18 g 4-[6-(2-Fluor-4,5-dihydroxyphenyl)-hexyloxy]-2-hydroxy-3-propylbenzoesäure, Smp. 110-111°.

Beispiel 144

Eine Lösung von 2,956 g 1,2-Dimethoxy-3,4,6-trimethylbenzol in 10 ml Methylenchlorid wurde unter Eisbadkühlung zu 2,4 g Aluminiumchlorid und 3,8 g 6-Bromhexanoylchlorid in 30 ml Methylenchlorid gegeben. Die Lösung wurde 45 Minuten bei 3° und 42 Stunden bei 23° gehalten. Danach wurde weitere 2 g Aluminiumchlorid und 3 g 6-Bromhexanoylchlorid zugesetzt und das Reaktionsgemisch 22 Stunden unter Rühren zum Rückfluss erhitzt. Aufarbeitung wie in Beispiel 117 und Reinigung durch HPLC mit 4% Aethylacetat-Hexan lieferte 0,95 g 6-Brom-1-(3,4-dimethoxy-2,5,6-trimethylphenyl)-1-hexanon als Oel.

Beispiel 145

Ein Gemisch von 0,194 g 6-Brom-1-(3,4-dimethoxy-2,5,6-trimethylphenyl)-1-hexanon, 0,55 g 2,4-Dihydroxy-3-propylbenzoesäuremethylester und 1,1 g Kaliumcarbonat in 20 ml Aceton und 2 ml DMF wurde 17 Stunden unter Rühren zum Rückfluss erhitzt. Nach Aufarbeitung wie in Beispiel 16 wurde das Rohprodukt durch Chromatographie in 100 g Silicagel gereinigt. Elution mit 10% Aethylacetat-Hexan lieferte 1,05 g 4-[[6-(3,4-Dimethoxy-2,5,6-trimethylphenyl)-6-oxohexyl]oxy]-2-hydroxy-3-propylbenzoesäuremethylester.

Beispiel 146

Eine Lösung von 1,05 g 4-[[6-(3,4-Dimethoxy-2,5,6-trimethylphenyl)-6-oxohexyl]oxy]-2-hydroxy-3-propylbenzoesäuremethylester in 30 ml Methanol und 7 ml 1N Natronlauge wurde 7 Stunden unter Rühren zum Rückfluss erhitzt. Aufarbeitung wie in Beispiel 24 lieferte 0,98 g 4-[[6-(3,4-Dimethoxy-2,5,6-trimethylphenyl)-6-oxohexyl]oxy]-2-hydroxy-3-propylbenzoesäure, Smp. 108-112°.

Beispiel 147

Zu 0,98 g 4-[[6-(3,4-Dimethoxy-2,5,6-trimethylphenyl)-6-oxohexyl]oxy]-2-hydroxy-3-propylbenzoesäure in 100 ml Methylenchlorid wurden bei -75° 7 ml 1M Bortribromid in Methylenchlorid gegeben. Das Reaktionsgemisch wurde 30 Minuten bei -75° gerührt und dann 18 Stunden bei -20° gehalten. Aufarbeitung wie in Beispiel 32 und Umkristallisation des Rohprodukts aus Aether-Hexan lieferte 0,51 g 4-[[6-(3,4-Dihydroxy-2,5,6-trimethylphenyl)-6-oxohexyl]oxy]-2-hydroxy-3-propylbenzoesäure, Smp. 169-170°.

Beispiel 148

Ein Gemisch von 1 g 4-[6-(2,3-Dihydroxyphenyl)hexyloxy]-2-hydroxy-3-propylbenzoesäure, 0,76 ml n-Hexyljodid und 0,26 g Natriumbicarbonat in 15 ml wasserfreiem Dimethylformamid wurde 16 Stunden unter Rühren auf 70° erwärmt. Das Lösungsmittel wurde an der Oelpumpe entfernt, Wasser wurde zugesetzt und das Produkt mit Aethylacetat extrahiert. Der getrocknete Extrakt wurde unter vermindertem Druck zu einem Oel eingeengt das durch Chromatographie an Silicagel mit 5% Aethylacetat-Toluol gereinigt wurde. Man erhielt 1,01 g 4-[6-(2,3-Dihydroxyphenyl)hexyloxy]-2-hydroxy-3-propylbenzoesäurehexylester als Oel.

Beispiel 149

Ein Gemisch von 5,8 g 1-(6-Bromhexyl)-2,3-bis(phenylmethyl)benzol, 3,5 g 3-Hydroxy-4-nitrobenzoesäurephenylmethylester, 3,5 g wasserfreies Kaliumcarbonat und 2,9 g Natriumjodid in 125 ml Aceton und 13 ml Dimethylformamid wurde 42 Stunden unter Rühren zum Rückfluss erhitzt. Aufarbeitung wie in Beispiel 16 und Reinigung durch HPLC mit 10% Aethylacetat-Hexan lieferte 5,45 g 4-Nitro-3-[6-[2,3-bis-(phenylmethoxy)phenyl]hexyloxy]benzoesäurephenylmethylester als Oel.

Beispiel 150

Eine Lösung von 5,4 g 4-Nitro-3-[6-[2,3-bis(phenylmethoxy)phenyl]hexyloxy]-benzoesäurephenylmethylester in 250 ml Tetrahydrofuran und 1 g 10% Palladium/Kohle wurde 17 Stunden bei Raumtemperatur unter Wasserstoffatmosphäre geschüttelt. Der Katalysator wurde über Celit abfiltriert und das Filtrat unter vermindertem Druck zu einem Feststoff eingeengt. Umkristallisation aus Aether-Methylenchlorid lieferte 1,8 g 4-Amino-3-[6-(2,3-dihydroxyphenyl)hexyloxy]benzoesäure,Smp. 130-132°.

Beispiel 151

Ein Gemisch von 5,8 g 1-(6-Bromhexyl)-2,3-bis(phenylmethoxy)benzol, 3,5 g 4-Hydroxy-3-nitrobenzoesäurephenylmethylester, 3,5 g Kaliumcarbonat und 2,9 g Natriumjodid in 125 ml Aceton und 13 ml Dimethylformamid wurde 5 Tage unter Rühren zum Rückfluss erhitzt. Aufarbeitung wie in Beispiel 16 und Reinigung durch HPLC mit 50% Methylenchlorid-Hexan lieferte 6,85 g 3-Nitro-4-[6-[2,3-bis(phenylmethoxy)-phenyl]hexyloxy]benzoesäurephenylmethylester als Oel.

Beispiel 152

Ein Lösung von 6,8 g 3-Nitro-4-[6-[2,3-bis(phenylmethoxy)phenyl]hexyloxy]-benzoesäurephenylmethylester in 250 ml Tetrahydrofuran und 1,3 g 10% Palladium/Kohle wurde 12 Stunden bei Raumtemperatur in einer Wasserstoffatmosphäre geschüttelt. Der Katalysator wurde über Celit abfiltriert und das Filtrat unter vermindertem Druck zu einem Feststoff eingeengt. Umkristallisation aus Aceton-Hexan lieferte 2,36 g 3-Amino-4-[6-(2,3-dihydroxyphenyl)hexyloxy]benzoesäure, Smp. 172-174°.

Beispiel 153

Zu 27 g 1,2-Dimethoxy-3-(1,1-dimethyläthyl)benzol in 350 ml wasserfreiem Tetrahydrofuran wurde unter Eiskochsalzkühlung bei -5° 87 ml 1,6M Butyllithium in Hexan im Verlauf von 30 Minuten gegeben. Das Reaktionsgemisch wurde 3 Stunden bei -5° gerührt und dann 1 Stunde unter Rückfluss. Nach Kühlen in einem Eisbad wurden 21,5 ml 1,6-Dibromhexan in 75 ml Tetrahydrofuran tropfenweise zugesetzt. Das Reaktionsgemisch wurde dann 17 Stunden unter Rühren zum Rückfluss erhitzt. Das Lösungsmittel wurde unter vermindertem Druck entfernt, 50 ml 3N Salzsäure wurden zugesetzt und das Produkt mit Aether extrahiert. Der Extrakt wurde mit Natriumbicarbonatlösung gewaschen, getrocknet und unter vermindertem Druck zu einem Oel eingeengt. Reinigung durch HPLC mit 20% Toluol-Hexan lieferte 7,6 g 1-(6-Bromhexyl)-2,3-dimethoxy-4-(1,1-dimethyläthyl)benzol.

Beispiel 154

Ein Gemisch von 4 g 1-(6-Bromhexyl)-2,3-dimethoxy-4-(1,1-dimethyläthyl)benzol, 3,2 g 2,4-Dihydroxy-3-propylbenzoesäurephenylmethylester, 3,1 g Kaliumcarbonat und 1,7 g Natriumjodid in 70 ml wasserfreiem Aceton und 7 ml wasserfreiem Dimethylformamid wurde 31 Stunden unter Rühren zum Rückfluss erhitzt. Aufarbeitung wie in Beispiel 16 und Reinigung durch HPLC mit 5% Aethylacetat-Hexan lieferte 5,3 g 2-Hydroxy-4-[6-[2,3-dimethoxy-4-(1,1-dimethyläthyl)phenyl]hexyloxy]-3-propylbenzoesäurephenylmethylester als Oel.

Eine Lösung von 2-Hydroxy-4-[6-[2,3-dimethoxy-4-(1,1-dimethyläthyl)phenyl]hexyloxy]-3-propylbenzoesäurephenylmethylester in 200 ml Tetrahydrofuran und 0,5 g 10% Palladium/Kohle wurde 3 Stunden in Wasserstoffatmosphäre geschüttelt. Das Reaktionsgemisch wurde durch Celit filtriert und das Filtrat eingeengt wobei 4 g 2-Hydroxy-4-[6-[2,3-dimethoxy-4-(1,1-dimethyläthyl)phenyl]hexyloxy]-3-propylbenzoesäure, Smp. 106-108° erhalten wurden.

Beispiel 155

Zu 4 g 2-Hydroxy-4-[6-[2,3-dimethoxy-4-(1,1-dimethyläthyl)phenyl]hexyloxy]-3-propylbenzoesäure, in 200 ml Methylenchlorid suspendiert, wurden bei -70° 26 ml 1M Bortribromid in Methylenchlorid im Verlauf von 30 Minuten zugegeben. Das Reaktionsgemisch wurde 30 Minuten bei 70° gerührt und 41 Stunden bei -20° gehalten. Aufarbeitung wie in Beispiel 32 und Umkristallisation des Rohprodukts aus Aether-Hexan lieferte 1,78 g 4-[6-[2,3-Dihydroxy-4-(1,1-dimethyläthyl)phenyl]hexyloxy]-2-hydroxy-3-propylbenzoesäure, Smp. 85-87°.

Beispiel 156

Ein Gemisch von 2,4 g 1-(6-Bromhexyl)-2,3-bis-(phenylmethoxy)benzol, 0,88 g 4-Hydroxybenzoesäureäthylester, 2,6 g Kaliumcarbonat und 0,8 Natriumjodid in 40 ml Aceton wurde 22 Stunden unter Rühren zum Rückfluss erhitzt. Aufarbeitung wie in Beispiel 16 und Umkristallisation des Rohproukts aus Aethylacetat-Hexan lieferte 2,3 g 4-[6-[2,3-bis(Phenylmethoxy)phenyl]hexyloxy]benzoesäureäthylester, Smp. 63-65°.

Eine Lösung von 2,3 g 4-[6-[2,3-bis(Phenylmethoxy)phenyl]hexyloxy]benzoesäureäthylester in 50 ml Aethylacetat und 0,3 g 10% Palladium/Kohle wurde 22 Stunden in einer Wasserstoffatmosphäre gerührt. Das Reaktionsgemisch wurde durch Celit filtriert und das Filtrat unter vermindertem Druck zu einem Feststoff eingeengt. Umkristallisation aus Aethylacetat-Hexan lieferte 1,3 g 4-[6-(2,3-Dihydroxyphenyl)-hexyloxy]benzoesäureäthylester, Smp. 45-47°.

Beispiel 157

Ein Gemisch von 1 g 4-[6-(2,3-Dihydroxyphenyl)hexyloxy]-2-hydroxybenzoesäure, 0,295 g Natriumbicarbonat und 2,4 ml Aethyljodid in 10 ml wasserfreiem Dimethylformamid wurde 6 Stunden bei 50° gerührt. Das Lösungsmittel wurde an der Oelpumpe entfernt und der Rückstand mit Natriumbicarbonatlösung versetzt. Das Produkt wurde mit Aethylacetat extrahiert und der getrocknete Extrakt unter vermindertem Druck zu einem Feststoff eingeengt. Umkristallisation aus Aether-Hexan lieferte 0,8 g 4-[6-(2,3-Dihydroxyphenyl)hexyloxy]-2-hydroxybenzoesäure-äthylester, Smp. 63-68°.

Beispiel 158

Ein Gemisch von 0,170 g 4-[6-(2,3-Dihydroxy-2,5-dimethylphenyl)hexyloxy]-2-hydroxy-3-propylbenzoesäure, 0,157 g Natriumbicarbonat und 1,4 ml Aethyljodid in 10 ml Dimethylformamid wurde 10 Stunden bei 50° gerührt. Aufarbeitung wie in Beispiel 16 lieferte ein Oel, das durch Chromatographie an 20 g Silicagel gereinigt wurde. Elution mit 25% Aethylacetat-Hexan lieferte ein Produkt das nach Umkristallisation aus Hexan 0,5 g 4-[6-(3,4-Dihydroxy-2,5-dimethylphenyl)hexyloxy]-2-hydroxy-3-propylbenzoesäureäthylester, Smp. 61-64° lieferte.

Beispiel 159

Tablettenformulierung (Feuchtgranulierung)

| Bestandteil | mg/Tablette | | |
|---|---|---|---|
| | 100 mg | 500mg | 1000mg |
| 1. 4-[6-(2,3-Dihydroxyphenyl) hexyloxy]-2-hydroxy-3-propyl-benzoesäure | 100 | 500 | 1000 |
| 2. Lactose | 132 | -- | -- |
| 3. Vorgelatinisierte Stärke | 16 | 30 | 50 |
| 4. Modifizierte Stärke | 30 | 40 | 50 |
| 5. Magnesiumstearat | 2 | 6 | 8 |
| TOTAL | 280 | 576 | 1108 |

Verfahren:

1. Bestandteile 1, 2, 3 und 4 werden gemischt und mit Wasser granuliert.
2. Das Granulat wird bei 50° getrocknet.
3. Das Granulat wird durch eine geeignete Mühle gegeben.
4. Bestandteil 5 wird zugesetzt und 3 Minuten zugemischt, das Gemisch wird verpresst

Beispiel 160

Kapsel

| Bestandteil | mg/Kapsel | | | |
|---|---|---|---|---|
| 1. 4-[6-(2,3-Dihydroxyphenyl) hexyloxy]-2-hydroxy-3-propylbenzoesäure | 25 | 50 | 100 | 500 |
| 2. wasserhaltige Lactose | 143 | 168 | 148 | -- |
| 3. Maisstärke | 20 | 20 | 40 | 70 |
| 4. Talk | 10 | 10 | 10 | 25 |
| 5. Magnesiumstearat | 2 | 2 | 2 | 5 |
| TOTAL | 200 | 250 | 300 | 600 |

Verfahren:

1. Die Bestandteile 1, 2 und 3 werden 30 Minuten gemischt.
2. Die Bestandteile 4 und 5 werden 3 Minuten zugemischt.
3. Die Masse wird in Kapseln abgefüllt.

Beispiel 161

Feuchtgranulat

| Bestandteil | mg/Tablette | |
|---|---|---|
| 1. 4-[6-(2,3-Dihydroxyphenyl) hexyloxy]-2-hydroxy-3-propylbenzoesäure | 25 | 50 |
| 2. Polyvinylpyrrolidon | 5 | 10 |
| 3. wasserfreie Lactose | 133 | 142 |
| 4. Microkristalline Cellulose | 25 | 30 |
| 5. Modifizierte Stärke | 10 | 15 |
| 6. Magnesiumstearat | 2 | 3 |
| TOTAL | 200 | 250 |

Verfahren:

1. Bestandteil 2 wird in Wasser gelöst.
2. Bestandteile 1, 3, 4 und 5 werden gemischt und mit der Lösung von Stufe 1 granuliert.
3. Die Masse wird über Nacht bei 45° getrocknet, gesiebt und Bestandteil 6 wird zugemischt. Die Masse wird zu Tabletten verpresst..

Beispiel 162

Crème

| Bestandteil | g/kg | möglicher Variationsbereich |
|---|---|---|
| 1. 4-[6-(2,3-Dihydroxyphenyl) hexyloxy]-2-hydroxy-3-propylbenzoesäure | 51.50 | -- |
| 2. Glycerylmonostearat | 100.00 | 80 - 120 |
| 3. Polysorbat 60 (Tween 69) | 20.00 | 15 - 25 |
| 4. Cetylalcohol | 50.00 | 40 - 60 |
| 5. Petrolatum | 70.00 | 50-90 |
| 6. Methylparaben | 1.50 | 1.25-1.75 |
| 7. Propylparaben | 0.50 | 0.4-0.6 |
| 8. Propylenglycol | 200.00 | 150-250 |
| 9. reines Wasser | 521.70 | 475-575 |
| Total | 1015.20 | |

Beispiel 163

Weichgelatinekapseln

| Bestandteil | mg/Kapsel | |
|---|---|---|
| 1. 4-[6-(2,3-Dihydroxyphenyl) hexyloxy]-2-hydroxy-3-propylbenzoesäure | 50 | 150 |
| 2. Polyäthylenglycol 400 | 325 | 550 |
| 3. Monoglyceride | 100 | 150 |
| 4. Polysorbat 80 | 25 | 50 |
| TOTAL | 500 | 1000 |

Verfahren:

1. Bestandteil 1 wird in Bestandteil 2 gelöst.
2. Bestandteil 3 wird zugemischt.
3. Bestandteil 4 wird zugemischt.
4. Die Masse wird in Weichgelatinekapseln abgefüllt.

Beispiel 164

Kügelchen (für enterale Applikation)

| Bestandteil | mg/Kapsel | | |
|---|---|---|---|
| 1. 4-[6-(2,3-Dihydroxyphenyl) hexyloxy]-2-hydroxy-3-propylbenzoesäure | 25 | 100 | 250 |
| 2. Microkristalline Cellulose | 100 | 200 | 250 |
| 3. Polyvinylpyrrolidon K-90 | 10 | 20 | 30 |
| TOTAL | 135 | 320 | 530 |

Verfahren:

1. Der Wirkstoff wird mit der mikrokristallinen Cellulose gemischt und mit einer Lösung von Polyvinylpyrrolidon K-90 granuliert.
2. Das Granulat wird durch eine Schneckenpresse gegeben und zu Kügelchen verarbeitet.
3. Die Kügelchen werden mit einem Ueberzug für enterale Applikation wie Polyvinylacetatphthalat, Hydroxypropylmethylcellulosephthalat, Celluloseacetatphthalat oder einem Acrylsäurepolymer versehen.
4. Die Kügelchen werden in Kapseln abgefüllt.

**Patentansprüche**

**Patentansprüche für folgende Vertragsstaaten : AT, BE, CH, DE, FR, GB, IT, LI, LU, NL, SE**

**1.** Verbindungen der Formel

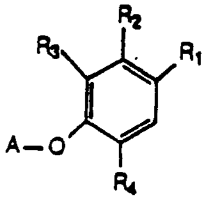

I

worin $R_1$ -C(O)OR, Wasserstoff, Acetyl, Hydroxy oder Alkanoyloxy; $R_2$ -C(O)OR, Hydroxy, Wasserstoff oder Alkanoyloxy; R Wasserstoff, nieder-Alkyl oder $-(CH_2)_nN$ (nieder-Alkyl)$_2$; $R_3$ Wasserstoff, nieder-Alkyl oder Amino; $R_4$ Wasserstoff, nieder-Alkyl, Halogen oder Amino darstellt, A eine Gruppe der Formel

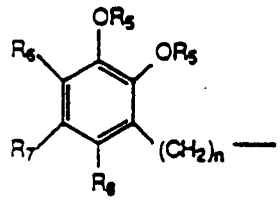

A'

darstellt, worin $R_5$ Wasserstoff oder Acyl; $R_6$ Wasserstoff, Halogen, nieder-Alkyl, Aryl oder Cycloalkyl; und $R_7$ und $R_8$ unabhängig voneinander Wasserstoff, nieder-Alkyl oder Halogen darstellen und n eine ganze Zahl von 2-10 ist, oder A eine Gruppe der Formel

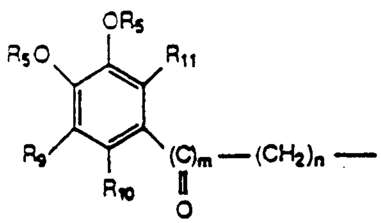

A"

darstellt, worin $R_5$ Wasserstoff oder Acyl; $R_9$ Wasserstoff oder nieder-Alkyl; $R_{10}$ Wasserstoff, nieder-Alkyl oder Halogen; $R_{11}$ Wasserstoff, nieder-Alkyl, Cycloalkyl oder Halogen darstellt, m 0 oder 1 und n eine ganze Zahl von 2-10 ist; vorausgesetzt, dass nicht mehr als ein Rest $R_1$ oder $R_2$ Hydroxy, Alkanoyloxy oder -C(O)OR ist,

und, falls R Wasserstoff ist, Salze davon mit pharmazeutisch akzeptablen Basen, und, falls R $(CH_2)_n$-N-(nieder-Alkyl)$_2$ ist, Säureadditionssalze davon mit pharmazeutisch akzeptablen Säuren.

**2.** Verbindungen der Formel

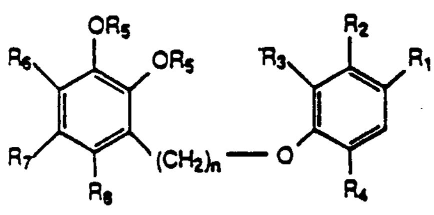

Ia

in denen $R_1$ Carboxy oder Acetyl, $R_2$ Hydroxy, $R_3$ Wasserstoff oder Propyl, $R_4$ Wasserstoff oder Chlor, n eine ganze Zahl von 2-10, $R_5$ Wasserstoff oder Acetyl, $R_6$ Wasserstoff, nieder-Alkyl oder Aryl und $R_7$ und $R_8$ Wasserstoff sind.

**3.** Verbindungen der Formel

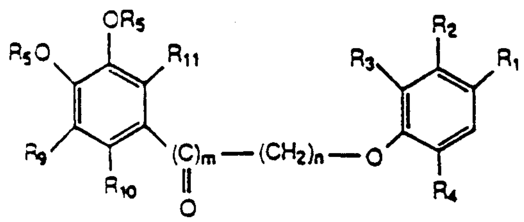

Ib

in denen $R_1$ Carboxy oder Acetyl, $R_2$ Hydroxy, $R_3$ Wasserstoff oder Propyl, $R_4$ Wasserstoff oder Chlor, m 0 oder 1, n eine ganze Zahl von 2-10, $R_5$ Wasserstoff oder Acetyl, $R_9$ und $R_{10}$ Wasserstoff und $R_{11}$ Wasserstoff oder Chlor sind.

**4.** Verbindungen der Formel I von Anspruch 1, in denen $R_6$ Wasserstoff, nieder-Alkyl, Aryl oder Cycloalkyl ist und die übrigen Symbole die in Anspruch 1 angegebene Bedeutung haben; und, falls R Wasserstoff ist, Salze davon mit pharmazeutisch akzeptablen Basen, und, falls R-$(CH_2)_n$-N-(nieder-Alkyl)$_2$ ist, Säureadditionssalze davon mit pharmazeutisch akzeptablen Säuren.

**5.** 4-[6-(2,3-Dihydroxyphenyl)hexyloxy]-2-hydroxybenzoesäure.

**6.** 4-[4-(2,3-Dihydroxyphenyl)butoxy]-2-hydroxy-3-propylbenzoesäure;
4-[6-(2,3-Dihydroxyphenyl)hexyloxy]-2-hydroxy-3-propylbenzoesäure;
4-[8-(2,3-Dihydroxyphenyl)octyloxy]-2-hydroxy-3-propylbenzoesäure;
4-[6-(2,3-Bis(acetyloxy)phenyl]hexyloxy]2-hydroxy-3-propylbenzoesäure; und
4-[6-[2,3-Dihydroxy-4-(1-methylethyl)phenyl]hexyloxy]-2-hydroxy-3-propylbenzoesäure.

**7.** 4-[3-(3,4-Dihydroxyphenyl)propoxy]-2-hydroxy-3-propylbenzoesäure;
4-[6-(3,4-Dihydroxyphenyl)hexyloxy]-2-hydroxy-3-propylbenzoesäure;
4-[[6-(3,4-Dihydroxyphenyl)-6-oxohexyl]oxy-2-hydroxy-3-propylbenzoesäure; und
1-[2-Hydroxy-4-[4-(2,3-dihydroxyphenyl)butoxy]-3-propylphenyl]äthanon.

**8.** 4-[6-[2,3-Dihydroxy-4-(1,1-dimethyläthyl)phenyl]hexyloxy]-2-hydroxy-3-propylbenzoesäure;
4-[6-[2,3-Dihydroxy-4-methylphenyl]hexyloxy]-2-hydroxy-3-propylbenzoesäure;
4-[4-[2,3-Dihydroxy-4-(2-methylpropyl)phenyl]butoxy]-2-hydroxy-3-propylbenzoesäure;
4-[6-[2,3-Dihydroxy-5,6-dimethylphenyl]hexyloxy]-2-hydroxy-3-propylbenzoesäure;
4-[5-[5-Chlor-2,3-dihydroxyphenyl]pentyloxy]-2-hydroxy-3-propylbenzoesäure;
4-[6-[2,3-Dihydroxy-6-fluorophenyl]hexyloxy]2-hydroxy-3-propylbenzoesäure;
4-[4-[2,3-Dihydroxy-4-cyclohexylphenyl]butoxy]-2-hydroxy-3-propylbenzoesäure;
4-[4-[2,3-Dihydroxy-4-(1,1-dimethyläthyl)phenyl]butoxy]-2-hydroxy-3-propylbenzoesäure;
4-[8-[2,3-Dihydroxy-4-(1,1-dimethyläthyl)phenyl]octyloxy]-2-hydroxy-3-propylbenzoesäure;
4-[4-[2,3-Dihydroxy-4-(1,1-dimethyläthyl)phenyl]butoxy]-2-hydroxybenzoesäure;
4-[8-[2,3-Dihydroxy-4-(1-methyläthyl)phenyl]octyloxy]-2-hydroxybenzoesäure;
4-[4-[2,3-Dihydroxyphenyl]butoxy]-3,5-dipropyl-2-hydroxybenzoesäure;
4-[4-[2,3-Dihydroxy-4-(1-methyläthyl)phenyl]butoxy]-3,5-dipropylbenzoesäure;
4-[6-[2,3-Dihydroxy-4-(1-methyläthyl)phenyl]hexyloxy]-3-propylbenzoesäure;
4-[6-[2,3-Bis(acetyloxy)-4-(1-methyläthyl)phenyl]hexyloxy]-2-hydroxy-3-propylbenzoesäure;
2-Acetyloxy-4-[6-(2,3-dihydroxy-4-(1-methyläthyl)phenyl]hexyloxy]-3-propylbenzoesäure;
4-[6-[2,3-Bis[4-methylbenzoyl)oxy]-4-(1-methyläthyl)phenyl]hexyloxy]-2-hydroxy-3-propylbenzoesäure;
1-[2-Hydroxy-4-[6-[2,3-dihydroxy-4-(1,1-dimethyläthyl)phenyl]hexyloxy]-3-propylphenyl]äthanon;
1-[2-Hydroxy-4-[8-[2,3-dihydroxy-6-fluorphenyl]octyloxy]-3-propylphenyl]äthanon;
1-[2-Hydroxy-4-[6-[6-chlor-2,3-dihydroxyphenyl]hexyloxy]-3-propylphenyl]äthanon;
1-[2-Hydroxy-4-[6-[5,6-dichlor-2,3-dihydroxyphenyl]hexyloxy]-3-propylphenyl]äthanon;

1-[2-Hydroxy-4-[6-[2,3-dihydroxy-4,5,6-trichlorphenyl]hexyloxy]-3-propylphenyl]äthanon;
5-[6-(2,3-Dihydroxy-4-(1-methyläthyl)phenyl]hexyloxy]-2-hydroxybenzoesäure;
4-[6-(2,3-Dihydroxy-4-(1-methyläthyl)phenyl]hexyloxy]benzoesäure;
4-[6-(2,3-Dihydroxy-4-(1-methyläthyl)phenyl]hexyloxy]-2-hydroxy-3-propylbenzoesäure-äthylester;
4-[6-(2,3-Dihydroxy-4-(1-methyläthyl)phenyl]hexyloxy]-2-hydroxy-3-propylbenzoesäure-äthylester;
4-[8-(3,4-Dihydroxyphenyl)octyloxy]-2-hydroxy-3-propylbenzoesäure;
4-[3-(3,4-Dihydroxyphenyl)propoxy]-2-hydroxy-3-propylbenzoesäure-äthylester;
4-[6-(3,4-Dihydroxyphenyl)hexyloxy]-2-hydroxy-benzoesäure;
4-[6-(3,4-Dihydroxyphenyl)hexyloxy]-3-propylbenzoesäure;
4-[8-(3,4-Dihydroxyphenyl)octyloxy]benzoesäure;
3-[8-(3,4-Dihydroxyphenyl)octyloxy]benzoesäure;
5-[8-(3,4-Dihydroxyphenyl)octyloxy]-2-hydroxybenzoesäure;
4-[[8-(3,4-Dihydroxyphenyl)-8-oxooctyl]oxy]-2-hydroxy-3-propylbenzoesäure;
4-[[4-(3,4-Dihydroxyphenyl)-4-oxobutyl]oxy]-2-hydroxy-3-propylbenzoesäure;
4-[6-(3,4-Dihydroxy-5-fluorphenyl)hexyloxy]-2-hydroxy-3-propylbenzoesäure;
4-[6-(3,4-Dihydroxy-6-fluorphenyl)hexyloxy]-2-hydroxy-3-propylbenzoesäure;
4-[6-(3,4-Dihydroxy-6-chlorphenyl)hexyloxy]-2-hydroxy-3-propylbenzoesäure;
4-[4-[3,4-Dihydroxy-5-(1-methyläthyl)phenyl]butoxy]-2-hydroxy-3-propylbenzoesäure;
4-[4-[3,4-Dihydroxy-5-(1,1-dimethyläthyl)phenyl]butoxy]-2-hydroxybenzoesäure;
4-[6-[3,4-Bis(acetyloxy)phenyl]hexyloxy]-2-hydroxy-3-propylbenzoesäure;
2-Acetyloxy-4-[6-(3,4-dihydroxyphenyl)hexyloxy]-3-propylbenzoesäure;
4-[[4-[3,4-Dihydroxy-5-(1-methyläthyl)phenyl]-4-oxobutyl]oxy]-2-hydroxybenzoesäure;
4-[6-[3,4-Dihydroxy-2-(1-methyläthyl)phenyl]hexyloxy]-2-hydroxy-3-propylbenzoesäure;
4-[[4-(3,4-Dihydroxy-2,5-dimethylphenyl)-4-oxobutyl]oxy]-2-hydroxy-3-propylbenzoesäure;
4-[4-(3,4-Dihydroxy-2,5-dimethylphenyl)butoxy]-2-hydroxy-3-propylbenzoesäure;
4-[[6-(3,4-Dihydroxy-2,5-6-trimethylphenyl)-6-oxohexyl]oxy]-2-hydroxy-3-propylbenzoesäure;
4-[6-(3,4-Dihydroxy-5,6-trimethylphenyl)hexyloxy]-2-hydroxy-3-propylbenzoesäure;
1-[2-Hydroxy-4-[6-(3,4-dihydroxyphenyl)hexyloxy]-3-propylphenyl-1-äthanon;
1-[2-Hydroxy-4-[8-(3,4-dihydroxyphenyl)octyloxy]-3-propylphenyl-1-äthanon;
1-[2-Hydroxy-4-[6-(3,4-dihydroxy-2,5-dimethylphenyl)hexyloxy]-3-propylphenyl-1-äthanon;
1-[2-Hydroxy-4-[6-(3,4-dihydroxy-6-chlorphenyl)hexyloxy]-3-propylphenyl-1-äthanon;
1-[2-Hydroxy-4-[6-(3,4-dihydroxy-2-chlorphenyl)hexyloxy]-3-propylphenyl-1-äthanon;
1-[2-Hydroxy-4-[6-(3,4-dihydroxy-6-fluorphenyl)hexyloxy]-3-propylphenyl-1-äthanon.

**9.** Verbindungen der Formel

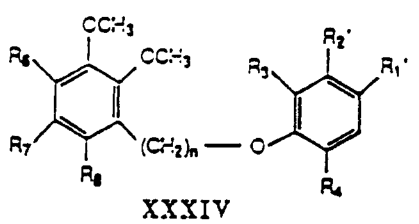

XXXIV

worin $R_1'$ -C(O)OR′, Acetyl, Wasserstoff oder Hydroxy; $R_2'$ -C(O)OR′, Wasserstoff oder Hydroxy; R′ Wasserstoff oder nieder-Alkyl; $R_3$ Wasserstoff oder nieder-Alkyl; $R_4$ Wasserstoff, nieder-Alkyl oder Halogen; $R_6$ Wasserstoff, nieder-Alkyl, Aryl oder Cycloalkyl; $R_7$ und $R_8$ Wasserstoff, nieder-Alkyl oder Halogen; und n eine ganze Zahl von 2-10 ist; vorausgesetzt, dass nicht mehr als ein Rest $R_1'$ oder $R_2'$ -C(O)OR′ oder Hydroxy ist.

**10.** 4-[6-(2,3-Dimethoxyphenyl)hexyloxy]-2-hydroxy-3-propylbenzoesäure und deren Methylester.

**11.** Verbindungen der Formel

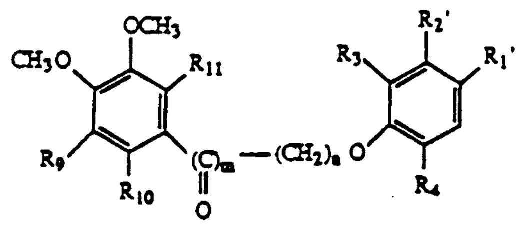

XXXVII

worin $R_1'$ -C(O)OR', Acetyl, Wasserstoff oder Hydroxy; $R_2'$ -C(O)OR', Wasserstoff oder Hydroxy; R' Wasserstoff oder nieder-Alkyl; $R_3$ Wasserstoff oder nieder-Alkyl; $R_4$ Wasserstoff, nieder-Alkyl oder Halogen; $R_9$ Wasserstoff oder nieder-Alkyl; $R_{10}$ Wasserstoff, nieder-Alkyl oder Halogen; $R_{11}$ Wasserstoff, nieder-Alkyl, Cycloalkyl oder Halogen; m 0 oder 1 und n eine ganze Zahl von 2-10 ist, vorausgesetzt, dass nicht mehr als ein Rest $R_1'$ oder $R_2'$ -C(O)OR' oder Hydroxy ist.

**12.** Die Verbindungen von Anspruch 1-8 zur Anwendung als Heilmittel.

**13.** Die Verbindungen von Anspruch 1-8 zur Verwendung als Wirkstoffe bei der Herstellung von Arzneimitteln zur Behandlung von entzündlichen Erkrankungen, kardiovaskulären Erkrankungen, Hauterkrankungen und bronchopulmonären Erkrankungen.

**14.** Pharmazeutische Präparate enthaltend eine Verbindung der Ansprüche 1-8 und einen pharmazeutischen Trägerstoff.

**15.** Verfahren zur Herstellung von Verbindungen der Ansprüche 1-8, dadurch gekennzeichnet, dass man eine Verbindung der Formel

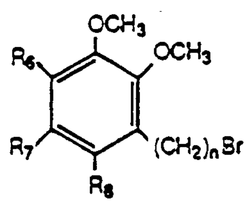

III

oder

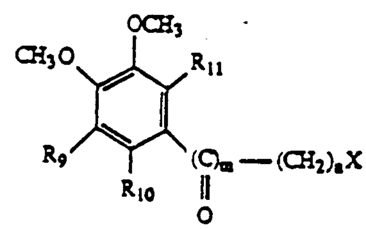

XXXVI

mit einer Verbindung der Formel

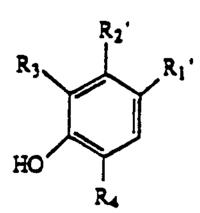

XXXIII

zu einer Verbindung der Formeln

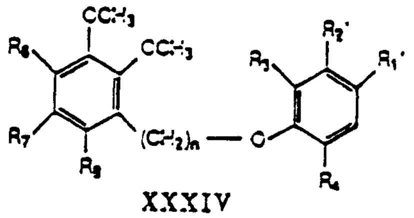

XXXIV

oder

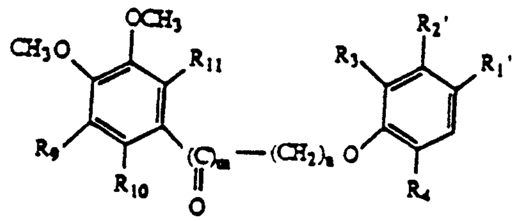

XXXVII

umsetzt oder
eine Verbindung der Formel

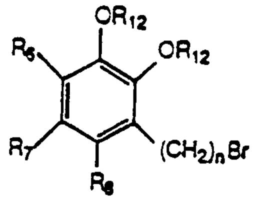

XXXIX

mit einer Verbindung der Formel

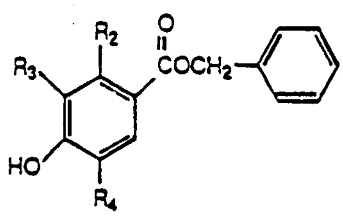

XXXX

zu einer Verbindung der Formel

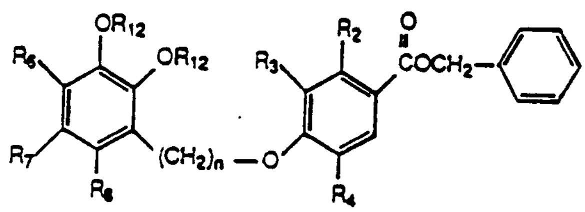

XXXXI

umsetzt,
wobei $R_1'$ Wasserstoff, nieder-Alkoxycarbonyl oder Acetyl; $R_2'$ Wasserstoff oder nieder-Alkoxycarbonyl; $R_{12}$ Benzyl oder Acyl; X Brom oder Methansulfonyloxy darstellen und die restlichen Symbole die oben angegebene Bedeutung haben, vorausgesetzt, dass nicht mehr als ein Rest $R_1'$ oder $R_2'$ nieder-Alkoxycarbonyl ist,
worauf man die Methoxygruppen in einer Verbindung der Formel XXXIV oder XXXVII oder den

Benzylester und eine gegebenenfalls anwesende Benzyläthergruppe $R_{12}$ in einer Verbindung der Formel XXXXI abspaltet, gewünschtenfalls eine Hydroxygruppe $R_2$ verestert und gewünschtenfalls eine Carboxygruppe $R_1$ oder $R_2$ verestert oder in ein Salz mit einer pharmazeutisch akzeptablen Base überführt oder eine die nieder-Alkyl-substituierte Aminogruppe in ein Salz mit einer pharmazeutisch akzeptablen Säure überführt.

**Patentansprüche für folgenden Vertragsstaat : ES**

**1.** Verfahren zur Herstellung von Verbindungen der Formel

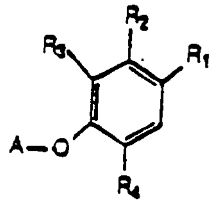

I

worin $R_1$ -C(O)OR, Wasserstoff, Acetyl, Hydroxy oder Alkanoyloxy; $R_2$ -C(O)OR, Hydroxy, Wasserstoff oder Alkanoyloxy; R Wasserstoff, nieder-Alkyl oder $-(CH_2)_nN$ (nieder-Alkyl)$_2$; $R_3$ Wasserstoff, nieder-Alkyl oder Amino: $R_4$ Wasserstoff, nieder-Alkyl, Halogen oder Amino darstellt, A eine Gruppe der Formel

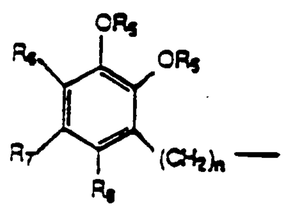

A'

darstellt, worin $R_5$ Wasserstoff oder Acyl; $R_6$ Wasserstoff, Halogen, nieder-Alkyl. Aryl oder Cycloalkyl: und $R_7$ und $R_8$ unabhängig voneinander Wasserstoff, nieder-Alkyl oder Halogen darstellen und n eine ganze Zahl von 2-10 ist, oder A eine Gruppe der Formel

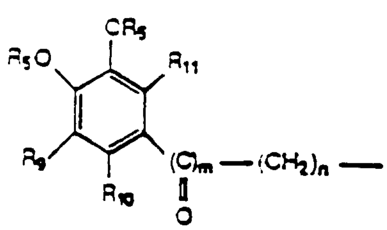

A"

darstellt, worin $R_5$ Wasserstoff oder Acyl; $R_9$ Wasserstoff oder nieder-Alkyl; $R_{10}$ Wasserstoff, nieder-Alkyl oder Halogen; $R_{11}$ Wasserstoff, nieder-Alkyl, Cycloalkyl oder Halogen darstellt, m 0 oder 1 und n eine ganze Zahl von 2-10 ist; vorausgesetzt, dass nicht mehr als ein Rest $R_1$ oder $R_2$ Hydroxy, Alkanoyloxy oder -C(O)OR ist,
und, falls R Wasserstoff ist, Salze davon mit pharmazeutisch akzeptablen Basen, und, falls R $(CH_2)_n$-N-(nieder-Alkyl)$_2$ ist, Säureadditionssalze davon mit pharmazeutisch akzeptablen Säuren, dadurch gekennzeichnet, dass man eine Verbindung der Formel

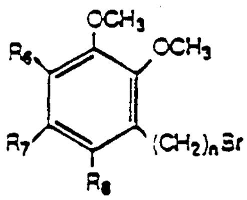

III

oder

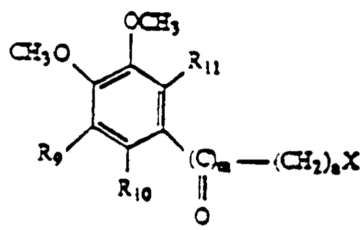

XXXVI

mit einer Verbindung der Formel

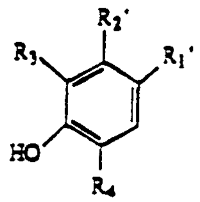

XXXIII

zu einer Verbindung der Formeln

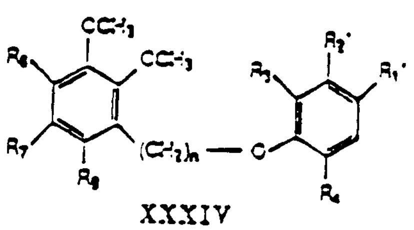

XXXIV

oder

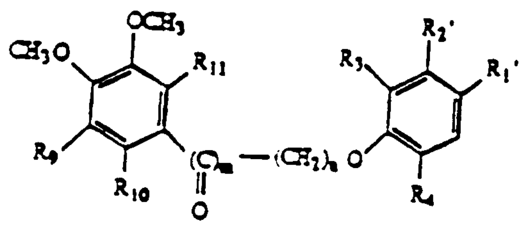

XXXVII

umsetzt oder
eine Verbindung der Formel

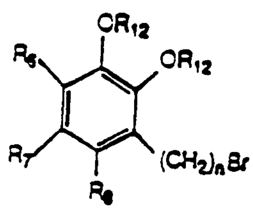

XXXIX

mit einer Verbindung der Formel

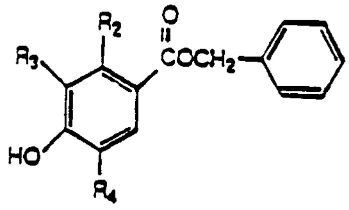

XXXX

zu einer Verbindung der Formel

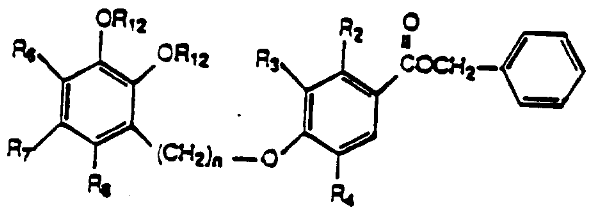

XXXXI

umsetzt,
wobei $R_1$' Wasserstoff, nieder-Alkoxycarbonyl oder Acetyl; $R_2$' Wasserstoff oder nieder-Alkoxycarbonyl; $R_{12}$ Benzyl oder Acyl; X Brom oder Methansulfonyloxy darstellen und die restlichen Symbole die oben angegebene Bedeutung haben, vorausgesetzt, dass nicht mehr als ein Rest $R_1$' oder $R_2$' nieder-Alkoxycarbonyl ist,
worauf man die Methoxygruppen in einer Verbindung der Formel XXXIV oder XXXVII oder den Benzylester und eine gegebenenfalls anwesende Benzyläthergruppe $R_{12}$ in einer Verbindung der Formel XXXXI abspaltet, gewünschtenfalls eine Hydroxygruppe $R_2$ verestert und gewünschtenfalls eine Carboxygruppe $R_1$ oder $R_2$ verestert oder in ein Salz mit einer pharmazeutisch akzeptablen Base überführt oder eine die nieder-Alkyl-substituierte Aminogruppe in ein Salz mit einer pharmazeutisch akzeptablen Säure überführt.

**2.** Verfahren nach Anspruch 1, dadurch gekennzeichnet, dass man Verbindungen der Formel

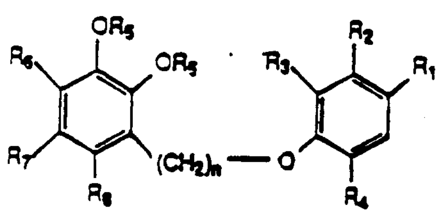

Ia

herstellt,
in denen $R_1$ Carboxy oder Acetyl, $R_2$ Hydroxy, $R_3$ Wasserstoff oder Propyl, $R_4$ Wasserstoff oder Chlor, n eine ganze Zahl von 2-10, $R_5$ Wasserstoff oder Acetyl, $R_6$ Wasserstoff, nieder-Alkyl oder Aryl und $R_7$ und $R_8$ Wasserstoff sind.

3. Verfahren nach Anspruch 1, dadurch gekennzeichnet, dass man Verbindungen der Formel

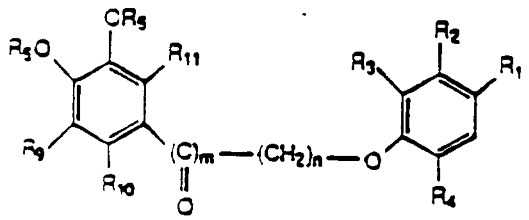

I b

herstellt,
in denen $R_1$ Carboxy oder Acetyl, $R_2$ Hydroxy, $R_3$ Wasserstoff oder Propyl, $R_4$ Wasserstoff oder Chlor, m 0 oder 1, n eine ganze Zahl von 2-10, $R_5$ Wasserstoff oder Acetyl, $R_9$ und $R_{10}$ Wasserstoff und $R_{11}$ Wasserstoff oder Chlor sind.

4. Verfahren nach Anspruch 1, worin
$R_6$ Wasserstoff, nieder-Alkyl, Aryl oder Cycloalkyl ist und die übrigen Symbole die in Anspruch 1 angegebene Bedeutung haben

5. Verfahren nach Anspruch 1, dadurch gekennzeichnet, dass man die 4-[6-(2,3-Dihydroxyphenyl)-hexyloxy]-2-hydroxybenzoesäure herstellt.

6. Verfahren nach Anspruch 1, dadurch gekennzeichnet, dass man eine Verbindung aus der Gruppe der folgenden herstellt:
4-[4-(2,3-Dihydroxyphenyl)butoxy]-2-hydroxy-3-propylbenzoesäure;
4-[6-(2,3-Dihydroxyphenyl)hexyloxy]-2-hydroxy-3-propylbenzoesäure;
4-[8-(2,3-Dihydroxyphenyl)octyloxy]-2-hydroxy-3-propylbenzoesäure;
4-[6-[2,3-Bis(acetyloxy)phenyl]hexyloxyl]2-hydroxy-3-propylbenzoesäure; und
4-[6-[2,3-Dihydroxy-4-(1-methylethyl)phenyl]hexyloxy]-2-hydroxy-3-propylbenzoesäure.

7. Verfahren nach Anspruch 1, dadurch gekennzeichnet, dass man eine Verbindung aus der Gruppe der folgenden herstellt:
4-[3-(3,4-Dihydroxyphenyl)propoxy]-2-hydroxy-3-propylbenzoesäure;
4-[6-(3,4-Dihydroxyphenyl)hexyloxy]-2-hydroxy-3-propylbenzoesäure;
4-[[6-(3,4-Dihydroxyphenyl)-6-oxohexyl]oxy]-2-hydroxy-3-propylbenzoesäure; und
1-[2-Hydroxy-4-[4-(2,3-dihydroxyphenyl)butoxy]-3-propylphenyl]äthanon.

8. Verfahren nach Anspruch 1, dadurch gekennzeichnet, dass man eine Verbindung aus der Gruppe der folgendedn herstellt:
4-(6-[2,3-Dihydroxy-4-(1,1-dimethyldäthyl)phenyl]hexyloxy]-2-hydroxy-3-propylbenzoesäure;
4-[6-[2,3-Dihydroxy-4-methylphenyl]hexyloxy]-2-hydroxy-3-propylbenzoesäure;
4-[4-[2,3-Dihydroxy-4-(2-methylpropyl)phenyl]butoxy]-2-hydroxy-3-propylbenzoesäure;
4-[6-[2,3-Dihydroxy-5,6-dimethylphenyl]hexyloxy]-2-hydroxy-3-propylbenzoesäure;
4-[5-[5-Chlor-2,3-dihydroxyphenyl]pentyloxy]-2-hydroxy-3-propylbenzoesäure;
4-[6-[2,3-Dihydroxy-6-fluorophenyl]hexyloxy]-2-hydroxy-3-propylbenzoesäure;
4-[6-[2,3-Dihydroxy-4-cyclohexylphenyl]butoxy]-2-hydroxy-3-propylbenzoesäure;
4-[4-[2,3-Dihydroxy-4-(1,1-dimethyläthyl)phenyl]butoxy]-2-hydroxy-3-propylbenzoesäure;
4-[8-[2,3-Dihydroxy-4-(1,1-dimethyläthyl)phenyl]octyloxy]-2-hydroxy-3-propylbenzoesäure;
4-[4-[2,3-Dihydroxy-4-(1,1-dimethyläthyl)phenyl]butoxy]-2-hydroxybenzoesäure;
4-[8-[2,3-Dihydroxy-4-(1-methyläthyl)phenyl]octyloxy]-2-hydroxybenzoesäure:
4-[4-[2,3-Dihydroxyphenyl]butoxy]-3,5-dipropyl-2-hydroxybenzoesäure;
4-[4-[2,3-Dihydroxy-4-(1-methyläthyl)phenyl]butoxy]-3,5-dilpropylbenzoesäure;
4-[6-[2,3-Dihydroxy-4-(1-methyläthyl)phenyl]hexyloxy]-3-propylbenzoesäure;
4-[6-[2,3-Bis(acetyloxy)-4-(1-methyläthyl)phenyl]hexyloxy]-2-hydroxy-3-propylbenzoesäure;
2-Acetyloxy-4-[6-(2,3-dihydroxy-4-(1-methyläthyl)phenyl]-hexyloxy]-3-propylbenzoesäure;
4-[6-[2,3-Bis[4-methylbenzoyl)oxy]-4-(1-methyläthyl)phenyl]hexyloxy]-2-hydroxy-3-propylbenzoesäure;
1-[2-Hydroxy-4-[6-[2,3-dihydroxy-4-(1,1-dimethyläthyl)phenyl]hexyloxy]-3-propylphenyl]äthanon;
1-[2-Hydroxy-4-[8-[2,3-dihydroxy-6-fluorphenyl]octyloxy]-3-propylphenyl]äthanon;
1-[2-Hydroxy-4-[6-[6-chlor-2,3-dihydroxyphenyl]hexyloxy]-3-propylphenyl]äthanon;
1-[2-Hydroxy-4-[6-[5,6-dichlor-2,3-dihydroxyphenyl]hexyloxy]-3-propylphenyl]äthanon;
1-[2-Hydroxy-4-[6-[2,3-dihydroxy-4,5,6-trichlorphenyl]hexyloxy]-3-propylphenyl]äthanon;
5-[6-(2,3-Dihydroxy-4-(1-methyläthyl)phenyl]hexyloxy]-2-hydroxybenzoesäure;
4-[6-[2,3-Dihydroxy-4-(1-methyläthyl)phenyl]hexyloxy]benzoesäure;
4-[6-[2,3-Dihydroxy-4-(1-methyläthyl)phenyl]hexyloxy]-2-hydroxy-3-propylbenzoesäure-äthylester;
4-[6-[2,3-Dihydroxy-4-(1-methläthyl)phenyl]hexyloxy]-2-hydroxy-3-propylbenzoesäure-äthylester;
4-[8-(3,4-Dihydroxyphenyl)octyloxy]-2-hydroxy-3-propylbenzoesäure;
4-[3-(3,4-Dihydroxyphenyl)propoxy]-2-hydroxy-3-propylbenzoesäure-äthylester;
4-[6-(3,4-Dihydroxyphenyl)hexyloxy]-2-hydroxy-benzoesäure;
4-[6-(3,4-Dihydroxyphenyl)hexyloxy]-3-propylbenzoesäure;
4-[8-(3,4-Dihydroxyphenyl)octyloxy]benzoesäure;
3-[8-(3,4-Dihydroxyphenyl)octyloxy]benzoesäure;
5-[8-(3,4-Dihydroxyphenyl)octyloxy]-2-hydroxybenzoesäure;
4-[[8-(3,4-Dihydroxyphenyl)-8-oxooctyl]oxy]-2-hydroxy-3-propylbenzoesäure;
4-[[4-(3,4-Dihydroxyphenyl)-4-oxobutyl]oxy]-2-hydroxy-3-propylbenzoesäure;
4-[6-(3,4-Dihydroxy-5-fluorphenyl)hexyloxy]-2-hydroxy-3-propylbenzoesäure;
4-[6-(3,4-Dihydroxy-6-fluorphenyl)hexyloxy]-2-hydroxy-3-propylbenzoesäure;
4-[6-(3,4-Dihydroxy-6-chlorphenyl)hexyloxy]-2-hydroxy-3-propylbenzoesäure;
4-[4-[3,4-Dihydroxy-5-(1-methyläthyl)phenyl]butoxy]-2-hydroxy-3-propylbenzoesäure;
4-[4-[3,4-Dihydroxy-5-(1,1-dimethyläthyl)phenyl]butoxy]-2-hydroxybenzoesäure;
4-[6-[3,4-Bis(acetyloxy)phenyl]hexyloxy]-2-hydroxy-3-propylbenzoesäure;
2-Acetyloxy-4-[6-(3,4-dihydroxyphenyl)hexyloxyl-3-propyl-benzoesäure;
4-[[4-[3,4-Dihydroxy-5-(1-methyläthyl)phenyl]-4-oxobutyl]oxy]-2-hydroxybenzoesäure;
4-[6-[3,4-Dihydroxy-2-(1-methyläthyl)phenyl]hexyloxy]-2-hydroxy-3-propylbenzoesäure;
4-[[4-(3,4-Dihydroxy-2,5-dimethylphenyl)-4-oxobutyl]oxy]-2-hydroxy-3-propylbenzoesäure;
4-[4-(3,4-Dihydroxy-2,5-dimethylphenyl)butoxy]-2-hydroxy-3-propylbenzoesäure;
4-[[6-(3,4-Dihydroxy-2,5-6-trimethylphenyl)-6-oxohexyl]oxy]-2-hydroxy-3-propylbenzoesäure;
4-[6-(3,4-Dihydroxy-5,6-trimethylphenyl)hexyloxy]-2-hydroxy-3-propylbenzoesäure;
1-[2-Hydroxy-4-[6-(3,4-dihydroxyphenyl)hexyloxy]-3-propylphenyl-1-äthanon;
1-[2-Hydroxy-4-[8-(3,4-dihydroxyphenyl)octyloxy]-3-propylphenyl-1-äthanon;
1-[2-Hydroxy-4-[6-(3,4-dihydroxy-2,5-dimethylphenyl)hexyloxy]-3-propylphenyl-1-äthanon;
1-[2-Hydroxy-4-[6-(3,4-dihydroxy-6-chlorphenyl)hexyloxy]-3-propylphenyl-1-äthanon;
1-[2-Hydroxy-4-[6-(3,4-dihydroxy-2-chlorphenyl)hexyloxy]-3-propylphenyl-1-äthanon;
1-[2-Hydroxy-4-[6-(3,4-dihydroxy-6-fluorphenyl)hexyloxy]-3-propylphenyl-1-äthanon.

**9.** Verwendung der Verbindungen der Formel I und der Salze davon nach Anspruch 1 bei der Herstellung von Arzneimitteln zur Behandlung von entzündlichen Erkrankungen, kardiovaskulären Erkrankungen, Hauterkrankungen und bronchopulmonären Erkrankungen.

**Patentansprüche für folgenden Vertragsstaat : GR**

**1.** Verfahren zur Herstellung von Verbindungen der Formel

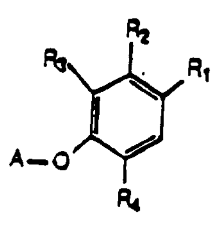

I

worin $R_1$ -C(O)OR, Wasserstoff, Acetyl, Hydroxy oder Alkanoyloxy; $R_2$ -C(O)OR, Hydroxy, Wasserstoff oder Alkanoyloxy; R Wasserstoff, nieder-Alkyl oder $-(CH_2)_nN$ (nieder-Alkyl)$_2$; $R_3$ Wasserstoff, nieder-

Alkyl oder Amino; $R_4$ Wasserstoff, nieder-Alkyl, Halogen oder Amino darstellt, A eine Gruppe der Formel

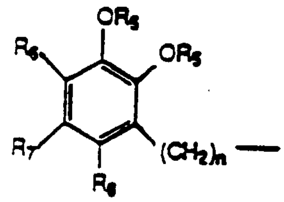

A'

darstellt, worin $R_5$ Wasserstoff oder Acyl; $R_6$ Wasserstoff, Halogen, nieder-Alkyl, Aryl oder Cycloalkyl; und $R_7$ und $R_8$ unabhängig voneinander Wasserstoff, nieder-Alkyl oder Halogen darstellen und n eine ganze Zahl von 2-10 ist, oder A eine Gruppe der Formel

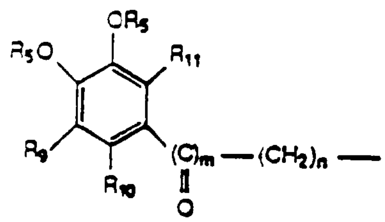

A"

darstellt, worin $R_5$ Wasserstoff oder Acyl; $R_9$ Wasserstoff oder nieder-Alkyl; $R_{10}$ Wasserstoff, nieder-Alkyl oder Halogen; $R_{11}$ Wasserstoff, nieder-Alkyl, Cycloalkyl oder Halogen darstellt, m 0 oder 1 und n eine ganze Zahl von 2-10 ist; vorausgesetzt, dass nicht mehr als ein Rest $R_1$ oder $R_2$ Hydroxy, Alkanoyloxy oder -C(O)OR ist,

und, falls R Wasserstoff ist, Salze davon mit pharmazeutisch akzeptablen Basen, und, falls R $(CH_2)_n$-N-(nieder-Alkyl)$_2$ ist, Säureadditionssalze davon mit pharmazeutisch akzeptablen Säuren, dadurch gekennzeichnet, dass man eine Verbindung der Formel

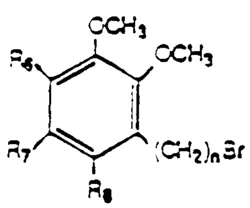

III

oder

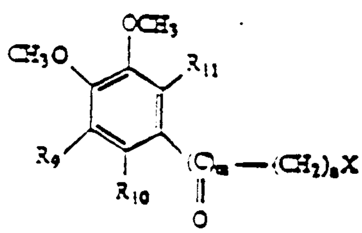

XXXVI

mit einer Verbindung der Formel

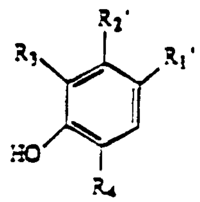

XXXIII

zu einer Verbindung der Formeln

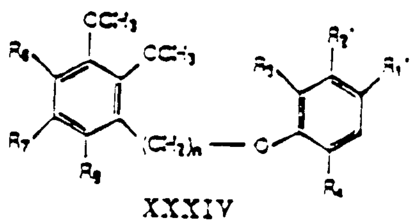

XXXIV

oder

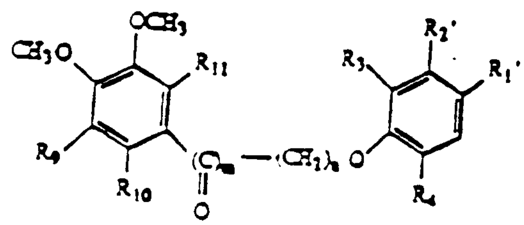

XXXVII

umsetzt oder
eine Verbindung der Formel

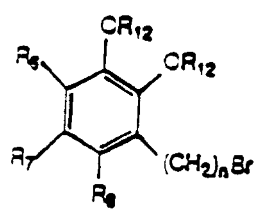

XXXIX

mit einer Verbindung der Formel

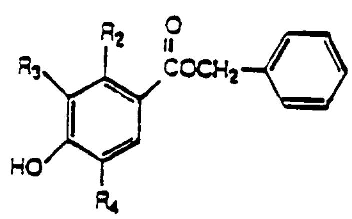

XXXX

zu einer Verbindung der Formel

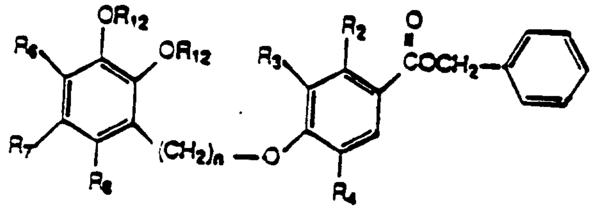

XXXXI

umsetzt,
wobei $R_1$' Wasserstoff, nieder-Alkoxycarbonyl oder Acetyl; $R_2$' Wasserstoff oder nieder-Alkoxycarbonyl; $R_{12}$ Benzyl oder Acyl; X Brom oder Methansulfonyloxy darstellen und die restlichen Symbole die oben angegebene Bedeutung haben, vorausgesetzt, dass nicht mehr als ein Rest $R_1$' oder $R_2$' nieder-Alkoxycarbonyl ist,
worauf man die Methoxygruppen in einer Verbindung der Formel XXXIV oder XXXVII oder den Benzylester und eine gegebenenfalls anwesende Benzyläthergruppe $R_{12}$ in einer Verbindung der Formel XXXXI abspaltet, gewünschtenfalls eine Hydroxygruppe $R_2$ verestert und gewünschtenfalls eine Carboxygruppe $R_1$ oder $R_2$ verestert oder in ein Salz mit einer pharmazeutisch akzeptablen Base überführt oder eine die nieder-Alkyl-substituierte Aminogruppe in ein Salz mit einer pharmazeutisch akzeptablen Säure überführt.

**2.** Verfahren nach Anspruch 1, dadurch gekennzeichnet, dass man Verbindungen der Formel

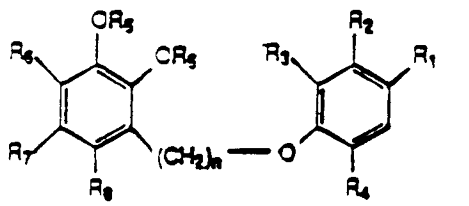

Ia

herstellt,
in denen $R_1$ Carboxy oder Acetyl, $R_2$ Hydroxy, $R_3$ Wasserstoff oder Propyl, $R_4$ Wasserstoff oder Chlor, n eine ganze Zahl von 2-10, $R_5$ Wasserstoff oder Acetyl, $R_6$ Wasserstoff, nieder-Alkyl oder Aryl und $R_7$ und $R_8$ Wasserstoff sind.

**3.** Verfahren nach Anspruch 1, dadurch gekennzeichnet, dass man Verbindungen der Formel

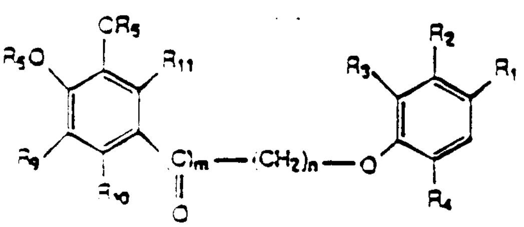

Ib

herstellt,
in denen $R_1$ Carboxy oder Acetyl, $R_2$ Hydroxy, $R_3$ Wasserstoff oder Propyl, $R_4$ Wasserstoff oder Chlor, m 0 oder 1, n eine ganze Zahl von 2-10, $R_5$ Wasserstoff oder Acetyl, $R_9$ und $R_{10}$ Wasserstoff und $R_{11}$ Wasserstoff oder Chlor sind.

**4.** Verfahren nach Anspruch 1, worin
$R_6$ Wasserstoff, nieder-Alkyl, Aryl oder Cycloalkyl ist und die übrigen Symbole die in Anspruch 1 angegebene Bedeutung haben

5. Verfahren nach Anspruch 1, dadurch gekennzeichnet, dass man die 4-[6-(2,3-Dihydroxyphenyl)-hexyloxy]-2-hydroxybenzoesäure herstellt.

6. Verfahren nach Anspruch 1, dadurch gekennzeichnet, dass man eine Verbindung aus der Gruppe der folgenden herstellt:
4-[4-(2,3-Dihydroxyphenyl)butoxy]-2-hydroxy-3-propylbenzoesäure;
4-[6-(2,3-Dihydroxyphenyl)hexyloxy]-2-hydroxy-3-propylbenzoesäure;
4-[8-(2,3-Dihydroxyphenyl)octyloxy]-2-hydroxy-3-propylbenzoesäure;
4-[6-[2,3-Bis(acetyloxy)phenyl]hexyloxy]2-hydroxy-3-propylbenzoesäure; und
4-[6-[2,3-Dihydroxy-4-(1-methylethyl)phenyl]hexyloxy]-2-hydroxy-3-propylbenzoesäure.

7. Verfahren nach Anspruch 1, dadurch gekennzeichnet, dass man eine Verbindung aus der Gruppe der folgenden herstellt:
4-[3-(3,4-Dihydroxyphenyl)propoxy]-2-hydroxy-3-propylbenzoesäure;
4-[6-(3,4-Dihydroxyphenyl)hexyloxy]-2-hydroxy-3-propylbenzoesäure;
4-[[6-(3,4-Dihydroxyphenyl)-6-oxohexyl]oxy]-2-hydroxy-3-propylbenzoesäure; und
1-[2-Hydroxy-4-[4-(2,3-dihydroxyphenyl)butoxy]-3-propylphenyl]äthanon.

8. Verfahren nach Anspruch 1, dadurch gekennzeichnet, dass man eine Verbindung aus der Gruppe der folgenden herstellt:
4-[6-[2,3-Dihydroxy-4-(1,1-dimethyläthyl)phenyl]hexyloxy]-2-hydroxy-3-propylbenzoesäure;
4-[6-[2,3-Dihydroxy-4-methylphenyl]hexyloxy]-2-hydroxy-3-propylbenzoesäure;
4-[4-[2,3-Dihydroxy-4-(2-methylpropyl)phenyl]butoxy]-2-hydroxy-3-propylbenzoesäure;
4-[6-(2,3-Dihydroxy-5,6-dimethylphenyl]hexyloxy]-2-hydroxy-3-propylbenzoesäure;
4-[5-[5-Chlor-2,3-dihydroxyphenyl]pentyloxy]-2-hydroxy-3-propylbenzoesäure;
4-[6-[2,3-Dihydroxy-6-fluorophenyl]hexyloxy]2-hydroxy-3-propylbenzoesäure;
4-[4-[2,3-Dihydroxy-4-cyclohexylphenyl]butoxy]-2-hydroxy-3-propylbenzoesäure;
4-[4-[2,3-DihydroxY-4-(1,1-dimethyläthyl)phenyl]butoxy]-2-hydroxy-3-propylbenzoesäure;
4-[8-[2,3-Dihydroxy-4-(1,1-dimethyläthyl)phenyl]octyloxy]-2-hydroxy-3-propylbenzoesäure;
4-[4-[2,3-Dihydroxy-4-(1,1-dimethyläthyl)phenyl]butoxy]-2-hydroxybenzoesäure;
4-[8-[2,3-Dihydroxy-4-(1-methyläthyl)phenyl]octyloxy]-2-hydroxybenzoesäure;
4-[4-[2,3-Dihydroxyphenyl]butoxy]-3,5-dipropyl-2-hydroxybenzoesäure;
4-[4-[2,3-Dihydroxy-4-(1-methyläthyl)phenyl]butoxy]-3,5-dipropylbenzoesäure;
4-[6-[2,3-Dihydroxy-4-(1-methyläthyl)phenyl]hexyloxy]-3-propylbenzoesäure;
4-[6-[2,3-Bis(acetyloxy)-4-(1-methyläthyl)phenyl]hexyloxy]-2-hydroxy-3-propylbenzoesäure;
2-Acetyloxy-4-[6-(2,3-dihydroxy-4-(1-methyläthyl)phenyl]hexyloxy]-3-propylbenzoesäure;
4-[6-[2,3-Bis[4-methylbenzoyl)oxy]-4-(1-methyläthyl)phenyl]hexyloxy]-2-hydroxy-3-propylbenzoesäure;
1-[2-Hydroxy-4-[6-[2,3-dihydroxy-4-(1,1-dimethyläthyl)phenyl]hexyloxy]-3-propylphenyl]äthanon;
1-[2-Hydroxy-4-[8-[2,3-dihydroxy-6-fluorphenyl]octyloxy]-3-propylphenyl]äthanon;
1-[2-Hydroxy-4-[6-[6-chlor-2,3-dihydroxyphenyl]hexyloxy]-3-propylphenyl]äthanon;
1-[2-Hydroxy-4-[6-[5,6-dichlor-2,3-dihydroxyphenyl]hexyloxy]-3-propylphenyl]äthanon;
1-[2-Hydroxy-4-[6-[2,3-dihydroxy-4,5,6-trichlorphenyl]hexyloxy]-3-propylphenyl]äthanon;
5-[6-(2,3-Dihydroxy-4-(1-methyläthyl)phenyl]hexyloxy]-2-hydroxybenzoesäure;
4-[6-[2,3-Dihydroxy-4-(1-methyläthyl)phenyl]hexyloxy]benzoesäure;
4-[6-[2,3-Dihydroxy-4-(1-methyläthyl)phenyl]hexyloxy]-2-hydroxy-3-propylbenzoesäure-äthylester;
4-[6-[2,3-Dihydroxy-4-(1-methyläthyl)phenyl]hexyloxy]-2-hydroxy-3-propylbenzoesäure-äthylester;
4-[8-(3,4-Dihydroxyphenyl)octyloxy]-2-hydroxy-3-propylbenzoesäure;
4-[3-(3,4-Dihydroxyphenyl)propoxy]-2-hydroxy-3-propylbenzoesäure-äthylester;
4-[6-(3,4-Dihydroxyphenyl)hexyloxy]-2-hydroxy-benzoesäure;
4-[6-(3,4-Dihydroxyphenyl)hexyloxy]-3-propylbenzoesäure;
4-[8-(3,4-Dihydroxyphenyl)octyloxy]benzoesäure;
3-[8-(3,4-Dihydroxyphenyl)octyloxy]benzoesäure;
5-[8-(3,4-Dihydroxyphenyl)octyloxy]-2-hydroxybenzoesäure;
4-[[8-(3,4-Dihydroxyphenyl)-8-oxooctyl]oxy]-2-hydroxy-3-propylbenzoesäure;
4-[[4-(3,4-Dihydroxyphenyl)-4-oxobutyl]oxy]-2-hydroxy-3-propylbenzoesäure;
4-[6-(3,4-Dihydroxy-5-fluorphenyl)hexyloxy]-2-hydroxy-3-propylbenzoesäure;
4-[6-(3,4-Dihydroxy-6-fluorphenyl)hexyloxy]-2-hydroxy-3-propylbenzoesäure;

4-[6-(3,4-Dihydroxy-6-chlorphenyl)hexyloxy]-2-hydroxy-3-propylbenzoesäure;
4-[4-[3,4-Dihydroxy-5-(1-methyläthyl)phenyl]butoxy]-2-hydroxy-3-propylbenzoesäure;
4-[4-[3,4-Dihydroxy-5-(1,1-dimethyläthyl)phenyl]butoxy]-2-hydroxybenzoesäure;
4-[6-[3,4-Bis(acetyloxy)phenyl]hexyloxy]-2-hydroxy-3-propylbenzoesäure;
2-Acetyloxy-4-[6-(3,4-dihydroxyphenyl)hexyloxy]-3-propylbenzoesäure;
4-[[4-[3,4-Dihydroxy-5-(1-methyläthyl)phenyl]-4-oxobutyl]oxy]-2-hydroxybenzoesäure;
4-[6-[3,4-Dihydroxy-2-(1-methyläthyl)phenyl]hexyloxy]-2-hydroxy-3-propylbenzoesäure;
4-[[4-(3,4-Dihydroxy-2,5-dimethylphenyl)-4-oxobutyl]oxy]-2-hydroxy-3-propylbenzoesäure;
4-[4-(3,4-Dihydroxy-2,5-dimethylphenyl)butoxy]-2-hydroxy-3-propylbenzoesäure;
4-[[6-(3,4-Dihydroxy-2,5-6-trimethylphenyl)-6-oxohexyl]oxy]-2-hydroxy-3-propylbenzoesäure;
4-[6-(3,4-Dihydroxy-5,6-trimethylphenyl)hexyloxy]-2-hydroxy-3-propylbenzoesäure;
1-[2-Hydroxy-4-[6-(3,4-dihydroxyphenyl)hexyloxy]-3-propylphenyl-1-äthanon;
1-[2-Hydroxy-4-[8-(3,4-dihydroxyphenyl)octyloxy]-3-propylphenyl-1-äthanon;
1-[2-Hydroxy-4-[6-(3,4-dihydroxy-2,5-dimethylphenyl)hexyloxy]-3-propylphenyl-1-äthanon;
1-[2-Hydroxy-4-[6-(3,4-dihydroxy-6-chlorphenyl)hexyloxy]-3-propylphenyl-1-äthanon;
1-[2-Hydroxy-4-[6-(3,4-dihydroxy-2-chlorphenyl)hexyloxy]-3-propylphenyl-1-äthanon;
1-[2-Hydroxy-4-[6-(3,4-dihydroxy-6-fluorphenyl)hexyloxy]-3-propylphenyl-1-äthanon.

**9.** Verwendung der Verbindungen der Formel I und der Salze davon nach Anspruch 1 bei der Herstellung von Arzneimitteln zur Behandlung von entzündlichen Erkrankungen, kardiovaskulären Erkrankungen, Hauterkrankungen und bronchopulmonären Erkrankungen.

**10.** Verbindungen der Formel

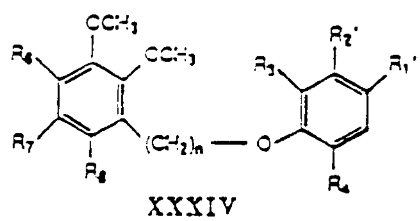

XXXIV

worin $R_1$' -C(O)OR', Acetyl, Wasserstoff oder Hydroxy; $R_2$' -C(O)OR', Wasserstoff oder Hydroxy; R' Wasserstoff oder nieder-Alkyl; $R_3$ Wasserstoff oder nieder-Alkyl; $R_4$ Wasserstoff, nieder-Alkyl oder Halogen; $R_6$ Wasserstoff, nieder-Alkyl, Aryl oder Cycloalkyl; $R_7$ und $R_8$ Wasserstoff, nieder-Alkyl oder Halogen; und n eine ganze Zahl von 2-10 ist; vorausgesetzt, dass nicht mehr als ein Rest $R_1$' oder $R_2$' -C(O)OR' oder Hydroxy ist.

**11.** 4-[6-(2,3-Dimethoxyphenyl)hexyloxy]-2-hydroxy-3-propylbenzoesäure und deren Methylester.

**12.** Verbindungen der Formel

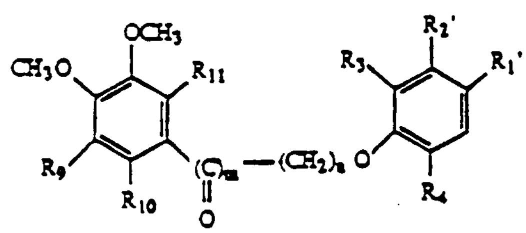

XXXVII

worin $R_1$' -C(O)OR', Acetyl, Wasserstoff oder Hydroxy; $R_2$' -C(O)OR', Wasserstoff oder Hydroxy; R' Wasserstoff oder nieder-Alkyl; $R_3$ Wasserstoff oder nieder-Alkyl; $R_4$ Wasserstoff, nieder-Alkyl oder Halogen; $R_9$ Wasserstoff oder nieder-Alkyl; $R_{10}$ Wasserstoff, nieder-Alkyl oder Halogen; $R_{11}$ Wasser-

stoff, nieder-Alkyl, Cycloalkyl oder Halogen; m 0 oder 1 und n eine ganze Zahl von 2-10 ist, vorausgesetzt, dass nicht mehr als ein Rest $R_1$' oder $R_2$' -C(O)OR' oder Hydroxy ist.

## Claims

### Claims for the following Contracting States : AT, BE, CH, DE, FR, GB, IT, LI, LU, NL, SE

**1.** Compounds of the formula

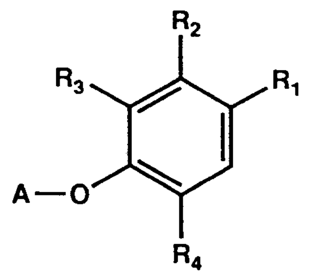

I

wherein $R_1$ represents -C(O)OR, hydrogen, acetyl, hydroxy or alkanoyloxy; $R_2$ represents -C(O)OR, hydroxy, hydrogen or alkanoyloxy; R represents hydrogen, lower-alkyl or $-(CH_2)_n$-N-(lower-alkyl)$_2$; $R_3$ represents hydrogen, lower-alkyl or amino; $R_4$ represents hydrogen, lower-alkyl, halogen or amino, A represents a group of the formula

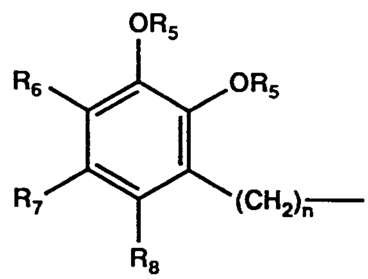

A'

wherein $R_5$ represents hydrogen or acyl; $R_6$ represents hydrogen, halogen, lower-alkyl, aryl or cycloalkyl; and $R_7$ and $R_8$ each independently represent hydrogen, lower-alkyl or halogen and n is an integer of 2-10, or A represents a group of the formula

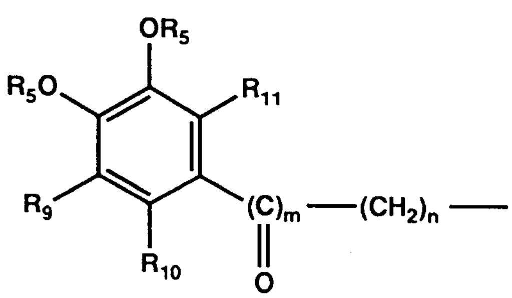

A"

wherein $R_5$ represents hydrogen or acyl; $R_9$ represents hydrogen or lower-alkyl; $R_{10}$ represents hydrogen, lower-alkyl or halogen, $R_{11}$ represents hydrogen, lower-alkyl, cycloalkyl or halogen, m is 0 or 1 and n is an integer of 2-10; provided that not more than one residue $R_1$ or $R_2$ is hydroxy, alkanoyloxy or -C(O)OR,
and, when R is hydrogen, salts thereof with pharmaceutically acceptable bases and, when R is $-(CH_2)_n$-N-(lower-alkyl)$_2$, acid addition salts thereof with pharmaceutically acceptable acids.

**2.** Compounds of the formula

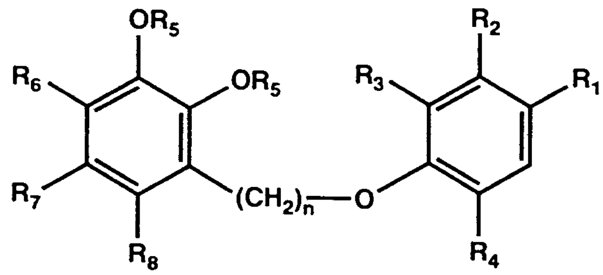

Ia

wherein $R_1$ is carboxy or acetyl, $R_2$ is hydroxy, $R_3$ is hydrogen or propyl, $R_4$ is hydrogen or chlorine, n is an integer of 2-10, $R_5$ is hydrogen or acetyl, $R_6$ is hydrogen, lower-alkyl or aryl and $R_7$ and $R_8$ are hydrogen.

**3.** Compounds of the formula

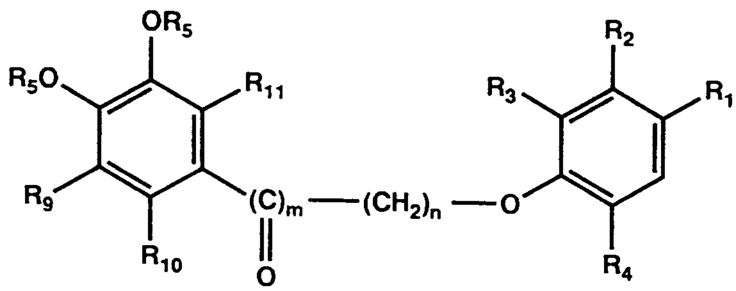

Ib

wherein $R_1$ is carboxy or acetyl, $R_2$ is hydroxy, $R_3$ is hydrogen or propyl, $R_4$ is hydrogen or chlorine, m is 0 or 1, n is an integer of 2-10, $R_5$ is hydrogen or acetyl, $R_9$ and $R_{10}$ are hydrogen and $R_{11}$ is hydrogen or chlorine.

**4.** Compounds of formula I of claim 1, wherein $R_6$ is hydrogen, lower-alkyl, aryl or cycloalkyl and the remaining symbols have the significance given in claim 1; and, when R is hydrogen, salts thereof with pharmaceutically acceptable bases and, when R is $-(CH_2)_n-N-(lower-alkyl)_2$, acid addition salts thereof with pharmaceutically acceptable acids.

**5.** 4-[6-(2,3-Dihydroxyphenyl)hexyloxy]-2-hydroxybenzoic acid.

**6.** 4-[4-(2,3-Dihydroxyphenyl)butoxy]-2-hydroxy-3-propylbenzoic acid;
4-[6-(2,3-dihydroxyphenyl)hexyloxy]-2-hydroxy-3-propylbenzoic acid;
4-[8-(2,3-dihydroxyphenyl)octyloxy]-2-hydroxy-3-propylbenzoic acid;
4-[6-[2,3-bis(acetyloxy)phenyl]hexyloxy]2-hydroxy-3-propylbenzoic acid; and
4-[6-[2,3-dihydroxy-4-(1-methylethyl)phenyl]hexyloxy]-2-hydroxy-3-propylbenzoic acid.

**7.** 4-[3-(3,4-Dihydroxyphenyl)propoxy]-2-hydroxy-3-propylbenzoic acid;
4-[6-(3,4-dihydroxyphenyl)hexyloxy]-2-hydroxy-3-propylbenzoic acid;
4-[[6-(3,4-dihydroxyphenyl)-6-oxohexyl]oxy]-2-hydroxy-3-propylbenzoic acid; and
1-[2-hydroxy-4-[4-(2,3-dihydroxyphenyl)butoxy]-3-propylphenyl]ethanone.

**8.** 4-[6-[2,3-Dihydroxy-4-(1,1-dimethylethyl)phenyl]hexyloxy]-2-hydroxy-3-propylbenzoic acid;
4-[6-[2,3-dihydroxy-4-methylphenyl]hexyloxy]-2-hydroxy-3-propylbenzoic acid;
4-[4-[2,3-dihydroxy-4-(2-methylpropyl)phenyl]butoxy]-2-hydroxy-3-propylbenzoic acid;
4-[6-[2,3-dihydroxy-5,6-dimethylphenyl]hexyloxy]-2-hydroxy-3-propylbenzoic acid;
4-[5-[5-chloro-2,3-dihydroxyphenyl]pentyloxy]-2-hydroxy-3-propylbenzoic acid;
4-[6-[2,3-dihydroxy-6-fluorophenyl]hexyloxy]-2-hydroxy-3-propylbenzoic acid;

4-[4-[2,3-dihydroxy-4-cyclohexylphenyl]butoxy]-2-hydroxy-3-propylbenzoic acid;
4-[4-[2,3-dihydroxy-4-(1,1-dimethylethyl)phenyl]butoxy]-2-hydroxy-3-propylbenzoic acid;
4-[8-[2,3-dihydroxy-4-(1,1-dimethylethyl)phenyl]octyloxy]-2-hydroxy-3-propylbenzoic acid;
4-[4-[2,3-dihydroxy-4-(1,1-dimethylethyl)phenyl]butoxy]-2-hydroxybenzoic acid;
4-[8-[2,3-dihydroxy-4-(1-methylethyl)phenyl]octyloxy]-2-hydroxybenzoic acid;
4-[4-[2,3-dihydroxyphenyl]butoxy]-3,5-dipropyl-2-hydroxybenzoicacid;
4-[4-[2,3-dhydroxy-4-(1-methylethyl)phenyl]butoxy]-3,5-dipropylbenzoic acid;
4-[6-[2,3-dihydroxy-4-(1-methylethyl)phenyl]hexyloxy]-3-propylbenzoic acid;
4-[6-[2,3-bis(acetyloxy)-4-(1-methylethyl)phenyl]hexyloxy]-2-hydroxy-3-propylbenzoic acid;
2-acetyloxy-4-[6-(2,3-dihydroxy-4-(1-methylethyl)phenyl]hexyloxy]-3-propylbenzoic acid;
4-[6-[2,3-bis[4-methylbenzoyl)oxy]-4-(1-methylethyl)phenyl]hexyloxy]-2-hydroxy-3-propylbenzoic acid;
1-[2-hydroxy-4-[6-[2,3-dihydroxy-4-(1,1-dimethylethyl)phenyl]hexyloxy]-3-propylphenyl]ethanone;
1-[2-hydroxy-4-[8-[2,3-dihydroxy-6-fluorophenyl]octyloxy]-3-propylphenyl]ethanone;
1-[2-hydroxy-4-[6-[6-chloro-2,3-dihydroxyphenyl]hexyloxy]-3-propylphenyl]ethanone;
1-[2-hydroxy-4-[6-[5,6-dichloro-2,3-dihydroxyphenyl]hexyloxy]-3-propylphenyl]ethanone;
1-[2-hydroxy-4-[6-[2,3-dihydroxy-4,5,6-trichlorophenyl]hexyloxy]-3-propylphenyl]ethanone;
5-[6-(2,3-dihydroxy-4-(1-methylethyl)phenyl]hexyloxy]-2-hydroxybenzoic acid;
4-[6-[2,3-dihydroxy-4-(1-methylethyl)phenyl]hexyloxy]benzoic acid;
4-[6-[2,3-dihydroxy-4-(1-methylethyl)phenyl]hexyloxy]-2-hydroxy-3-propylbenzoic acid ethyl ester;
4-[6-[2,3-dihydroxy-4-(1-methylethyl)phenyl]hexyloxy]-2-hydroxy-3-propylbenzoic acid ethyl ester;
4-[8-(3,4-dihydroxyphenyl)octyloxy]-2-hydroxy-3-propylbenzoic acid;
4-[3-(3,4-dihydroxyphenyl)propoxy]-2-hydroxy-3-propylbenzoic acid ethyl ester;
4-[6-(3,4-dihydroxyphenyl)hexyloxy]-2-hydroxy-benzoic acid;
4-[6-(3,4-dihydroxyphenyl)hexyloxy]-3-propylbenzoic acid;
4-[8-(3,4-dihydroxyphenyl)octyloxy]benzoic acid;
3-[8-(3,4-dihydroxyphenyl)octyloxy]benzoic acid;
5-[8-(3,4-dihydroxyphenyl)octyloxy]-2-hydroxybenzoic acid;
4-[[8-(3,4-dihydroxyphenyl)-8-oxooctyl]oxy]-2-hydroxy-3-propylbenzoic acid;
4-[[4-(3,4-dihydroxyphenyl)-4-oxobutyl]oxy]-2-hydroxy-3-propylbenzoic acid;
4-[6-(3,4-dihydroxy-5-fluorophenyl)hexyloxy]-2-hydroxy-3-propylbenzoic acid;
4-[6-(3,4-dihydroxy-6-fluorophenyl)hexyloxy]-2-hydroxy-3-propylbenzoic acid;
4-[6-(3,4-dihydroxy-6-chlorophenyl)hexyloxy]-2-hydroxy-3-propylbenzoic acid;
4-[4-[3,4-dihydroxy-5-(1-methylethyl)phenyl]butoxy]-2-hydroxy-3-propylbenzoic acid;
4-[4-[3,4-dihydroxy-5-(1,1-dimethylethyl)phenyl]butoxy]-2-hydroxybenzoic acid;
4-[6-[3,4-bis(acetyloxy)phenyl]hexyloxy]-2-hydroxy-3-propylbenzoic acid;
2-acetyloxy-4-[6-(3,4-dihydroxyphenyl)hexyloxy]-3-propylbenzoicacid;
4-[[4-[3,4-dihydroxy-5-(1-methylethyl)phenyl]-4-oxobutyl]oxy]-2-hydroxybenzoic acid;
4-[6-[3,4-dihydroxy-2-(1-methylethyl)phenyl]hexyloxy]-2-hydroxy-3-propylbenzoic acid;
4-[[4-(3,4-dihydroxy-2,5-dimethylphenyl)-4-oxobutyl]oxy]-2-hydroxy-3-propylbenzoic acid;
4-[4-(3,4-dihydroxy-2,5-dimethylphenyl)butoxy]-2-hydroxy-3-propylbenzoic acid;
4-[[6-(3,4-dihydroxy-2,5-6-trimethylphenyl)-6-oxohexyl]oxy]-2-hydroxy-3-propylbenzoic acid;
4-[6-(3,4-dihydroxy-5,6-trimethylphenyl)hexyloxy]-2-hydroxy-3-propylbenzoic acid;
1-[2-hydroxy-4-[6-(3,4-dihydroxyphenyl)hexyloxy]-3-propylphenyl-1-ethanone;
1-[2-hydroxy-4-[8-(3,4-dihydroxyphenyl)octyloxy]-3-propylphenyl-1-ethanone;
1-[2-hydroxy-4-[6-(3,4-dihydroxy-2,5-dimethylphenyl)hexyloxy]-3-propylphenyl-1-ethanone;
1-[2-hydroxy-4-[6-(3,4-dihydroxy-6-chlorophenyl)hexyloxy]-3-propylphenyl-1-ethanone;
1-[2-hydroxy-4-[6-(3,4-dihydroxy-2-chlorophenyl)hexyloxy]-3-propylphenyl-1-ethanone;
1-[2-hydroxy-4-[6-(3,4-dihydroxy-6-fluorophenyl)hexyloxy]-3-propylphenyl-1-ethanone.

**9.** Compounds of the formula

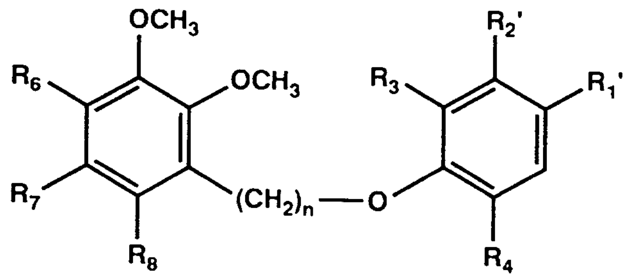

XXXIV

wherein $R_1$' is -C(O)OR', acetyl, hydrogen or hydroxy; $R_2$' is -C(O)OR', hydrogen or hydroxy; R' is hydrogen or lower-alkyl; $R_3$ is hydrogen or lower-alkyl; $R_4$ is hydrogen, lower-alkyl or halogen; $R_6$ is hydrogen, lower-alkyl, aryl or cycloalkyl; $R_7$ and $R_8$ are hydrogen, lower-alkyl or halogen; and n is an integer of 2-10; provided that not more than one residue $R_1$' or $R_2$' is C(O)OR' or hydroxy.

**10.** 4-[6-(2,3-Dimethoxyphenyl)hexyloxy]-2-hydroxy-3-propylbenzoic acid and its methyl ester.

**11.** Compounds of the formula

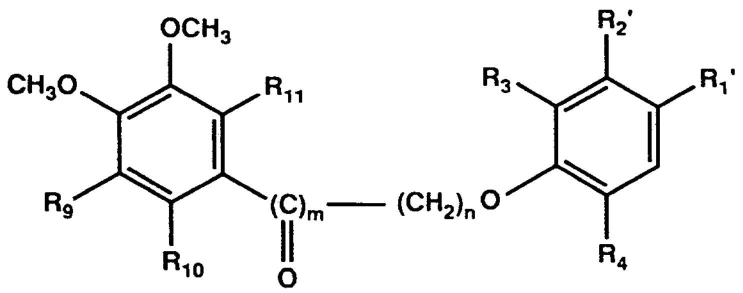

XXXVII

wherein $R_1$' is -C(O)OR', acetyl, hydrogen or hydroxy; $R_2$' is -C(O)OR', hydrogen or hydroxy; R' is hydrogen or lower-alkyl; $R_3$ is hydrogen or lower-alkyl; $R_4$ is hydrogen, lower-alkyl or halogen; $R_9$ is hydrogen or lower-alkyl; $R_{10}$ is hydrogen, lower-alkyl or halogen; $R_{11}$ is hydrogen, lower-alkyl, cycloalkyl or halogen; m is 0 or 1 and n is integer of 2-10, provided that not more than one residue $R_1$' or $R_2$' is -C(O)OR' or hydroxy.

**12.** The compounds of claim 1-8 for use as medicaments.

**13.** The compounds of claim 1-8 for use as active substances in the manufacture of medicaments for the treatment of inflammatory diseases, cardiovascular diseases, skin diseases and bronchopulmonary diseases.

**14.** Pharmaceutical preparations containing a compound of claims 1-8 and a pharmaceutical carrier material.

**15.** A process for the preparation of compounds of claims 1-8, characterized by reacting a compound of the formula

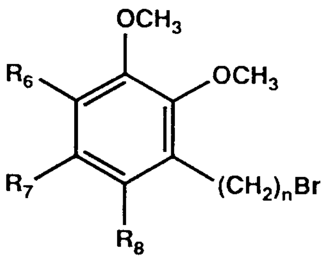

III

or

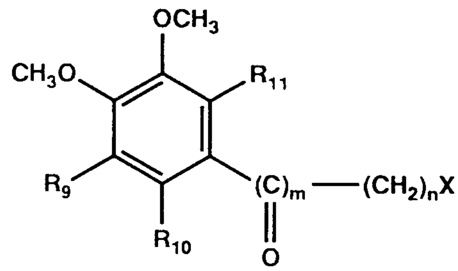

XXXVI

with a compound of the formula

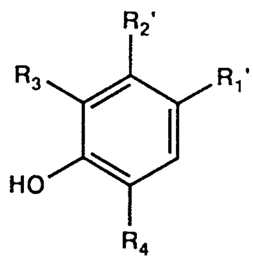

XXXIII

to give a compound of the formulae

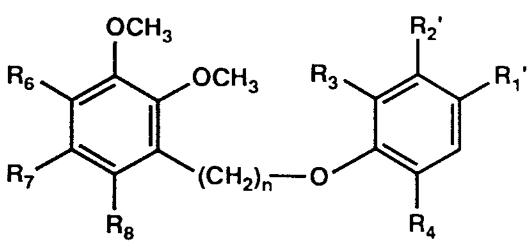

XXXIV

or

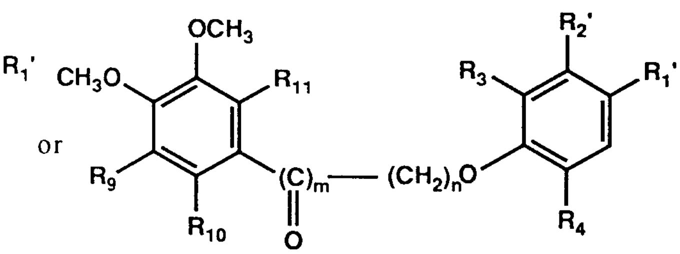

XXXVII

or reacting a compound of the formula

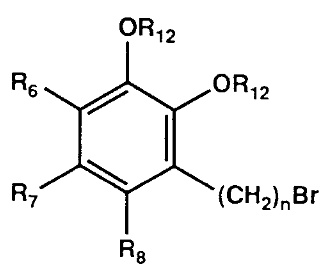

XXXIX

with a compound of the formula

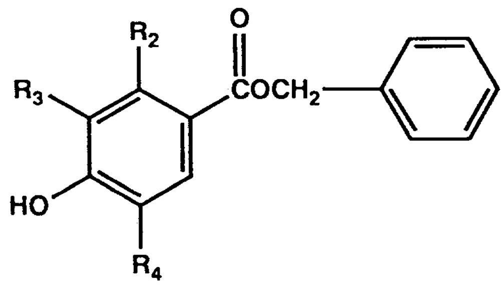

XXXX

to give a compound of the formula

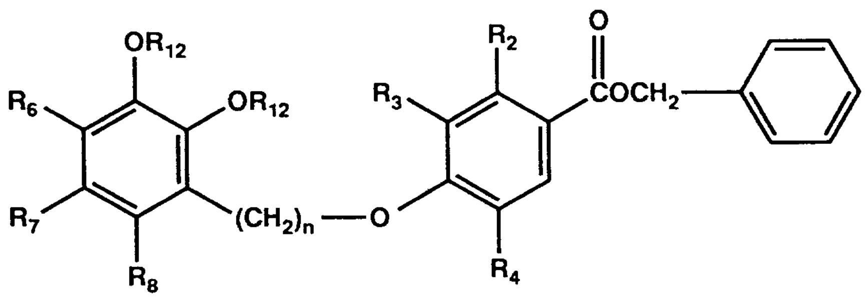

XXXXI

wherein $R_1$' represents hydrogen, lower-alkoxycarbonyl or acetyl; $R_2$' represents hydrogen or lower-alkoxycarbonyl; $R_{12}$ represents benzyl or acyl; X represents bromine or methanesulphonyloxy; and the remaining symbols have the significance given above, provided that not more than one residue $R_1$' or $R_2$' is hydroxy or lower-alkoxycarbonyl,
whereupon the methoxy groups in a compound of formula XXXIV or XXXVII or the benzyl ester and an optionally present benzyl ether group $R_{12}$ in a compound of formula XXXXI are cleaved off, if desired, a hydroxy group $R_2$ is esterified and, if desired, a carboxy group $R_1$ or $R_2$ is esterified or converted into a salt with a pharmaceutically acceptable base or a lower-alkyl-substituted amino group is converted into a salt with a pharmaceutically acceptable acid.

**Claims for the following Contracting State : ES**

**1.** A process for the preparation of compounds of the formula

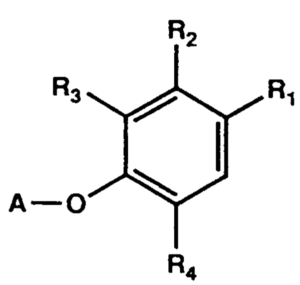

I

wherein $R_1$ represents -C(O)OR, hydrogen, acetyl, hydroxy or alkanoyloxy; $R_2$ represents -C(O)OR, hydroxy, hydrogen or alkanoyloxy; R represents hydrogen, lower-alkyl or $-(CH_2)_n$-N-(lower-alkyl)$_2$; $R_3$

represents hydrogen, lower-alkyl or amino; $R_4$ represents hydrogen, lower-alkyl, halogen or amino, A represents a group of the formula

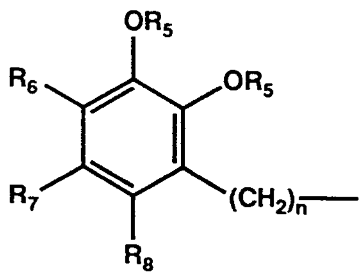

A'

wherein $R_5$ represents hydrogen or acyl; $R_6$ represents hydrogen, halogen, lower-alkyl, aryl or cycloalkyl; and $R_7$ and $R_8$ each independently represent hydrogen, lower-alkyl or halogen and n is an integer of 2-10, or A represents a group of the formula

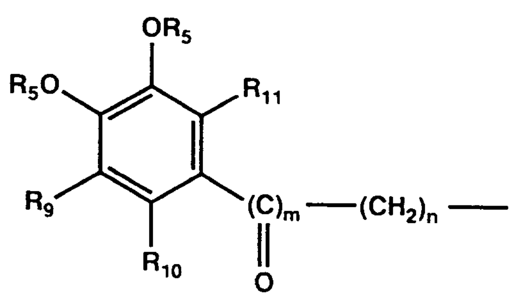

A"

wherein $R_5$ represents hydrogen or acyl; $R_9$ represents hydrogen or lower-alkyl; $R_{10}$ represents hydrogen, lower-alkyl or halogen, $R_{11}$ represents hydrogen, lower-alkyl, cycloalkyl or halogen, m is 0 or 1 and n is an integer of 2-10; provided that not more than one residue $R_1$ or $R_2$ is hydroxy, alkanoyloxy or -C(O)OR,
and, when R is hydrogen, salts thereof with pharmaceutically acceptable bases and, when R is $-(CH_2)_n-N-(lower-alkyl)_2$, acid addition salts thereof with pharmaceutically acceptable acids, characterized by reacting a compound of the formula

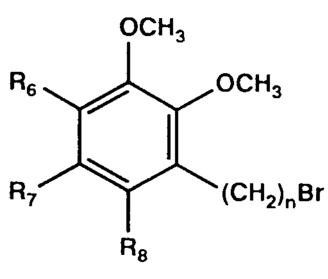

III

or

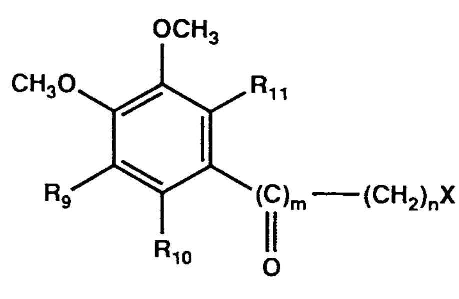

XXXVI

with a compound of the formula

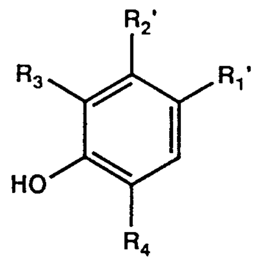

XXXIII

to give a compound of the formulae

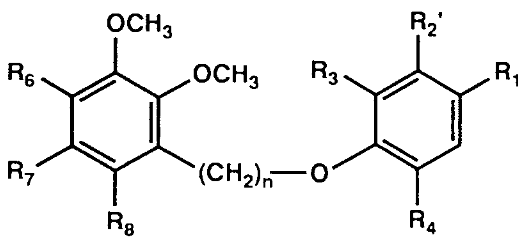

XXXIV

or

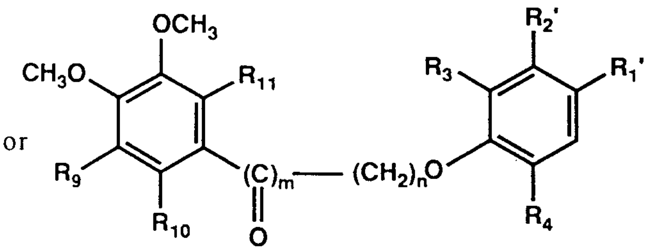

XXXVII

or reacting a compound of the formula

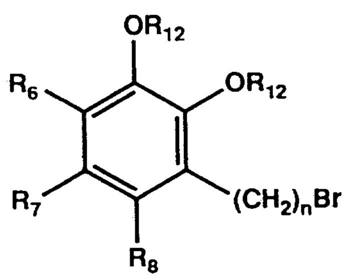

XXXIX

with a compound of the formula

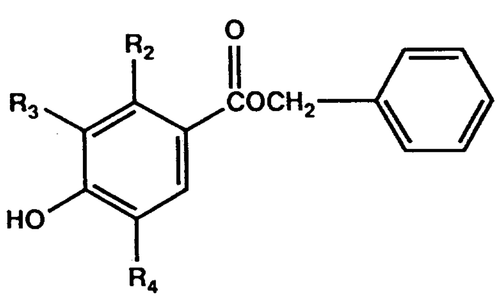

XXXX

to give a compound of the formula

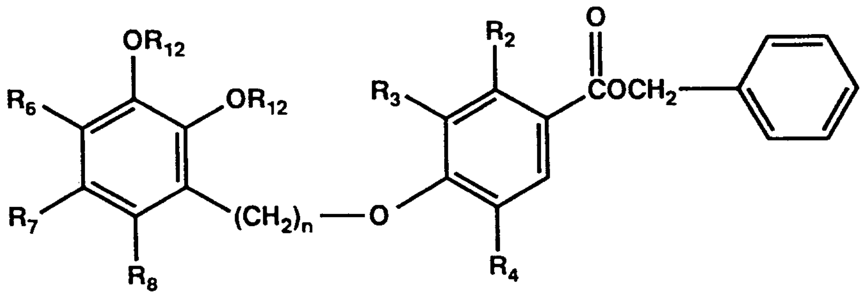

XXXXI

wherein $R_1$' represents hydrogen, lower-alkoxycarbonyl or acetyl; $R_2$' represents hydrogen or lower-alkoxycarbonyl; $R_{12}$ represents benzyl or acyl; X represents bromine or methanesulphonyloxy; and the remaining symbols have the significance given above, provided that not more than one residue $R_1$' or $R_2$' is hydroxy or lower-alkoxycarbonyl,
whereupon the methoxy groups in a compound of formula XXXIV or XXXVII or the benzyl ester and an optionally present benzyl ether group $R_{12}$ in a compound of formula XXXXI are cleaved off, if desired, a hydroxy group $R_2$ is esterified and, if desired, a carboxy group $R_1$ or $R_2$ is esterified or converted into a salt with a pharmaceutically acceptable base or a lower-alkyl-substituted amino group is converted into a salt with a pharmaceutically acceptable acid.

**2.** A process according to claim 1, characterized in that compounds of the formula

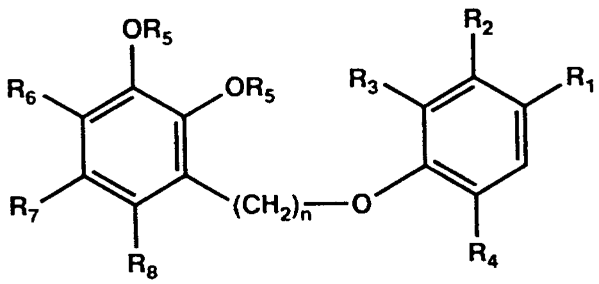

Ia

wherein $R_1$ is carboxy or acetyl, $R_2$ is hydroxy, $R_3$ is hydrogen or propyl, $R_4$ is hydrogen or chlorine, n is an integer of 2-10, $R_5$ is hydrogen or acetyl, $R_6$ is hydrogen, lower-alkyl or aryl and $R_7$ and $R_8$ are hydrogen,
is prepared.

**3.** A process according to claim 1, characterized in that compounds of the formula

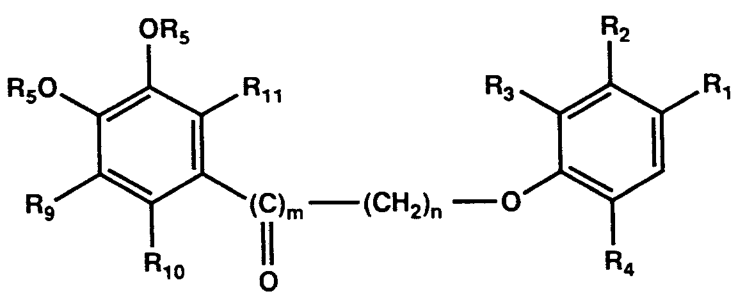

Ib

wherein $R_1$ is carboxy or acetyl, $R_2$ is hydroxy, $R_3$ is hydrogen or propyl, $R_4$ is hydrogen or chlorine, m is 0 or 1, n is an integer of 2-10, $R_5$ is hydrogen or acetyl, $R_9$ and $R_{10}$ are hydrogen and $R_{11}$ is hydrogen or chlorine,
are prepared.

**4.** A process according to claim 1, wherein $R_6$ is hydrogen, lower-alkyl, aryl or cycloalkyl and the remaining symbols have the significance given in claim 1.

**5.** A process according to claim 1, characterized in that 4-[6-(2,3-dihydroxyphenyl)hexyloxy]-2-hydroxybenzoic acid is prepared.

**6.** A process according to claim 1, characterized in that a compound from the following group is prepared:
4-[4-(2,3-Dihydroxyphenyl)butoxy]-2-hydroxy-3-propylbenzoic acid;
4-[6-(2,3-dihydroxyphenyl)hexyloxy]-2-hydroxy-3-propylbenzoic acid;
4-[8-(2,3-dihydroxyphenyl)octyloxy]-2-hydroxy-3-propylbenzoic acid;
4-[6-[2,3-bis(acetyloxy)phenyl]hexyloxy]-2-hydroxy-3-propylbenzoic acid; and
4-[6-[2,3-dihydroxy-4-(1-methylethyl)phenyl]hexyloxy]-2-hydroxy-3-propylbenzoic acid.

**7.** A process according to claim 1, characterized in that a compound from the following group is prepared:
4-[3-(3,4-Dihydroxyphenyl)propoxy]-2-hydroxy-3-propylbenzoic acid;
4-[6-(3,4-dihydroxyphenyl)hexyloxy]-2-hydroxy-3-propylbenzoic acid;
4-[[6-(3,4-dihydroxyphenyl)-6-oxohexyl]oxy]-2-hydroxy-3-propylbenzoic acid; and
1-[2-hydroxy-4-[4-(2,3-dihydroxyphenyl)butoxy]-3-propylphenyl]ethanone.

**8.** A process according to claim 1, characterized in that a compound from the following group is prepared:
4-[6-[2,3-Dihydroxy-4-(1,1-dimethylethyl)phenyl]hexyloxy]-2-hydroxy-3-propylbenzoic acid;
4-[6-[2,3-dihydroxy-4-methylphenyl]hexyloxy]-2-hydroxy-3-propylbenzoic acid;
4-[4-[2,3-dihydroxy-4-(2-methylpropyl)phenyl]butoxy]-2-hydroxy-3-propylbenzoic acid;
4-[6-[2,3-dihydroxy-5,6-dimethylphenyl]hexyloxy]-2-hydroxy-3-propylbenzoic acid;
4-[5-[5-chloro-2,3-dihydroxyphenyl]pentyloxy]-2-hydroxy-3-propylbenzoic acid;
4-[6-[2,3-dihydroxy-6-fluorophenyl]hexyloxy]-2-hydroxy-3-propylbenzoic acid;
4-[4-[2,3-dihydroxy-4-cyclohexylphenyl]butoxy]-2-hydroxy-3-propylbenzoic acid;
4-[4-[2,3-dihydroxy-4-(1,1-dimethylethyl)phenyl]butoxy]-2-hydroxy-3-propylbenzoic acid;
4-[8-(2,3-dihydroxy-4-(1,1-dimethylethyl)pheny]octyloxy]-2-hydroxy-3-propylbenzoic acid;
4-[4-[2,3-dihydroxy-4-(1,1-dimethylethyl)phenyl]butoxy]-2-hydroxybenzoic acid;
4-[8-[2,3-dihydroxy-4-(1-methylethyl)phenyl]octyloxy]-2-hydroxybenzoic acid;
4-[4-[2,3-dihydroxyphenyl]butoxy]-3,5-dipropyl-2-hydroxybenzoicacid;
4-[4-[2,3-dihydroxy-4-(1-methylethyl)phenyl]butoxy]-3,5-dipropylbenzoic acid;
4-[6-[2,3-dihydroxy-4-(1-methylethyl)phenyl]hexyloxy]-3-propylbenzoic acid;
4-[6-[2,3-bis(acetyloxy)-4-(1-methylethyl)phenyl]hexyloxy]-2-hydroxy-3-propylbenzoic acid;
2-acetyloxy-4-[6-(2,3-dihydroxy-4-(1-methylethyl)phenyl]-hexyloxy]-3-propylbenzoic acid;
4-[6-[2,3-bis[4-methylbenzoyl)oxy]-4-(1-methylethyl)phenyl]hexyloxy]-2-hydroxy-3-propybenzoic acid;
1-[2-hydroxy-4-[6-[2,3-dihydroxy-4-(1,1-dimethylethyl)phenyl]hexyloxy]-3-propylphenyl]ethanone;
1-[2-hydroxy-4-[8-[2,3-dihydroxy-6-fluorophenyl]octyloxy]-3-propylphenyl]ethanone;
1-[2-hydroxy-4-[6-[6-chloro-2,3-dihydroxyphenyl]hexyloxy]-3-propylphenyl]ethanone;
1-[2-hydroxy-4-[6-[5,6-dichloro-2,3-dihydroxyphenyl]hexyloxy]-3-propylphenyl]ethanone;
1-[2-hydroxy-4-[6-[2,3-dihydroxy-4,5,6-trichlorophenyl]hexyloxy]-3-propylphenyl]ethanone;
5-[6-(2,3-dihydroxy-4-(1-methylethyl)phenyl]hexyloxy]-2-hydroxybenzoic acid;
4-[6-[2,3-dihydroxy-4-(1-methylethyl)phenyl]hexyloxy]benzoic acid;
4-[6-[2,3-dihydroxy-4-(1-methylethyl)phenyl]hexyloxy]-2-hydroxy-3-propylbenzoic acid ethyl ester;
4-[6-[2,3-dihydroxy-4-(1-methylethyl)phenyl]hexyloxy]-2-hydroxy-3-propylbenzoic acid ethyl ester;
4-[8-(3,4-dihydroxyphenyl)octyloxy]-2-hydroxy-3-propylbenzoic acid;
4-[3-(3,4-dihydroxyphenyl)propoxy]-2-hydroxy-3-propylbenzoic acid ethyl ester;
4-[6-(3,4-dihydroxyphenyl)hexyloxy]-2-hydroxy-benzoic acid;
4-[6-(3,4-dihydroxyphenyl)hexyloxy]-3-propylbenzoic acid;
4-[8-(3,4-dihydroxyphenyl)octyloxy]benzoic acid;
3-[8-(3,4-dihydroxyphenyl)octyloxy]benzoic acid;

5-[8-(3,4-dihydroxyphenyl)octyloxy]-2-hydroxybenzoic acid;
4-[[8-(3,4-dihydroxyphenyl)-8-oxooctyl]oxy]-2-hydroxy-3-propylbenzoic acid;
4-[[4-(3,4-dihydroxyphenyl)-4-oxobutyl]oxy]-2-hydroxy-3-propylbenzoic acid;
4-[6-(3,4-dihydroxy-5-fluorophenyl)hexyloxy]-2-hydroxy-3-propylbenzoic acid;
4-[6-(3,4-dihydroxy-6-fluorophenyl)hexyloxy]-2-hydroxy-3-propylbenzoic acid;
4-[6-(3,4-dihydroxy-6-chlorophenyl)hexyloxy]-2-hydroxy-3-propylbenzoic acid;
4-[4-[3,4-dihydroxy-5-(1-methylethyl)phenyl]butoxy]-2-hydroxy-3-propylbenzoic acid;
4-[4-[3,4-dihydroxy-5-(1,1-dimethylethyl)phenyl]butoxy]-2-hydroxybenzoic acid;
4-[6-[3,4-bis(acetyloxy)phenyl]hexyloxy]-2-hydroxy-3-propylbenzoic acid;
2-acetyloxy-4-[6-(3,4-dihydroxyphenyl)hexyloxy]-3-propylbenzoicacid;
4-[[4-[3,4-dihydroxy-5-(1-methylethyl)phenyl]-4-oxobutyl]oxy]-2-hydroxybenzoic acid;
4-[6-[3,4-dihydroxy-2-(1-methylethyl)phenyl]hexyloxy]-2-hydroxy-3-propylbenzoic acid;
4-[[4-(3,4-dihydroxy-2,5-dimethylphenyl)-4-oxobutyl]oxy]-2-hydroxy-3-propylbenzoic acid;
4-[4-(3,4-dihydroxy-2,5-dimethylphenyl)butoxy]-2-hydroxy-3-propylbenzoic acid;
4-[[6-(3,4-dihydroxy-2,5-6-trimethylphenyl)-6-oxohexyl]oxy]-2-hydroxy-3-propylbenzoic acid;
4-[6-(3,4-dihydroxy-5,6-trimethylphenyl)hexyloxy]-2-hydroxy-3-propylbenzoic acid;
1-[2-hydroxy-4-[6-(3,4-dihydroxyphenyl)hexyloxy]-3-propylphenyl-1-ethanone;
1-[2-hydroxy-4-[8-(3,4-dihydroxyphenyl)octyloxy]-3-propylphenyl-1-ethanone;
1-[2-hydroxy-4-[6-(3,4-dihydroxy-2,5-dimethylphenyl)hexyloxy]-3-propylphenyl-1-ethanone;
1-[2-hydroxy-4-[6-(3,4-dihydroxy-6-chlorophenyl)hexyloxy]-3-propylphenyl-1-ethanone;
1-[2-hydroxy-4-[6-(3,4-dihydroxy-2-chlorophenyl)hexyloxy]-3-propylphenyl-1-ethanone;
1-[2-hydroxy-4-[6-(3,4-dihydroxy-6-fluorophenyl)hexyloxy]-3-propylphenyl-1-ethanone.

**9.** The use of the compounds of formula I and salts thereof according to claim 1 for the manufacture of medicaments for the treatment of inflammatory diseases, cardiovascular diseases, skin diseases and bronchopulmonary diseases.

**Claims for the following Contracting State : GR**

**1.** A process for the preparation of compounds of the formula

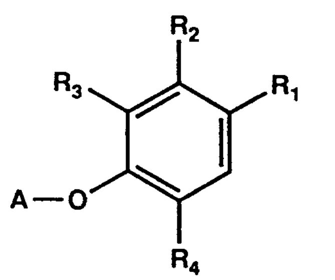

I

wherein $R_1$ represents -C(O)OR, hydrogen, acetyl, hydroxy or alkanoyloxy; $R_2$ represents -C(O)OR, hydroxy, hydrogen or alkanoyloxy; R represents hydrogen, lower-alkyl or $-(CH_2)_n-N-(\text{lower-alkyl})_2$; $R_3$ represents hydrogen, lower-alkyl or amino; $R_4$ represents hydrogen, lower-alkyl, halogen or amino, A represents a group of the formula

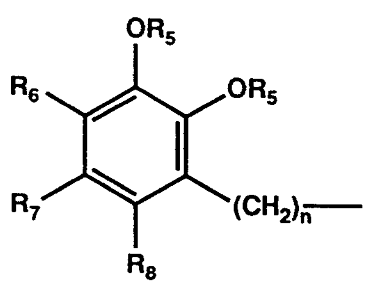

A'

wherein $R_5$ represents hydrogen or acyl; $R_6$ represents hydrogen, halogen, lower-alkyl, aryl or cycloalkyl; and $R_7$ and $R_8$ each independently represent hydrogen, lower-alkyl or halogen and n is an integer of 2-10, or A represents a group of the formula

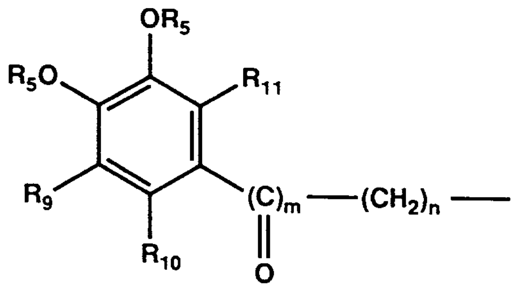

A"

wherein $R_5$ represents hydrogen or acyl; $R_9$ represents hydrogen or lower-alkyl; $R_{10}$ represents hydrogen, lower-alkyl or halogen, $R_{11}$ represents hydrogen, lower-alkyl, cycloalkyl or halogen, m is 0 or 1 and n is an integer of 2-10; provided that not more than one residue $R_1$ or $R_2$ is hydroxy, alkanoyloxy or -C(O)OR,
and, when R is hydrogen, salts thereof with pharmaceutically acceptable bases and, when R is $-(CH_2)_n-N-(lower-alkyl)_2$, acid addition salts thereof with pharmaceutically acceptable acids, characterized by reacting a compound of the formula

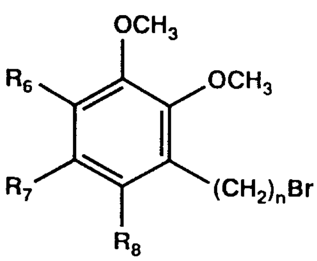

III

or

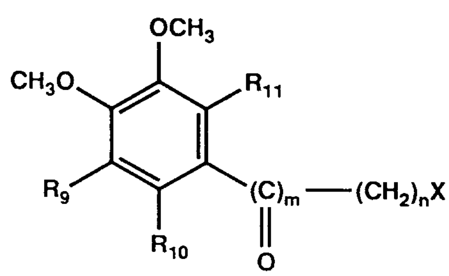

XXXVI

with a compound of the formula

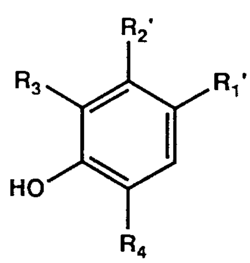

XXXIII

to give a compound of the formulae

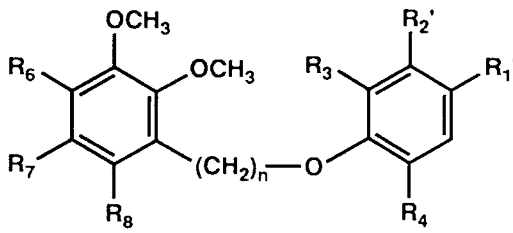

or

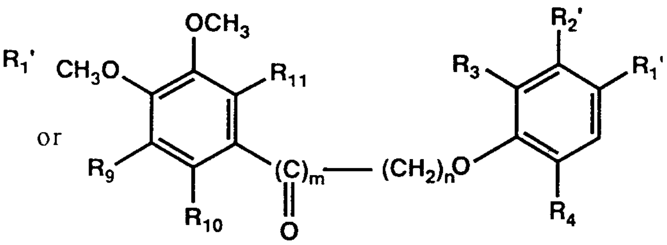

XXXIV

XXXVII

or reacting a compound of the formula

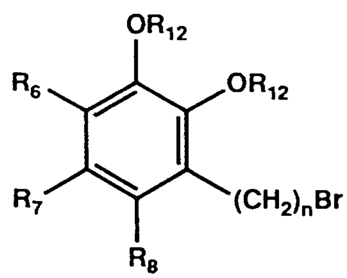

XXXIX

with a compound of the formula

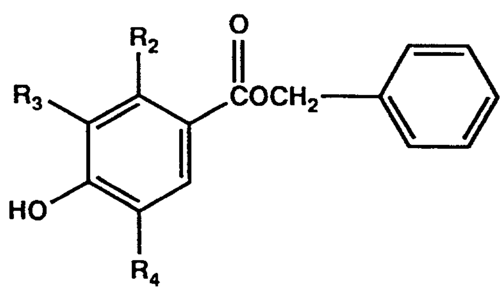

XXXX

to give a compound of the formula

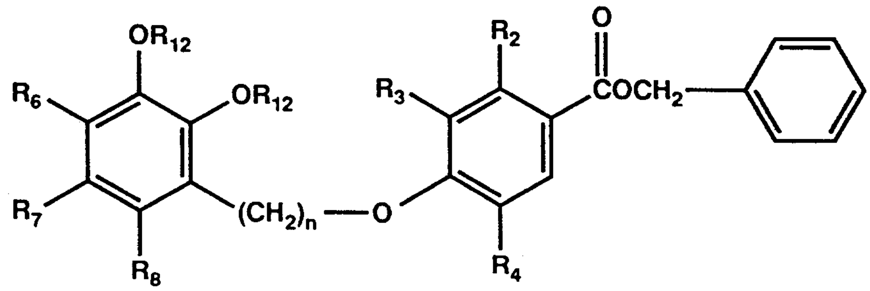

XXXXI

wherein $R_1$' represents hydrogen, lower-alkoxycarbonyl or acetyl; $R_2$' represents hydrogen or lower-alkoxycarbonyl; $R_{12}$ represents benzyl or acyl; X represents bromine or methanesulphonyloxy; and the remaining symbols have the significance given above, provided that not more than one residue $R_1$' or $R_2$' is hydroxy or lower-alkoxycarbonyl,
whereupon the methoxy groups in a compound of formula XXXIV or XXXVII or the benzyl ester and an optionally present benzyl ether group $R_{12}$ in a compound of formula XXXXI are cleaved off, if desired, a hydroxy group $R_2$ is esterified and, if desired, a carboxy group $R_1$ or $R_2$ is esterified or converted into a salt with a pharmaceutically acceptable base or a lower-alkyl-substituted amino group is converted into a salt with a pharmaceutically acceptable acid.

**2.** A process according to claim 1, characterized in that compounds of the formula

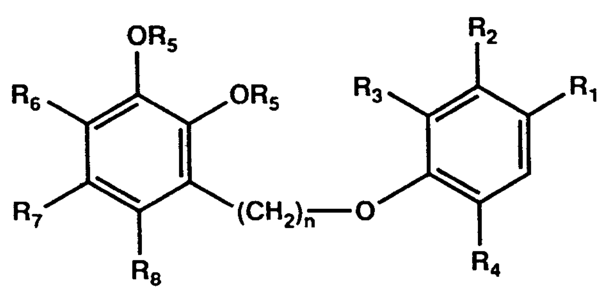

Ia

wherein $R_1$ is carboxy or acetyl, $R_2$ is hydroxy, $R_3$ is hydrogen or propyl, $R_4$ is hydrogen or chlorine, n is an integer of 2-10, $R_5$ is hydrogen or acetyl, $R_6$ is hydrogen, lower-alkyl or aryl and $R_7$ and $R_8$ are hydrogen,
is prepared.

**3.** A process according to claim 1, characterized in that compounds of the formula

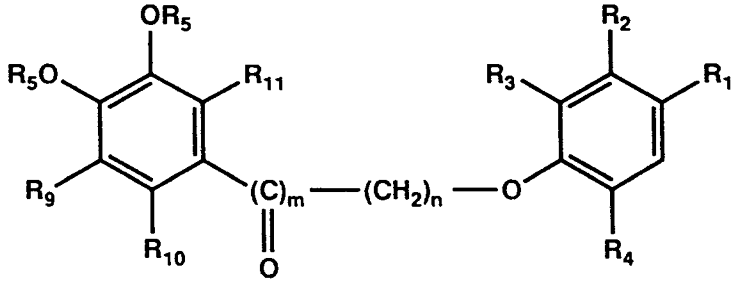

Ib

wherein $R_1$ is carboxy or acetyl, $R_2$ is hydroxy, $R_3$ is hydrogen or propyl, $R_4$ is hydrogen or chlorine, m is 0 or 1, n is an integer of 2-10, $R_5$ is hydrogen or acetyl, $R_9$ and $R_{10}$ are hydrogen and $R_{11}$ is hydrogen or chlorine,
are prepared.

**4.** A process according to claim 1, wherein $R_6$ is hydrogen, lower-alkyl, aryl or cycloalkyl and the remaining symbols have the significance given in claim 1.

**5.** A process according to claim 1, characterized in that 4-[6-(2,3-dihydroxyphenyl)hexyloxy]-2-hydroxybenzoic acid is prepared.

**6.** A process according to claim 1, characterized in that a compound from the following group is prepared:
4-[4-(2,3-Dihydroxyphenyl)butoxy]-2-hydroxy-3-propylbenzoic acid;
4-[6-(2,3-dihydroxyphenyl)hexyloxy]-2-hydroxy-3-propylbenzoic acid;
4-[8-(2,3-dihydroxyphenyl)octyloxy]-2-hydroxy-3-propylbenzoic acid;
4-[6-[2,3-bis(acetyloxy)phenyl]hexyloxy]2-hydroxy-3-propylbenzoic acid; and
4-[6-[2,3-dihydroxy-4-(1-methylethyl)phenyl]hexyloxy]-2-hydroxy-3-propylbenzoic acid.

**7.** A process according to claim 1, characterized in that a compound from the following group is prepared:
4-[3-(3,4-Dihydroxyphenyl)propoxy]-2-hydroxy-3-propylbenzoic acid;
4-[6-(3,4-dihydroxyphenyl)hexyloxy]-2-hydroxy-3-propylbenzoic acid;
4-[[6-(3,4-dihydroxyphenyl)-6-oxohexyl]oxy]-2-hydroxy-3-propylbenzoic acid; and
1-[2-hydroxy-4-[4-(2,3-dihydroxyphenyl)butoxy]-3-propylphenyl]ethanone.

**8.** A process according to claim 1, characterized in that a compound from the following group is prepared:
4-[6-[2,3-Dihydroxy-4-(1,1-dimethylethyl)phenyl]hexyloxy]-2-hydroxy-3-propylbenzoic acid;
4-[6-[2,3-dihydroxy-4-methylphenyl]hexyloxy]-2-hydroxy-3-propylbenzoic acid;
4-[4-[2,3-dihydroxy-4-(2-methylpropyl)phenyl]butoxy]-2-hydroxy-3-propylbenzoic acid;
4-[6-[2,3-dihydroxy-5,6-dimethylphenyl]hexyloxy]-2-hydroxy-3-propylbenzoic acid;
4-[5-[5-chloro-2,3-dihydroxyphenyl]pentyloxy]-2-hydroxy-3-propylbenzoic acid;
4-[6-[2,3-dihydroxy-6-fluorophenyl]hexyloxy]-2-hydroxy-3-propylbenzoic acid;
4-[4-[2,3-dihydroxy-4-cyclohexylphenyl]butoxy]-2-hydroxy-3-propylbenzoic acid;
4-[4-[2,3-dihydroxy-4-(1,1-dimethylethyl)phenyl]butoxy]-2-hydroxy-3-propylbenzoic acid;
4-[8-[2,3-dihydroxy-4-(1,1-dimethylethyl)phenyl]octyloxy]-2-hydroxy-3-propylbenzoic acid;
4-[4-[2,3-dihydroxy-4-(1,1-dimethylethyl)phenyl]butoxy]-2-hydroxybenzoic acid;
4-[8-[2,3-dihydroxy-4-(1-methylethyl)phenyl]octyloxy]-2-hydroxybenzoic acid;
4-[4-[2,3-dihydroxyphenyl]butoxy]-3,5-dipropyl-2-hydroxybenzoicacid;
4-[4-[2,3-dihydroxy-4-(1-methylethyl)phenyl]butoxy]-3,5-dipropylbenzoic acid;
4-[6-[2,3-dihydroxy-4-(1-methylethyl)phenyl]hexyloxy]-3-propylbenzoic acid;
4-[6-[2,3-bis(acetyloxy)-4-(1-methylethyl)phenyl]hexyloxy]-2-hydroxy-3-propylbenzoic acid;
2-acetyloxy-4-[6-(2,3-dihydroxy-4-(1-methylethyl)phenyl]-hexyloxy]-3-propylbenzoic acid;
4-[6-[2,3-bis[4-methylbenzoyl)oxy]-4-(1-methylethyl)phenyl]hexyloxy]-2-hydroxy-3-propylbenzoic acid;
1-[2-hydroxy-4-[6-[2,3-dihydroxy-4-(1,1-dimethylethyl)phenyl]hexyloxy]-3-propylphenyl]ethanone;
1-[2-hydroxy-4-[8-[2,3-dihydroxy-6-fluorophenyl]octyloxy]-3-propylphenyl]ethanone;
1-[2-hydroxy-4-[6-[6-chloro-2,3-dihydroxyphenyl]hexyloxy]-3-propylphenyl]ethanone;
1-[2-hydroxy-4-[6-[5,6-dichloro-2,3-dihydroxyphenyl]hexyloxy]-3-propylphenyl]ethanone;
1-[2-hydroxy-4-[6-[2,3-dihydroxy-4,5,6-trichlorophenyl]hexyloxy]-3-propylphenyl]ethanone;
5-[6-(2,3-dihydroxy-4-(1-methylethyl)phenyl]hexyloxy]-2-hydroxybenzoic acid;
4-[6-[2,3-dihydroxy-4-(1-methylethyl)phenyl]hexyloxy]benzoic acid;
4-[6-[2,3-dihydroxy-4-(1-methylethyl)pheny]hexyloxy]-2-hydroxy-3-propylbenzoic acid ethyl ester;
4-[6-[2,3-dihydroxy-4-(1-methylethyl)phenyl]hexyoxy]-2-hydroxy-3-propylbenzoic acid ethyl ester;
4-[8-(3,4-dihydroxyphenyl)octyloxy]-2-hydroxy-3-propylbenzoic acid;
4-[3-(3,4-dihydroxyphenyl)propoxy]-2-hydroxy-3-propylbenzoic acid ethyl ester;
4-[6-(3,4-dihydroxyphenyl)hexyloxy]-2-hydroxy-benzoic acid;
4-[6-(3,4-dihydroxyphenyl)hexyloxy]-3-propylbenzoic acid;
4-[8-(3,4-dihydroxyphenyl)octyloxy]benzoic acid;
3-[8-(3,4-dihydroxyphenyl)octyloxy]benzoic acid;

5-[8-(3,4-dihydroxyphenyl)octyloxy]-2-hydroxybenzoic acid;
4-[[8-(3,4-dihydroxyphenyl)-8-oxooctyl]oxy]-2-hydroxy-3-propylbenzoic acid;
4-[[4-(3,4-dihydroxyphenyl)-4-oxobutyl]oxy]-2-hydroxy-3-propylbenzoic acid;
4-[6-(3,4-dihydroxy-5-fluorophenyl)hexyloxy]-2-hydroxy-3-propylbenzoic acid;
4-[6-(3,4-dihydroxy-6-fluorophenyl)hexyloxy]-2-hydroxy-3-propylbenzoic acid;
4-[6-(3,4-dihydroxy-6-chlorophenyl)hexyloxy]-2-hydroxy-3-propylbenzoic acid;
4-[4-[3,4-dihydroxy-5-(1-methylethyl)phenyl]butoxy]-2-hydroxy-3-propylbenzoic acid;
4-[4-[3,4-dihydroxy-5-(1,1-dimethylethyl)phenyl]butoxy]-2-hydroxybenzoic acid;
4-[6-[3,4-bis(acetyloxy)phenyl]hexyloxy]-2-hydroxy-3-propylbenzoic acid;
2-acetyloxy-4-[6-(3,4-dihydroxyphenyl)hexyloxy]-3-propylbenzoicacid;
4-[[4-[3,4-dihydroxy-5-(1-methylethyl)phenyl]-4-oxobutyl]oxy]-2-hydroxybenzoic acid;
4-[6-[3,4-dihydroxy-2-(1-methylethyl)phenyl]hexyloxy]-2-hydroxy-3-propylbenzoic acid;
4-[[4-(3,4-dihydroxy-2,5-dimethylphenyl)-4-oxobutyl]oxy]-2-hydroxy-3-propylbenzoic acid;
4-[4-(3,4-dihydroxy-2,5-dimethylphenyl)butoxy]-2-hydroxy-3-propylbenzoic acid;
4-[[6-(3,4-dihydroxy-2,5-6-trimethylphenyl)-6-oxohexyl]oxy]-2-hydroxy-3-propylbenzoic acid;
4-[6-(3,4-dihydroxy-5,6-trimethylphenyl)hexyloxy]-2-hydroxy-3-propylbenzoic acid;
1-[2-hydroxy-4-[6-(3,4-dihydroxyphenyl)hexyloxy]-3-propylphenyl-1-ethanone;
1-[2-hydroxy-4-[8-(3,4-dihydroxyphenyl)octyloxy]-3-propylphenyl-1-ethanone;
1-[2-hydroxy-4-[6-(3,4-dihydroxy-2,5-dimethylphenyl)hexyloxy]-3-propylphenyl-1-ethanone;
1-[2-hydroxy-4-[6-(3,4-dihydroxy-6-chlorophenyl)hexyloxy]-3-propylphenyl-1-ethanone;
1-[2-hydroxy-4-[6-(3,4-dihydroxy-2-chlorophenyl)hexyloxy]-3-propylphenyl-1-ethanone;
1-[2-hydroxy-4-[6-(3,4-dihydroxy-6-fluorophenyl)hexyloxy]-3-propylphenyl-1-ethanone.

**9.** The use of the compounds of formula I and salts thereof according to claim 1 for the manufacture of medicaments for the treatment of inflammatory diseases, cardiovascular diseases, skin diseases and bronchopulmonary diseases.

**10.** Compounds of the formula

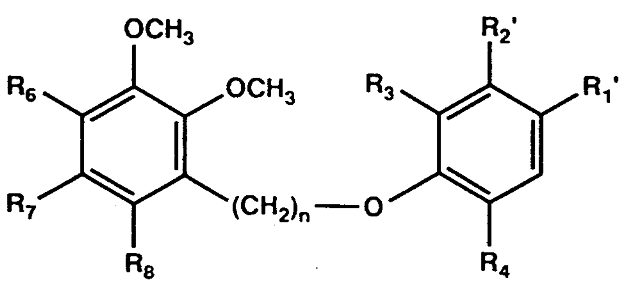

XXXIV

wherein $R_1$' is -C(O)OR', acetyl, hydrogen or hydroxy; $R_2$' is -C(O)OR', hydrogen or hydroxy; R' is hydrogen or lower-alkyl; $R_3$ is hydrogen or lower-alkyl; $R_4$ is hydrogen, lower-alkyl or halogen; $R_6$ is hydrogen, lower-alkyl, aryl or cycloalkyl; $R_7$ and $R_8$ are hydrogen, lower-alkyl or halogen; and n is an integer of 2-10; provided that not more than one residue $R_1$' or $R_2$' is C(O)OR' or hydroxy.

**11.** 4-[6-(2,3-Dimethoxyphenyl)hexyloxy]-2-hydroxy-3-propylbenzoic acid and its methyl ester.

**12.** Compounds of the formula

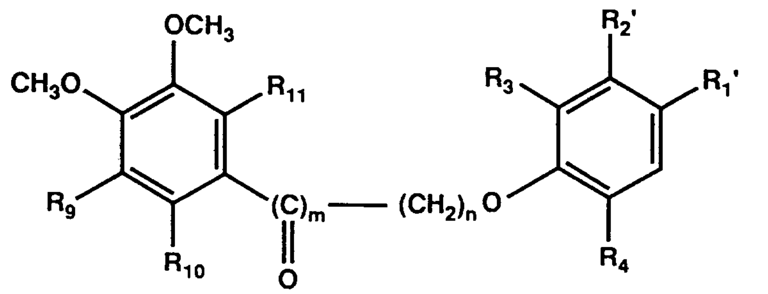

XXXVII

wherein $R_1'$ is -C(O)OR', acetyl, hydrogen or hydroxy; $R_2'$ is -C(O)OR', hydrogen or hydroxy; R' is hydrogen or lower-alkyl; $R_3$ is hydrogen or lower-alkyl; $R_4$ is hydrogen, lower-alkyl or halogen; $R_9$ is hydrogen or lower-alkyl; $R_{10}$ is hydrogen, lower-alkyl or halogen; $R_{11}$ is hydrogen, lower-alkyl, cycloalkyl or halogen; m is 0 or 1 and n is integer of 2-10, provided that not more than one residue $R_1'$ or $R_2'$ is -C(O)OR' or hydroxy.

## Revendications

**Revendications pour les Etats contractants suivants : AT, BE, CH, DE, FR, GB, IT, LI, LU, NL, SE**

**1.** Composés de formule

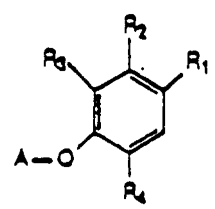

I

dans laquelle $R_1$ représente -C(O)OR, un hydrogène, un acétyle, un hydroxy ou un alcanoyloxy ; $R_2$ représente - C(O)OR, un hydroxy, un hydrogène ou un alcanoyloxy ; R représente un hydrogène, un alcoyle inférieur ou un - $(CH_2)_nN$ (alcoyle inférieur)$_2$ ; $R_3$ représente un hydrogène, un alcoyle inférieur ou un amino ; $R_4$ représente un hydrogène, un alcoyle inférieur, un halogène ou un amino, A représente un groupe de formule

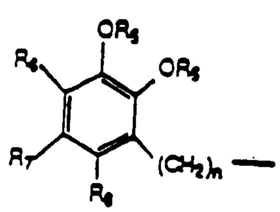

A'

dans laquelle $R_5$ représente un hydrogène ou un acyle ; $R_6$ représente un hydrogène, un halogène, un alcoyle inférieur, un aryle ou un cycloalcoyle ; et $R_7$ et $R_8$ représentent indépendamment l'un de l'autre un hydrogène, un alcoyle inférieur ou un halogène et n est un nombre allant de 2 à 10, ou A représente un groupe de formule

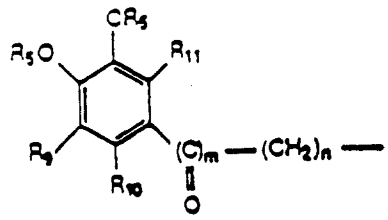

A"

où $R_5$ représente un hydrogène ou un acyle ; $R_9$ représente un hydrogène ou un alcoyle inférieur ; $R_{10}$ représente un hydrogène, un alcoyle inférieur ou un halogène ; $R_{11}$ représente un hydrogène, un alcoyle inférieur, un cycloalcoyle ou un halogène, m vaut 0 ou 1 et n est un nombre entier allant de 2 à 10 ; avec la condition que pas plus d'un radical $R_1$ ou $R_2$ est un hydroxy, un alcanoyloxy ou -C(O)OR, et, si R est un hydrogène, leurs sels avec des bases pharmaceutiquement acceptables, et si R représente $(CH_2)_n$-N-(alcoyle inférieur)$_2$, leurs sels d'addition d'acides avec des acides pharmaceutiquement acceptables.

**2.** Composés de formule

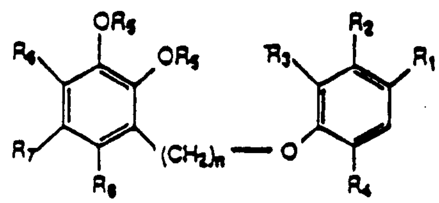

Ia

dans laquelle $R_1$ représente un carboxy ou un acétyle, $R_2$ un hydroxy, $R_3$ ou hydrogène ou un propyle, $R_4$ un hydrogène ou un chlore, n est un nombre entier allant de 2 à 10, $R_5$ est un hydrogène ou un acétyle, $R_6$ est un hydrogène, un alcoyle inférieur ou un aryle et $R_7$ et $R_8$ représentent un hydrogène.

**3.** Composés de formule

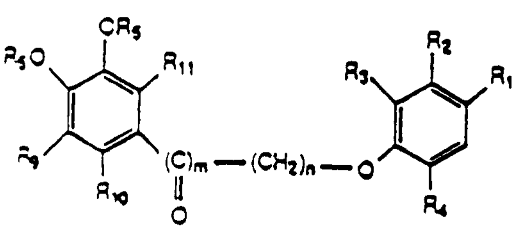

Ib

dans laquelle $R_1$ représente un carboxy ou un acétyle, $R_2$ représente un hydroxy, $R_3$ représente un hydrogène ou un propyle, $R_4$ représente un hydrogène ou un chlore, m vaut 0 ou 1, n est un nombre entier allant de 2 à 10, $R_5$ un hydrogène ou un acétyle, $R_9$ et $R_{10}$ un hydrogène et $R_{11}$ un hydrogène ou un chlore.

**4.** Composés de formule I de la revendication 1, où $R_6$ représente un hydrogène, un alcoyle inférieur, un aryle ou un cycloalcoyle, et les autres symboles ont la signification indiquée dans la revendication 1 ; et, si R est un hydrogène, leurs sels avec des bases pharmaceutiquement acceptables et, si R représente - $(CH_2)_n$-N-(alcoyle inférieur)$_2$, leurs sels d'addition d'acides avec des acides pharmaceutiquement acceptables.

**5.** Acide 4-[6-(2,3-dihydroxyphényl)hexyloxy]-2-hydroxybenzoïque.

**6.** Acide 4-[4-(2,3-dihydroxyphényl)butoxy]-2-hydroxy-3-propyl-benzoïque ;
Acide 4-[6-(2,3-dihydroxyphényl)hexyloxy]-2-hydroxy-3-propyl-benzoïque ;
Acide 4-[8-(2,3-dihydroxyphényl)octyloxy]-2-hydroxy-3-propyl-benzoïque ;
Acide 4-[6-[2,3-bis(acétyloxy)phényl]hexyloxy]-2-hydroxy-3-propylbenzoïque ; et
Acide 4-[6-[2,3-dihydroxy-4-(1-méthyléthyl)phényl]hexyloxy]-2-hydroxy-3-propylbenzoïque.

**7.** Acide 4-[3-(3,4-dihydroxyphényl)propoxy]-2-hydroxy-3-propylbenzoïque ;
Acide 4-[6-(3,4-dihydroxyphényl)hexyloxy]-2-hydroxy-3-propyl-benzoïque ;
Acide 4-[[6-(3,4-dihydroxyphényl)-6-oxohexyl]oxy]-2-hydroxy-3-propylbenzoïque ; et
1-[2-hydroxy-4-[4-(2,3-dihydroxyphényl)butoxy]-3-propylphényl]éthanone ;

**8.** Acide 4-[6-[2,3-dihydroxy-4-(1,1-diméthyléthyl)phényl]hexyloxy]-2-hydroxy-3-propylbenzoïque ;
Acide 4-[6-[2,3-dihydroxy-4-méthylphényl]hexyloxy]-2-hydroxy-3-propylbenzoïque ;
Acide 4-[4-[2,3-dihydroxy-4-(2-méthylpropyl)phényl]butoxy-2-hydroxy-3-propylbenzoïque ;
Acide 4-[6-[2,3-dihydroxy-5,6-diméthylphényl]-hexyloxy]-2-hydroxy-3-propylbenzoïque ;
Acide 4-[5-[5-chloro-2,3-dihydroxyphényl]entyloxy]-2-hydroxy-3-propylbenzoïque ;
Acide 4-[6-[2,3-dihydroxy-6-fluorophényl]exyloxy]-2-hydroxy-3-propylbenzoïque ;
Acide 4-[4-[2,3-dihydroxy-4-cyclohexylphényl]-utoxy]-2-hydroxy-3-propylbenzoïque ;
Acide 4-[4-[2,3-dihydroxy-4-(1,1-diméthyléthyl)phényl]butoxy]-2-hydroxy-3-propylbenzoïque ;
Acide 4-[8-[2,3-dihydroxy-4-(1,1-diméthyléthyl)phényl]octyloxy]-2-hydroxy-3-propylbenzoïque ;
Acide 4-[4-[2,3-dihydroxy-4-(1,1-diméthyléthyl)phényl]butoxy]-2-hydroxybenzoïque ;
Acide 4-[8-[2,3-dihydroxy-4-(1-méthyléthyl)phényl]octyloxy]-2-hydroxybenzoïque ;
Acide 4-[4-[2,3-dihydroxyphényl]butoxy]-3,5-dipropyl-2-hydroxybenzoïque ;
Acide 4-[4-[2,3-dihydroxy-4-(1-méthyléthyl)phényl]butoxy]-3,5-dipropylbenzoïque ;
Acide 4-[6-[2,3-dihydroxy-4-(1-méthyléthyl)phényl]hexyloxy]-3-propylbenzoïque ;
Acide 4-[6-[2,3-bis-acétyloxy)-4-(1-méthyléthyl)phényl]hexyloxy]-2-hydroxy-3-propylbenzoïque ;
Acide 2-acétyloxy-4-[6-(2,3-dihydroxy-4-(1-méthyléthyl)phényl]-hexyloxy]-3-propylbenzoïque ;
Acide 4-[6-[2,3-bis[4-méthylbenzoyl)oxy]-4-(1-méthyléthyl)-phényl]hexyloxy]-2-hydroxy-3-propylbenzoïque ;
1-[2-hydroxy-4-[6-[2,3-dihydroxy-4-(1,1-diméthyléthyl)-phényl]hexyloxy]-3-propylphényl]éthanone ;
1-[2-hydroxy-4-[8-[2,3-dihydroxy-6-fluorophényl]octyloxy]-3-propylphényl]éthanone ;
1-[2-hydroxy-4-(6-[6-chloro-2,3-dihydroxyphényl]-hexyloxy]-3-propylphényl]éthanone ;
1-[2-hydroxy-4-[6-[5,6-dichloro-2,3-dihydroxyphényl]hexyloxy]-3-propylphényl]éthanone ;
1-[2-hydroxy-4-[6-[2,3-dihydroxy-4,5,6-trichlorophényl]-hexyloxy]-3-propylphényl]éthanone ;
Acide 5-[6-(2,3-dihydroxy-4-(1-méthyléthyl)phényl]hexyloxy]-2-hydroxybenzoïque ;
Acide 4-[6-[2,3-dihydroxy-4-(1-méthyléthyl)-phényl]hexyloxy]-benzoïque ;
Ester éthylique de l'acide 4-[6-[2,3-dihydroxy-4-(1-méthyléthyl)phényl]hexyloxy]-2-hydroxy-3-propylbenzoïque ;
Ester éthylique de l'acide 4-[6-[2,3-dihydroxy-4-(1-méthyléthyl)phényl]hexyloxy]-2-hydroxy-3-propylbenzoïque ;
Acide 4-[8-(3,4-dihydroxyphényl)octyloxy]-2-hydroxy-3-propyl-benzoïque ;
Ester éthylique de l'acide 4-[3-(3,4-dihydroxyphényl)propoxy]-2-hydroxy-3-propylbenzoïque ;
Acide 4-[6-(3,4-dihydroxyphényl)hexyloxy]-2-hydroxy-benzoïque ;
Acide 4-[6-(3,4-dihydroxyphényl)hexyloxy]-3-propylbenzoïque ;
Acide 4-[8-(3,4-dihydroxyphényl)octyloxy]benzoïque ;
Acide 3-[8-(3,4-dihydroxyphényl)octyloxy]benzoïque ;
Acide 5-[8-(3,4-dihydroxyphényl)octyloxy]-2-hydroxybenzoïque ;
Acide 4-[[8-(3,4-dihydroxyphényl)-8-oxooctyl]oxy]-2-hydroxy-3-propylbenzoïque ;
Acide 4-[[4-(3,4-dihydroxyphényl)-4-oxobutyl]-oxy]-2-hydroxy-3-propylbenzoïque ;
Acide 4-[6-(3,4-dihydroxy-5-fluorophényl)hexyloxy]-2-hydroxy-3-propylbenzoïque ;
Acide 4-[6-(3,4-dihydroxy-6-fluorophényl)hexyloxy]-2-hydroxy-3-propylbenzoïque ;
Acide 4-[6-(3,4-dihydroxy-6-chlorophényl)hexyloxy]-2-hydroxy-3-propylbenzoïque ;
Acide 4-[4-[3,4-dihydroxy-5-(1-méthyléthyl)phényl]butoxy]-2-hydroxy-3-propylbenzoïque ;
Acide 4-[4-[3,4-dihydroxy-5-(1,1-diméthyléthyl)-phényl]butoxy]-2-hydroxybenzoïque ;
Acide 4-[6-[3,4-bis(acétyloxy)phényl]hexyloxy]-2-hydroxy-3-propylbenzoïque ;
Acide 2-acétyloxy-4-[6-(3,4-dihydroxyphényl)hexyloxy]-3-propylbenzoïque ;
Acide 4-[[4-[3,4-dihydroxy-5-(1-méthyléthyl)phényl]-4-oxobutyl]-oxy]-2-hydroxybenzoïque ;
Acide 4-[6-[3,4-dihydroxy-2-(1-méthyléthyl)phényl]hexyloxy]-2-hydroxy-3-proylbenzoïque ;

Acide 4-[[4-(3,4-dihydroxy-2,5-diméthylphényl)-4-oxobutyl]oxy]-2-hydroxy-3-propylbenzoïque ;
Acide 4-[4-(3,4-dihydroxy-2,5-diméthylphényl)butoxy]-2-hydroxy-3-propylbenzoïque ;
Acide 4-[[6-(3,4-dihydroxy-2,5,6-triméthylphényl)-6-oxohexyl]-oxy]-2-hydroxy-3-propylbenzoïque ;
Acide 4-[6-(3,4-dihydroxy-5,6-triméthylphényl)-hexyloxy]-2-hydroxy-3-propylbenzoïque ;
1-[2-hydroxy-4-[6-(3,4-dihydroxyphényl)hexyloxy]-3-propyl-phényl-1-éthanone ;
1-[2-hydroxy-4-[8-(3,4-dihydroxyphényl)octyloxy]-3-propyl-phényl-1-éthanone ;
1-[2-hydroxy-4-[6-(3,4-dihydroxy-2,5-diméthyl-phényl)hexyloxy]-3-propylphényl-1-éthanone ;
1-[2-hydroxy-4-[6-(3,4-dihydroxy-6-chlorophényl)-hexyloxy]-3-propylphényl-1-éthanone ;
1-[2-hydroxy-4-[6-(3,4-dihydroxy-2-chlorophényl)hexyloxy]-3-propylphényl-1-éthanone ;
1-[2-hydroxy-4-[6-(3,4-dihydroxy-6-fluorophényl)hexyloxy]-3-propylphényl-1-éthanone.

**9.** Composés de formule

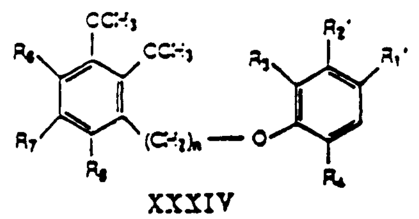

XXXIV

dans laquelle $R_1$' représente -C(O)OR', un acétyle, un hydrogène ou un hydroxy ; $R_2$' représente un -C(O)OR', un hydrogène ou un hydroxy ; R' représente un hydrogène ou un alcoyle inférieur ; $R_3$ représente un hydrogène ou un alcoyle inférieur ; $R_4$ représente un hydrogène, un alcoyle inférieur ou un halogène ; $R_6$ représente un hydrogène, alcoyle inférieur, aryle ou cycloalcoyle ; $R_7$ et $R_8$ représentent un hydrogène, alcoyle inférieur ou halogène ; et n représente un nombre entier allant de 2 à 10 ; en supposant que pas plus d'un radical $R_1$' ou $R_2$' représente -C(O)OR' ou un hydroxy.

**10.** Acide 4-[6-(2,3-diméthoxyphényl)hexyloxy]-2-hydroxy-3-propylbenzoïque et son ester méthylique.

**11.** Composés de formule

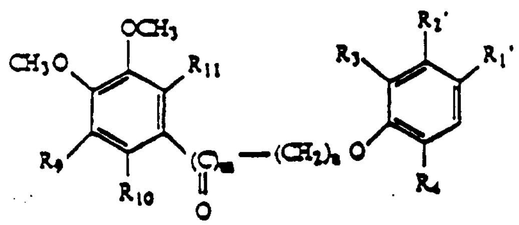

XXXVII

dans laquelle $R_1$' représente -C(O)OR', un acétyle, un hydrogène ou un hydroxy ; $R_2$' représente -C(O)-OR', un hydrogène ou un hydroxy ; R' représente un hydrogène ou un alcoyle inférieur, $R_3$ représente un hydrogène ou un alcoyle inférieur ; $R_4$ représente un hydrogène, alcoyle inférieur, ou halogène ; $R_9$ représente un hydrogène ou un alcoyle inférieur ; $R_{10}$ représente un hydrogène, alcoyle inférieur ou halogène ; $R_{11}$ représente un hydrogène, alcoyle inférieur, cycloalcoyle ou halogène ; m vaut 0 ou 1 et n est un nombre entier compris entre 2 et 10, en supposant que pas plus d'un radical $R_1$' ou $R_2$' représente -C(O)OR' ou un hydroxy.

**12.** Les composés des revendications 1-8 aux fins d'application comme médicaments.

**13.** Les composés des revendications 1-8 aux fins d'application comme substances actives dans la préparation de médicaments pour le traitement des maladies inflammatoires, des maladies cardiovasculaires, des maladies de la peau et des maladies bronchopulmonaires.

**14.** Préparations pharmaceutiques contenant un composé des revendications 1-8 et un support pharmaceutique.

**15.** Procédé de préparation de composés des revendications 1-8, caractérisé en ce qu'on fait réagir un composé de formule

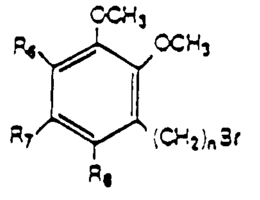

III

ou

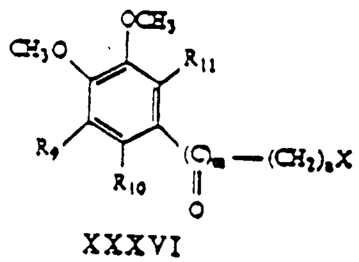

XXXVI

avec un composé de formule

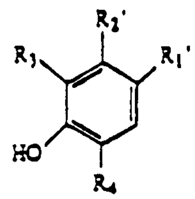

XXXIII

pour donner un composé de formule

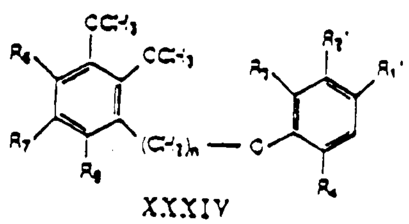

XXXIV

ou

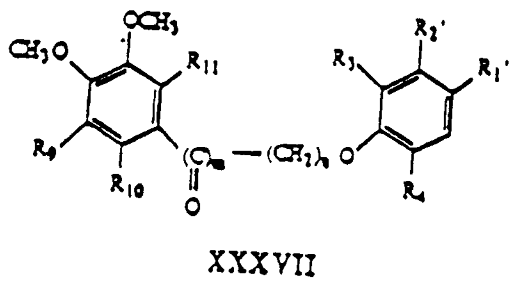

XXXVII

ou
en ce qu'on fait réagir un composé de formule

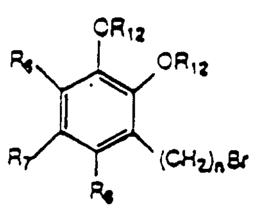

XXXIX

avec un composé de formule

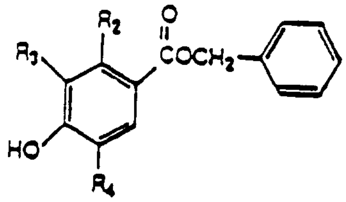

XXXX

pour donner un composé de formule

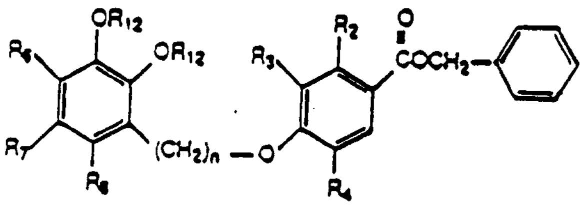

XXXXI

dans laquelle $R_1$' représente un hydrogène, un alcoxy inférieur carbonyle, ou un acétyle ; $R_2$' représente un hydrogène ou un alcoxy inférieur carbonyle ; $R_{12}$ représente un benzyle ou un acyle ; X représente un brome ou un méthanesulfonyloxy et les autres symboles ont la signification indiquée ci-dessus, en supposant que pas plus d'un radical $R_1$' ou $R_2$' ne représente un alcoxy inférieur carbonyle,

après quoi on sépare les groupes méthoxy dans un composé de formule XXXIV ou XXXVII ou l'ester benzylique et un groupe benzyléther $R_{12}$ éventuellement présent dans un composé de formule XXXXI, si on le désire on estérifie un groupe $R_2$ et si on le désire on estérifie un groupe carboxy $R_1$ ou $R_2$ ou le transforme en un sel avec une base pharmaceutiquement acceptable, ou on transforme un groupe amino substitué par un alcoyle inférieur en un sel avec un acide pharmaceutiquement acceptable.

**Revendications pour l'Etat contractant suivant : ES**

**1.** Procédé de préparation de composés de formule

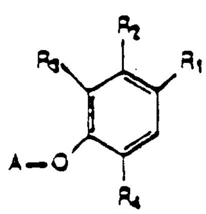

I

dans laquelle $R_1$ représente -C(O)OR, un hydrogène, un acétyle, un hydroxy ou un alcanoyloxy ; $R_2$ représente - C(O)OR, un hydroxy, un hydrogène ou un alcanoyloxy ; R représente un hydrogène, un alcoyle inférieur ou un - $(CH_2)_nN$ (alcoyle inférieur)$_2$ ; $R_3$ représente un hydrogène, un alcoyle inférieur ou un amino ; $R_4$ représente un hydrogène, un alcoyle inférieur, un halogène ou un amino, A représente un groupe de formule

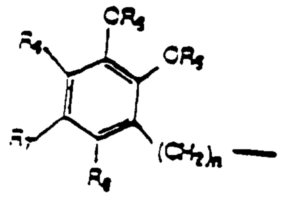

A '

dans laquelle $R_5$ représente un hydrogène ou un acyle ; $R_6$ représente un hydrogène, un halogène, un alcoyle inférieur, un aryle ou un cycloalcoyle ; et $R_7$ et $R_8$ représentent indépendamment l'un de l'autre un hydrogène, un alcoyle inférieur ou un halogène et n est un nombre allant de 2 à 10, ou A représente un groupe de formule

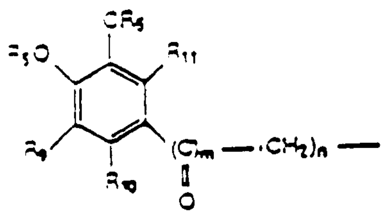

A "

où $R_5$ représente un hydrogène ou un acyle ; $R_9$ représente un hydrogène ou un alcoyle inférieur ; $R_{10}$ représente un hydrogène, un alcoyle inférieur ou un halogène ; $R_{11}$ représente un hydrogène, un alcoyle inférieur, un cycloalcoyle ou un halogène, m vaut 0 ou 1 et n est un nombre entier allant de 2 à 10 ; avec la condition que pas plus d'un radical $R_1$ ou $R_2$ est un hydroxy, un alcanoyloxy ou -C(O)OR, et, si R est un hydrogène, leurs sels avec des bases pharmaceutiquement acceptables, et si R représente $(CH_2)_n$-N-(alcoyle inférieur)$_2$, leurs sels d'addition d'acides avec des acides pharmaceutiquement acceptables,

caractérisé en ce qu'on fait réagir un composé de formule

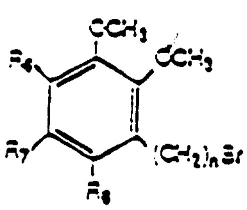

III

ou

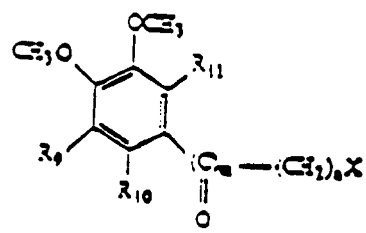

XXXVI

avec un composé de formule

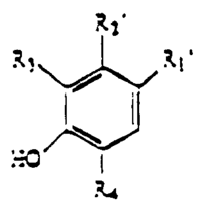

XXXIII

pour donner un composé de formule

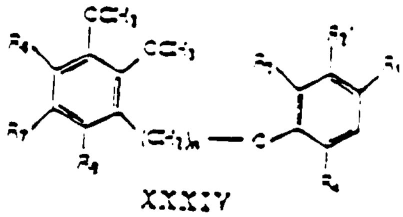

XXXIV

ou

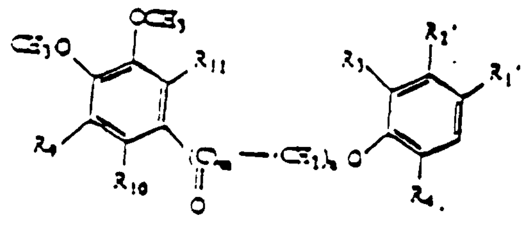

XXXVII

ou
en ce qu'on fait réagir un composé de formule

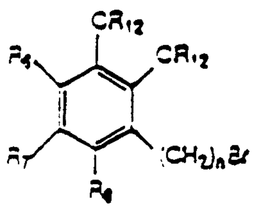

XXXIX

avec un composé de formule

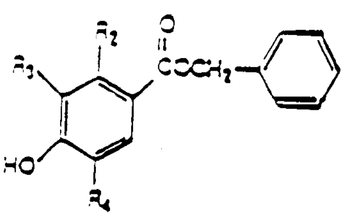

XXXX

pour donner un composé de formule

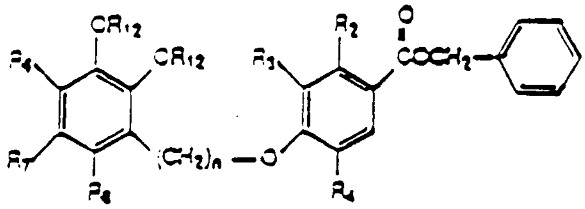

XXXXI

dans laquelle $R_1$' représente un hydrogène, un alcoxy inférieur carbonyle, ou un acétyle ; $R_2$' représente un hydrogène ou un alcoxy inférieur carbonyle ; $R_{12}$ représente un benzyle ou un acyle ; X représente un brome ou un méthanesulfonyloxy et les autres symboles ont la signification indiquée ci-dessus, en supposant que pas plus d'un radical $R_1$' ou $R_2$' ne représente un alcoxy inférieur carbonyle,

après quoi on sépare les groupes méthoxy dans un composé de formule XXXIV ou XXXVII ou

l'ester benzylique et un groupe benzyléther $R_{12}$ éventuellement présent dans un composé de formule XXXXI, si on le désire on estérifie un groupe $R_2$ et si on le désire on estérifie un groupe carboxy $R_1$ ou $R_2$ ou le transforme en un sel avec une base pharmaceutiquement acceptable, ou on transforme un groupe amino substitué par un alcoyle inférieur en un sel avec un acide pharmaceutiquement acceptable.

2. Procédé selon la revendication 1, caractérisé en ce qu'on prépare des composés de formule

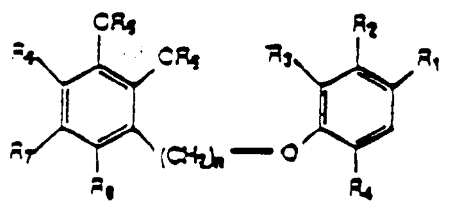

Ia

dans laquelle $R_1$ représente un carboxy ou un acétyle, $R_2$ un hydroxy, $R_3$ un hydrogène ou un propyle, $R_4$ un hydrogène ou un chlore, n est un nombre entier allant de 2 à 10, $R_5$ représente un hydrogène, ou un acétyle, $R_6$ représente un hydrogène, un alcoyle inférieur ou un aryle, et $R_7$ et $R_8$ représentent un hydrogène.

3. Procédé selon la revendication 1, caractérisé en ce qu'on prépare des composés de formule

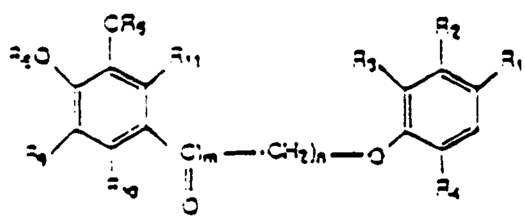

Ib

dans laquelle $R_1$ représente un carboxy ou un acétyle, $R_2$ représente un hydroxy, $R_3$ représente un hydrogène ou un propyle, $R_4$ représente un hydrogène ou un chlore, m vaut 0 ou 1, n est un nombre entier allant de 2 à 10, $R_5$ représente un hydrogène ou un acétyle, $R_9$ et $R_{10}$ représentent un hydrogène et $R_{11}$ représente un hydrogène ou un chlore.

4. Procédé selon la revendication 1, dans lequel $R_6$ représente un hydrogène, un alcoyle inférieur, un aryle ou un cycloalcoyle et les autres symboles ont la signification indiquée dans la revendication 1.

5. Procédé selon la revendication 1, caractérisé en ce qu'on prépare l'acide 4-[6-(2,3-dihydroxyphényl)-hexyloxy]-2-hydroxybenzoïque.

6. Procédé selon la revendication 1, caractérisé en ce qu'on prépare un composé du groupe des composés suivants :
   Acide 4-[4-(2,3-dihydroxyphényl)butoxy]-2-hydroxy-3-propylbenzoïque ;
   Acide 4-[6-(2,3-dihydroxyphényl)hexyloxy]-2-hydroxy-3-propylbenzoïque ;
   Acide 4-[8-(2,3-dihydroxyphényl)octyloxy]-2-hydroxy-3-propylbenzoïque ;
   Acide 4-[6-[2,3-bis(acétyloxy)phényl]hexyloxy]-2-hydroxy-3-propylbenzoïque ; et
   Acide 4-[6-[2,3-dihydroxy-4-(1-méthyléthyl)phényl]-hexyloxy]-2-hydroxy-3-propylbenzoïque.

7. Procédé selon la revendication 1, caractérisé en ce qu'on prépare un composé du groupe des composés suivants :
   Acide 4-[3-(3,4-dihydroxyphényl)propoxy]-2-hydroxy-3-propylbenzoïque ;

Acide 4-[6-(3,4-dihydroxyphényl)hexyloxy]-2-hydroxy-3-propyl-benzoïque ;
Acide 4-[[6-(3,4-dihydroxyphényl)-6-oxohexyl]oxy]-2-hydroxy-3-propylbenzoïque ; et
1-[2-hydroxy-4-[4-(2,3-dihydroxyphényl)butoxy]-3-propylphényl]éthanone.

**8.** Procédé selon la revendication 1, caractérisé en ce qu'on prépare un composé du groupe des composés suivants :
Acide 4-[6-[2,3-dihydroxy-4-(1,1-diméthyléthyl)phényl]hexyloxy]-2-hydroxy-3-propylbenzoïque ;
Acide 4-[6-[2,3-dihydroxy-4-méthylphényl]hexyloxy]-2-hydroxy-3-propylbenzoïque ;
Acide 4-[4-[2,3-dihydroxy-4-(2-méthylpropyl)phényl]butoxy-2-hydroxy-3-propylbenzoïque ;
Acide 4-[6-[2,3-dihydroxy-5,6-diméthylphényl]-hexyloxy]-2-hydroxy-3-propylbenzoïque ;
Acide 4-[5-[5-chloro-2,3-dihydroxyphényl]pentyloxy]-2-hydroxy-3-propylbenzoïque ;
Acide 4-[6-[2,3-dihydroxy-6-fluorophényl]hexyloxy]-2-hydroxy-3-propylbenzoïque ;
Acide 4-[4-[2,3-dihydroxy-4-cyclohexylphényl]-butoxy]-2-hydroxy-3-propylbenzoïque ;
Acide 4-[4-[2,3-dihydroxy-4-(1,1-diméthyléthyl)phényl]butoxy]-2-hydroxy-3-propylbenzoïque ;
Acide 4-[8-[2,3-dihydroxy-4-(1,1-diméthyléthyl)phényl]octyloxy]-2-hydroxy-3-propylbenzoïque ;
Acide 4-[4-[2,3-dihydroxy-4-(1,1-diméthyléthyl)phényl]butoxy]-2-hydroxybenzoïque ;
Acide 4-[8-[2,3-dihydroxy-4-(1-méthyléthyl)phényl]octyloxy]-2-hydroxybenzoïque ;
Acide 4-[4-[2,3-dihydroxyphényl]butoxy]-3,5-dipropyl-2-hydroxybenzoïque ;
Acide 4-[4-[2,3-dihydroxy-4-(1-méthyléthyl)phényl]butoxy]-3,5-dipropylbenzoïque ;
Acide 4-[6-[2,3-dihydroxy-4-(1-méthyléthyl)phényl]hexyloxy]-3-propylbenzoïque ;
Acide 4-[6-[2,3-bis-acétyloxy)-4-(1-méthyléthyl)phényl]hexyloxy]-2-hydroxy-3-propylbenzoïque ;
Acide 2-acétyloxy-4-[6-(2,3-dihydroxy-4-(1-méthyléthyl)phényl]-hexyloxy]-3-propylbenzoïque ;
Acide 4-[6-[2,3-bis[4-méthylbenzoyl)oxy]-4-(1-méthyléthyl)-phényl]hexyloxy]-2-hydroxy-3-propylbenzoïque ;
1-[2-hydroxy-4-[6-[2,3-dihydroxy-4-(1,1-diméthyléthyl)-phényl]hexyloxy]-3-propylphényl]éthanone ;
1-[2-hydroxy-4-[8-[2,3-dihydroxy-6-fluorophényl]octyloxy]-3-propylphényl]éthanone ;
1-[2-hydroxy-4-(6-[6-chloro-2,3-dihydroxyphényl]-hexyloxy]-3-propylphényl]éthanone ;
1-[2-hydroxy-4-[6-[5,6-dichloro-2,3-dihydroxyphényl]hexyloxy]-3-propylphényl]éthanone ;
1-[2-hydroxy-4-[6-[2,3-dihydroxy-4,5,6-trichlorophényl]-hexyloxy]-3-propylphényl]éthanone ;
Acide 5-[6-(2,3-dihydroxy-4-(1-méthyléthyl)phényl]hexyloxy]-2-hydroxybenzoïque ;
Acide 4-[6-[2,3-dihydroxy-4-(1-méthyléthyl)phényl]hexyloxy]-benzoïque ;
Ester éthylique de l'acide 4-[6-[2,3-dihydroxy-4-(1-méthyléthyl)phényl]hexyloxy]-2-hydroxy-3-propylbenzoïque ;
Ester éthylique de l'acide 4-[6-[2,3-dihydroxy-4-(1-méthyléthyl)phényl]hexyloxy]-2-hydroxy-3-propylbenzoïque ;
Acide 4-[8-(3,4-dihydroxyphényl)octyloxy]-2-hydroxy-3-propyl-benzoïque ;
Ester éthylique de l'acide 4-[3-(3,4-dihydroxyphényl)propoxy]-2-hydroxy-3-propylbenzoïque ;
Acide 4-[6-(3,4-dihydroxyphényl)hexyloxy]-2-hydroxy-benzoïque ;
Acide 4-[6-(3,4-dihydroxyphényl)hexyloxy]-3-propylbenzoïque ;
Acide 4-[8-(3,4-dihydroxyphényl)octyloxy]benzoïque ;
Acide 3-[8-(3,4-dihydroxyphényl)octyloxy]benzoïque ;
Acide 5-[8-(3,4-dihydroxyphényl)octyloxy]-2-hydroxybenzoïque ;
Acide 4-[[8-(3,4-dihydroxyphényl)-8-oxooctyl]oxy]-2-hydroxy-3-propylbenzoïque ;
Acide 4-[[4-(3,4-dihydroxyphényl)-4-oxobutyl]-oxy]-2-hydroxy-3-propylbenzoïque ;
Acide 4-[6-(3,4-dihydroxy-5-fluorophényl)hexyloxy]-2-hydroxy-3-propylbenzoïque ;
Acide 4-[6-(3,4-dihydroxy-6-fluorophényl)hexyloxy]-2-hydroxy-3-propylbenzoïque ;
Acide 4-[6-(3,4-dihydroxy-6-chlorophényl)hexyloxy]-2-hydroxy-3-propylbenzoïque ;
Acide 4-[4-[3,4-dihydroxy-5-(1-méthyléthyl)phényl]butoxy]-2-hydroxy-3-propylbenzoïque ;
Acide 4-[4-[3,4-dihydroxy-5-(1,1-diméthyléthyl)-phényl]butoxy]-2-hydroxybenzoïque ;
Acide 4-[6-[3,4-bis(acétyloxy)phényl]hexyloxy]-2-hydroxy-3-propylbenzoïque ;
Acide 2-acétyloxy-4-[6-(3,4-dihydroxyphényl)hexyloxy]-3-propylbenzoïque ;
Acide 4-[[4-[3,4-dihydroxy-5-(1-méthyléthyl)phényl]-4-oxobutyl]-oxy]-2-hydroxybenzoïque ;
Acide 4-[6-[3,4-dihydroxy-2-(1-méthyléthyl)phényl]hexyloxy]-2-hydroxy-3-proylbenzoïque ;
Acide 4-[[4-(3,4-dihydroxy-2,5-diméthylphényl)-4-oxobutyl]oxy]-2-hydroxy-3-propylbenzoïque ;
Acide 4-[4-(3,4-dihydroxy-2,5-diméthylphényl)butoxy]-2-hydroxy-3-propylbenzoïque ;
Acide 4-[[6-(3,4-dihydroxy-2,5,6-triméthylphényl)-6-oxohexyl]-oxy]-2-hydroxy-3-propylbenzoïque ;
Acide 4-[6-(3,4-dihydroxy-5,6-triméthylphényl)-hexyloxy]-2-hydroxy-3-propylbenzoïque ;
1-[2-hydroxy-4-[6-(3,4-dihydroxyphényl)hexyloxy]-3-propyl-phényl-1-éthanone ;

1-[2-hydroxy-4-[8-(3,4-dihydroxyphényl)octyloxy]-3-propyl-phényl-1-éthanone ;
1-[2-hydroxy-4-[6-(3,4-dihydroxy-2,5-diméthyl-phényl)hexyloxy]-3-propylphényl-1-éthanone ;
1-[2-hydroxy-4-[6-(3,4-dihydroxy-6-chlorophényl)-hexyloxy]-3-propylphényl-1-éthanone ;
1-[2-hydroxy-4-[6-(3,4-dihydroxy-2-chlorophényl)hexyloxy]-3-propylphényl-1-éthanone ;
1-[2-hydroxy-4-[6-(3,4-dihydroxy-6-fluorophényl)hexyloxy]-3-propylphényl-1-éthanone.

**9.** Application des composés de formule I et de leurs sels selon la revendication 1 à la préparation de médicaments pour le traitement des maladies inflammatoires, des maladies cardiovasculaires, des maladies de la peau et des maladies bronchopulmonaires.

**Revendications pour l'Etat contractant suivant : GR**

**1.** Procédé de préparation de composés de formule

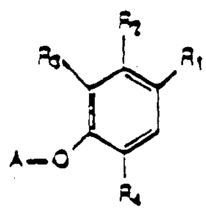

I

dans laquelle $R_1$ représente -C(O)OR, un hydrogène, un acétyle, un hydroxy ou un alcanoyloxy ; $R_2$ représente - C(O)OR, un hydroxy, un hydrogène ou un alcanoyloxy ; R représente un hydrogène, un alcoyle inférieur ou un - $(CH_2)_nN$ (alcoyle inférieur)$_2$ ; $R_3$ représente un hydrogène, un alcoyle inférieur ou un amino ; $R_4$ représente un hydrogène, un alcoyle inférieur, un halogène ou un amino, A représente un groupe de formule

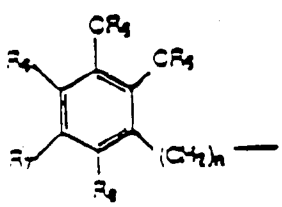

A'

dans laquelle $R_5$ représente un hydrogène ou un acyle ; $R_6$ représente un hydrogène, un halogène, un alcoyle inférieur, un aryle ou un cycloalcoyle ; et $R_7$ et $R_8$ représentent indépendamment l'un de l'autre un hydrogène, un alcoyle inférieur ou un halogène et n est un nombre allant de 2 à 10, ou A représente un groupe de formule

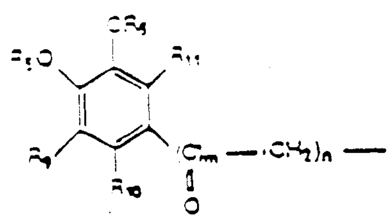

A"

où $R_5$ représente un hydrogène ou un acyle ; $R_9$ représente un hydrogène ou un alcoyle inférieur ; $R_{10}$ représente un hydrogène, un alcoyle inférieur ou un halogène ; $R_{11}$ représente un hydrogène, un alcoyle inférieur, un cycloalcoyle ou un halogène, m vaut 0 ou 1 et n est un nombre entier allant de 2 à

10 ; avec la condition que pas plus d'un radical $R_1$ ou $R_2$ est un hydroxy, un alcanoyloxy ou -C(O)OR, et, si R est un hydrogène, leurs sels avec des bases pharmaceutiquement acceptables, et si R représente $(CH_2)_n$-N-(alcoyle inférieur)$_2$, leurs sels d'addition d'acides avec des acides pharmaceutiquement acceptables,

caractérisé en ce qu'on fait réagir un composé de formule

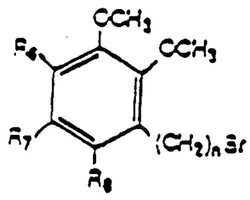

III

ou

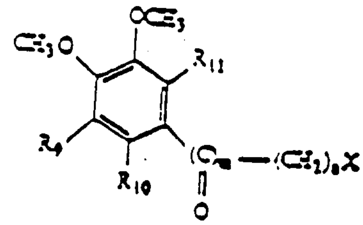

XXXVI

avec un composé de formule

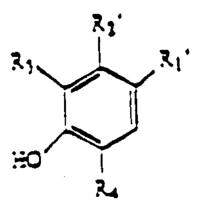

XXXIII

pour donner un composé de formule

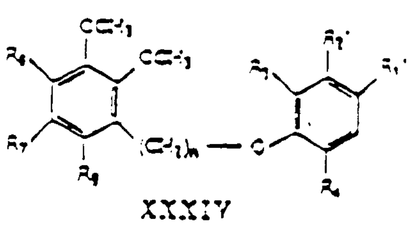

XXXIV

ou

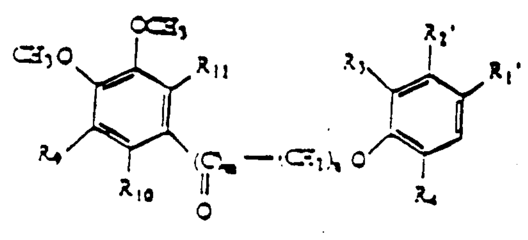

XXXVII

ou

en ce qu'on fait réagir un composé de formule

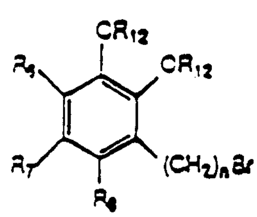

XXXIX

avec un composé de formule

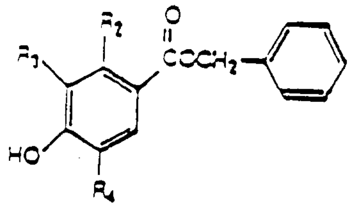

XXXX

pour donner un composé de formule

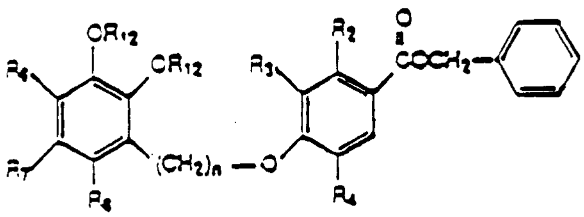

XXXXI

dans laquelle $R_1$' représente un hydrogène, un alcoxy inférieur carbonyle, ou un acétyle ; $R_2$' représente un hydrogène ou un alcoxy inférieur carbonyle ; $R_{12}$ représente un benzyle ou un acyle ; X représente un brome ou un méthanesulfonyloxy et les autres symboles ont la signification indiquée ci-dessus, en supposant que pas plus d'un radical $R_1$' ou $R_2$' ne représente un alcoxy inférieur carbonyle,

après quoi on sépare les groupes méthoxy dans un composé de formule XXXIV ou XXXVII ou l'ester benzylique et un groupe benzyléther $R_{12}$ éventuellement présent dans un composé de formule XXXXI, si on le désire on estérifie un groupe $R_2$ et si on le désire on estérifie un groupe carboxy $R_1$ ou $R_2$ ou le transforme en un sel avec une base pharmaceutiquement acceptable, ou on transforme un groupe amino substitué par un alcoyle inférieur en un sel avec un acide pharmaceutiquement acceptable.

**2.** Procédé selon la revendication 1, caractérisé en ce qu'on prépare des composés de formule

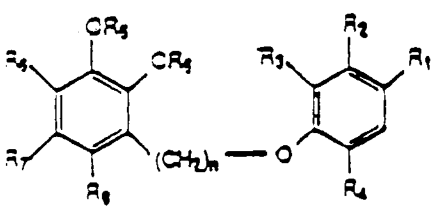

I a

dans laquelle $R_1$ représente un carboxy ou un acétyle, $R_2$ un hydroxy, $R_3$ un hydrogène ou un propyle, $R_4$ un hydrogène ou un chlore, n est un nombre entier allant de 2 à 10, $R_5$ représente un hydrogène, ou un acétyle, $R_6$ représente un hydrogène, un alcoyle inférieur ou un aryle, et $R_7$ et $R_8$ représentent un hydrogène.

**3.** Procédé selon la revendication 1, caractérisé en ce qu'on prépare des composés de formule

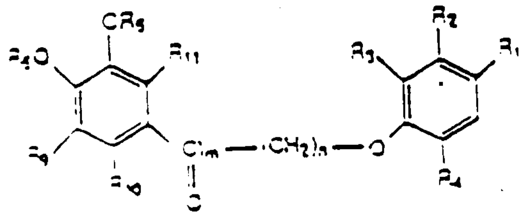

I b

dans laquelle $R_1$ représente un carboxy ou un acétyle, $R_2$ représente un hydroxy, $R_3$ représente un hydrogène ou un propyle, $R_4$ représente un hydrogène ou un chlore, m vaut 0 ou 1, n est un nombre entier allant de 2 à 10, $R_5$ représente un hydrogène ou un acétyle, $R_9$ et $R_{10}$ représentent un hydrogène et $R_{11}$ représente un hydrogène ou un chlore.

**4.** Procédé selon la revendication 1, dans lequel $R_6$ représente un hydrogène, un alcoyle inférieur, un aryle ou un cycloalcoyle et les autres symboles ont la signification indiquée dans la revendication 1.

**5.** Procédé selon la revendication 1, caractérisé en ce qu'on prépare l'acide 4-[6-(2,3-dihydroxyphényl)-hexyloxy]-2-hydroxybenzoïque.

**6.** Procédé selon la revendication 1, caractérisé en ce qu'on prépare un composé du groupe des composés suivants :

Acide 4-[4-(2,3-dihydroxyphényl)butoxy]-2-hydroxy-3-propylbenzoïque ;
Acide 4-[6-(2,3-dihydroxyphényl)hexyloxy]-2-hydroxy-3-propylbenzoïque ;
Acide 4-[8-(2,3-dihydroxyphényl)octyloxy]-2-hydroxy-3-propylbenzoïque ;
Acide 4-[6-[2,3-bis(acétyloxy)phényl]hexyloxy]-2-hydroxy-3-propylbenzoïque ; et
Acide 4-[6-[2,3-dihydroxy-4-(1-méthyléthyl)phényl]-hexyloxy]-2-hydroxy-3-propylbenzoïque.

**7.** Procédé selon la revendication 1, caractérisé en ce qu'on prépare un composé du groupe des composés suivants :

Acide 4-[3-(3,4-dihydroxyphényl)propoxy]-2-hydroxy-3-propylbenzoïque ;
Acide 4-[6-(3,4-dihydroxyphényl)hexyloxy]-2-hydroxy-3-propyl-benzoïque ;
Acide 4-[[6-(3,4-dihydroxyphényl)-6-oxohexyl]oxy]-2-hydroxy-3-propylbenzoïque ; et
1-[2-hydroxy-4-[4-(2,3-dihydroxyphényl)butoxy]-3-propylphényl]éthanone.

**8.** Procédé selon la revendication 1, caractérisé en ce qu'on prépare un composé du groupe des composés suivants :

Acide 4-[6-[2,3-dihydroxy-4-(1,1-diméthyléthyl)phényl]hexyloxy]-2-hydroxy-3-propylbenzoïque ;
Acide 4-[6-[2,3-dihydroxy-4-méthylphényl]hexyloxy]-2-hydroxy-3-propylbenzoïque ;
Acide 4-[4-[2,3-dihydroxy-4-(2-méthylpropyl)phényl]butoxy-2-hydroxy-3-propylbenzoïque ;
Acide 4-[6-[2,3-dihydroxy-5,6-diméthylphényl]-hexyloxy]-2-hydroxy-3-propylbenzoïque ;
Acide 4-[5-[5-chloro-2,3-dihydroxyphényl]pentyloxy]-2-hydroxy-3-propylbenzoïque ;
Acide 4-[6-[2,3-dihydroxy-6-fluorophényl]hexyloxy]-2-hydroxy-3-propylbenzoïque ;
Acide 4-[4-[2,3-dihydroxy-4-cyclohexylphényl]-butoxy]-2-hydroxy-3-propylbenzoïque ;
Acide 4-[4-[2,3-dihydroxy-4-(1,1-diméthyléthyl)phényl]butoxy]-2-hydroxy-3-propylbenzoïque ;
Acide 4-[8-[2,3-dihydroxy-4-(1,1-diméthyléthyl)phényl]octyloxy]-2-hydroxy-3-propylbenzoïque ;
Acide 4-[4-[2,3-dihydroxy-4-(1,1-diméthyléthyl)phényl]butoxy]-2-hydroxybenzoïque ;
Acide 4-[8-[2,3-dihydroxy-4-(1-méthyléthyl)phényl]octyloxy]-2-hydroxybenzoïque ;
Acide 4-[4-[2,3-dihydroxyphényl]butoxy]-3,5-dipropyl-2-hydroxybenzoïque ;
Acide 4-[4-[2,3-dihydroxy-4-(1-méthyléthyl)phényl]butoxy]-3,5-dipropylbenzoïque ;
Acide 4-[6-[2,3-dihydroxy-4-(1-méthyléthyl)phényl]hexyloxy]-3-propylbenzoïque ;
Acide 4-[6-[2,3-bis-acétyloxy)-4-(1-méthyléthyl)phényl]hexyloxy]-2-hydroxy-3-propylbenzoïque ;
Acide 2-acétyloxy-4-[6-(2,3-dihydroxy-4-(1-méthyléthyl)phényl]-hexyloxy]-3-propylbenzoïque ;
Acide 4-[6-[2,3-bis[4-méthylbenzoyl)oxy]-4-(1-méthyléthyl)-phényl]hexyloxy]-2-hydroxy-3-propylbenzoïque ;
1-[2-hydroxy-4-[6-[2,3-dihydroxy-4-(1,1-diméthyléthyl)-phényl]hexyloxy]-3-propylphényl]éthanone ;
1-[2-hydroxy-4-[8-[2,3-dihydroxy-6-fluorophényl]octyloxy]-3-propylphényl]éthanone ;

1-[2-hydroxy-4-(6-[6-chloro-2,3-dihydroxyphényl]-hexyloxy]-3-propylphényl]éthanone ;
1-[2-hydroxy-4-[6-[5,6-dichloro-2,3-dihydroxyphényl]hexyloxy]-3-propylphényl]éthanone ;
1-[2-hydroxy-4-[6-[2,3-dihydroxy-4,5,6-trichlorophényl]-hexyloxy]-3-propylphényl]éthanone ;
Acide 5-[6-(2,3-dihydroxy-4-(1-méthyléthyl)phényl]hexyloxy]-2-hydroxybenzoïque ;
Acide 4-[6-[2,3-dihydroxy-4-(1-méthyléthyl)phényl]hexyloxy]-benzoïque ;
Ester éthylique de l'acide 4-[6-[2,3-dihydroxy-4-(1-méthyléthyl)phényl]hexyloxy]-2-hydroxy-3-propylbenzoïque ;
Ester éthylique de l'acide 4-[6-[2,3-dihydroxy-4-(1-méthyléthyl)phényl]hexyloxy]-2-hydroxy-3-propylbenzoïque ;
Acide 4-[8-(3,4-dihydroxyphényl)octyloxy]-2-hydroxy-3-propyl-benzoïque ;
Ester éthylique de l'acide 4-[3-(3,4-dihydroxyphényl)propoxy]-2-hydroxy-3-propylbenzoïque ;
Acide 4-[6-(3,4-dihydroxyphényl)hexyloxy]-2-hydroxy-benzoïque ;
Acide 4-[6-(3,4-dihydroxyphényl)hexyloxy]-3-propylbenzoïque ;
Acide 4-[8-(3,4-dihydroxyphényl)octyloxy]benzoïque ;
Acide 3-[8-(3,4-dihydroxyphényl)octyloxy]benzoïque ;
Acide 5-[8-(3,4-dihydroxyphényl)octyloxy]-2-hydroxybenzoïque ;
Acide 4-[[8-(3,4-dihydroxyphényl)-8-oxooctyl]oxy]-2-hydroxy-3-propylbenzoïque ;
Acide 4-[[4-(3,4-dihydroxyphényl)-4-oxobutyl]-oxy]-2-hydroxy-3-propylbenzoïque ;
Acide 4-[6-(3,4-dihydroxy-5-fluorophényl)hexyloxy]-2-hydroxy-3-propylbenzoïque ;
Acide 4-[6-(3,4-dihydroxy-6-fluorophényl)hexyloxy]-2-hydroxy-3-propylbenzoïque ;
Acide 4-[6-(3,4-dihydroxy-6-chlorophényl)hexyloxy]-2-hydroxy-3-propylbenzoïque ;
Acide 4-[4-[3,4-dihydroxy-5-(1-méthyléthyl)phényl]butoxy]-2-hydroxy-3-propylbenzoïque ;
Acide 4-[4-[3,4-dihydroxy-5-(1,1-diméthyléthyl)-phényl]butoxy]-2-hydroxybenzoïque ;
Acide 4-[6-[3,4-bis(acétyloxy)phényl]hexyloxy]-2-hydroxy-3-propylbenzoïque ;
Acide 2-acétyloxy-4-[6-(3,4-dihydroxyphényl)hexyloxy]-3-propylbenzoïque ;
Acide 4-[[4-[3,4-dihydroxy-5-(1-méthyléthyl)phényl]-4-oxobutyl]-oxy]-2-hydroxybenzoïque ;
Acide 4-[6-[3,4-dihydroxy-2-(1-méthyléthyl)phényl]hexyloxy]-2-hydroxy-3-proylbenzoïque ;
Acide 4-[[4-(3,4-dihydroxy-2,5-diméthylphényl)-4-oxobutyl]oxy]-2-hydroxy-3-propylbenzoïque ;
Acide 4-[4-(3,4-dihydroxy-2,5-diméthylphényl)butoxy]-2-hydroxy-3-propylbenzoïque ;
Acide 4-[[6-(3,4-dihydroxy-2,5,6-triméthylphényl)-6-oxohexyl]-oxy]-2-hydroxy-3-propylbenzoïque ;
Acide 4-[6-(3,4-dihydroxy-5,6-triméthylphényl)-hexyloxy]-2-hydroxy-3-propylbenzoïque ;
1-[2-hydroxy-4-[6-(3,4-dihydroxyphényl)hexyloxy]-3-propyl-phényl-1-éthanone ;
1-[2-hydroxy-4-[8-(3,4-dihydroxyphényl)octyloxy]-3-propyl-phényl-1-éthanone ;
1-[2-hydroxy-4-[6-(3,4-dihydroxy-2,5-diméthyl-phényl)hexyloxy]-3-propylphényl-1-éthanone ;
1-[2-hydroxy-4-[6-(3,4-dihydroxy-6-chlorophényl)-hexyloxy]-3-propylphényl-1-éthanone ;
1-[2-hydroxy-4-[6-(3,4-dihydroxy-2-chlorophényl)hexyloxy]-3-propylphényl-1-éthanone ;
1-[2-hydroxy-4-[6-(3,4-dihydroxy-6-fluorophényl)hexyloxy]-3-propylphényl-1-éthanone.

**9.** Application des composés de formule I et de leurs sels selon la revendication 1 à la préparation de médicaments pour le traitement des maladies inflammatoires, des maladies cardiovasculaires, des maladies de la peau et des maladies bronchopulmonaires.

**10.** Composés de formule

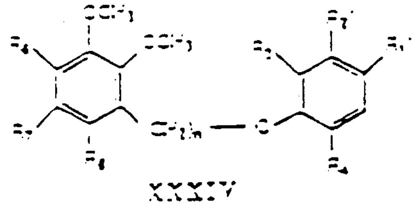

XXXIV

dans laquelle $R_1$' représente -C(O)OR', un acétyle, hydrogène ou hydroxy ; $R_2$' représente -C(O)OR', un hydrogène ou hydroxy ; R' représente un hydrogène ou un alcoyle inférieur ; $R_3$ représente un hydrogène ou un alcoyle inférieur ; $R_4$ représente un hydrogène, alcoyle inférieur ou halogène ; $R_6$ représente un hydrogène, alcoyle inférieur, aryle ou cycloalcoyle ; $R_7$ et $R_8$ représente un hydrogène, alcoyle inférieur ou halogène ; et n est un nombre entier allant de 2 à 10 ; en supposant que pas plus

d'un des radicaux $R_1$' et $R_2$' ne représente -C(O)OR' ou un hydroxy.

**11.** Acide 4-[6-(2,3-diméthoxyphényl)hexyloxy]-2-hydroxy-3-propylbenzoïque et son ester méthylique.

**12.** Composés de formule

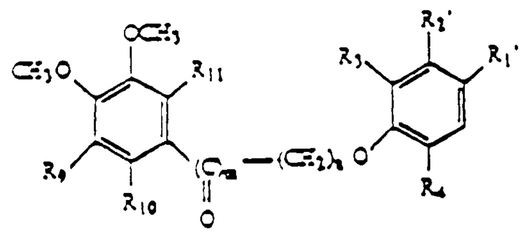

XXXVII

dans laquelle $R_1$' représente -C(O)OR', un acétyle, un hydrogène ou un hydroxy ; $R_2$' représente -C(O)-OR', un hydrogène ou un hydroxy ; R' représente un hydrogène ou un alcoyle inférieur ; $R_3$ représente un hydrogène ou un alcoyle inférieur ; $R_4$ représente un hydrogène, alcoyle inférieur ou halogène ; $R_9$ représente un hydrogène ou alcoyle inférieur ; $R_{10}$ représente un hydrogène, alcoyle inférieur ou halogène ; $R_{11}$ représente un hydrogène, alcoyle inférieur, cycloalcoyle ou halogène ; m vaut 0 ou 1 et n est un nombre entier allant de 2 à 10, en supposant que pas plus d'un radical $R_1$' et $R_2$' ne représente -C(O)OR' ou un hydroxy.